# EUROPEAN PATENT APPLICATION

(11) **EP 4 796 124 A1**
(43) Date of publication of application: **26.08.2026**
(21) Application number: 25159350.5
(22) Date of filing: 21.02.2025
(51) Int. Cl.: A61K 40/42, A61K 40/15, A61K 40/32

(54) **NOVEL CHIMERIC T CELL RECEPTOR CONSTRUCT FOR ENHANCED IMMUNE CELL-BASED IMMUNOTHERAPY**

(71) Applicant: Medigene Immunotherapies GmbH, 82152 Planegg-Martinsried (DE)
(72) Inventor: Mishra, Kanuj, 85748 Garching bei München (DE); Hoefl, Valentin, 82110 Germering (DE); Lösch, Barbara, 81245 München (DE)

(57) **Abstract**

The present invention relates to a functional chimeric T cell receptor (TCR) construct that can be expressed in various (immune) cell types. The present invention also relates to a method of producing the TCR construct and nucleic acids encoding the same. The invention also relates to their uses and pharmaceutical compositions comprising the same.

## Description

### FIELD OF INVENTION

The present invention relates to a functional chimeric T cell receptor (TCR) construct that can be expressed in various (immune) cell types. The present invention also relates to a method of producing the TCR construct and nucleic acids encoding the same. The invention also relates to their uses and pharmaceutical compositions comprising the same.

### BACKGROUND

During antigen processing, antigens are degraded inside cells and then carried to the cell surface by major histocompatibility complex (MHC) molecules. T cells are able to recognise this peptide:MHC complex at the surface of the antigen presenting cell. There are two different classes of MHC molecules, MHC I and MHC II, that deliver peptides from different cellular compartments to the cell surface. The T cell receptor or TCR is the molecule found on the surface of T cells that is responsible for recognizing antigens bound to MHC molecules. The TCR heterodimer consists of an α (alpha) and β (beta) chain in 95% of T cells, whereas 5% of T cells have TCRs consisting of γ (gamma) and δ (delta) chains. Engagement of the TCR with antigen and MHC results in activation of its T lymphocyte through a series of biochemical events mediated by associated enzymes, co-receptors, and specialized accessory molecules.

Each chain of the TCR is a member of the immunoglobulin superfamily and possesses one N-terminal immunoglobulin (Ig)-variable (V) domain, one Ig-constant (C) domain, a transmembrane/cell membrane-spanning region, and a short cytoplasmic tail at the C-terminal end. The variable domain of both the TCR α-chain and β-chain have three hypervariable or complementarity determining regions (CDRs). CDR3 is the main CDR responsible for recognizing processed antigen, although CDR1 of the alpha chain has also been shown to interact with the N-terminal part of the antigenic peptide, whereas CDR1 of the beta chain interacts with the C-terminal part of the peptide. CDR2 is thought to recognize the MHC molecule. Framework regions (FRs) are positioned between the CDRs. These regions provide the structure of the TCR variable region. The repertoire of TCR variable regions is generated by combinatorial joining of variable (V), joining (J) and diversity (D) genes; and by N region diversification (nucleotides inserted by the enzyme deoxynucleotidyl-transferase). α and γ chains are formed from recombination events between the V and J segments. β and δ chains are formed from recombination events involving the V, D and J segments.

Each recombined TCR possesses unique antigen specificity, determined by the structure of the antigen-binding site formed by the α and β chains in case of αβ T cells or γ and δ chains in case of γδ T cells.

The structure allows the TCR to associate with other molecules like CD3 which possess γ, δ, and ε chains sharing many structural features and the ζ-chain (zeta chain, CD247), which is structurally distinct and forms homodimers. These accessory molecules have negatively charged transmembrane regions and are vital to propagating the signal from the TCR into the cell. The CD3 γ, δ and ε chains and the ζ-chains, together with the TCR, form what is known as the T cell receptor complex. Signaling via the TCR is initiated when the TCR recognizes and binds to a peptide-MHC complex. This interaction leads to the phosphorylation of immunoreceptor tyrosine-based activation motifs (ITAMs) on the cytoplasmic tails of CD3 molecules, in particular the CD3ζ homodimer, by the kinase Lck, inducing downstream signalling pathways. The signal from the T cell receptor complex is enhanced by simultaneous binding of the MHC molecules by a specific co-receptor. On helper T cells, this co-receptor is CD4 (specific for class II MHC); whereas on cytotoxic T cells, this coreceptor is CD8 (specific for class I MHC). The co-receptor not only ensures the specificity of the TCR for an antigen, but also allows prolonged engagement between the antigen presenting cell and the T cell and recruits essential molecules (e.g., Lck) inside the cell involved in the signalling of the activated T lymphocyte.

Interestingly, signalling via γδ TCRs does not necessarily depend on MHC antigen presentation, yet the activating ligands are still widely unknown.

TCR gene therapy is a form of immunotherapy that involves genetically engineering T cells by transferring antigen-specific TCR chains into recipient T-cells, thus redirecting the specificity of T lymphocytes to target antigens of interest. The concept of immunotherapy is based on the specificity of the adaptive immune response for the recognition and elimination of pathogens as well as tumor cells. The aim of a successful immunotherapy is the manipulation or reprogramming of the patient's immune response in order to specifically target pathogen-infected cells or tumor cells for destruction by the immune system.

Therapeutic approaches used to reprogram the immune system in the treatment of infectious diseases and cancer include active immunotherapy comprising the use of vaccination strategies, including dendritic cell (DC) vaccines, as well as passive immunotherapy comprising the application of specific antibodies or genetically engineered lymphocytes or the adoptive transfer of T cells specifically recognizing target antigens displayed by pathogen-infected cells or cancer cells. However, using T cells for immunotherapy has its limitations: while T cells expressing an engineered chimeric antigen receptor (CAR) typically recognize antigens expressed on the cell surface, a long-standing problem in TCR gene therapy, in which T cells are used that express engineered TCRs and thus allow for recognition of various MHC-presented antigens including intracellular proteins, is the dependency on antigen recognition in the context of MHC. One tumor escape mechanism is the downregulation of MHC and subsequent escape of TCR-T cell recognition.

Recent approaches have thus focused on employing immune cells other than T cells for advanced immunotherapies (e.g. in NK or B cells as in Qin, V.M.; D'Souza, C.; Neeson, P.J.; Zhu, J.J. Chimeric Antigen Receptor beyond CAR-T Cells. Cancers 2021, 13, 404. https://doi.org/10.3390/cancers13030404). Natural Killer (NK) cells are integral components of the innate immune system, playing a pivotal role in the body's defense against neoplastic and virally infected cells. Unlike their adaptive T cell counterparts, NK cells possess the unique ability to recognize and eliminate target cells without prior antigen exposure. Typically, MHC negative cells are recognized as targets and killed by NK cells. These inherent characteristics render NK cells an attractive platform for cellular immunotherapy development. However, the therapeutic potential of NK cells is somewhat limited by their lack of antigen-specific targeting capabilities, a feature that T cells possess due to their T cell receptors (TCRs). The close developmental relationship and similarities in cellular signalling and cytotoxicity mechanisms between T cells and NK cells provide a compelling rationale for exploring the expression of functional TCRs on NK cells. In this way combining the benefits of MHC-dependent recognition of tumor targets as well as recognizing tumor cells, that down-regulated the MHC expression on their surface.

Researchers have previously attempted to introduce TCRs into NK cells by expressing all or part of the CD3 complex (γ, δ, ε, and ζ chains) along with TCR α and β chains. However, this approach presents several challenges:
1. Complex Procedure: The process is cost intensive, time-consuming and involves multiple steps, including the initial introduction of CD3 chains, screening, and subsequent introduction of TCR α and β chains.
2. Payload Burden: The requirement to transfer multiple genes into NK cells places a significant burden on the gene transfer payload capacity.
3. Efficiency Concerns: The multi-step process may lead to reduced efficiency in generating functional TCR-expressing NK cells.

There is thus a need for a functional TCR construct that can reliably be introduced into cells. Depending on the recipient cell this would allow to make use of the advantageous characteristics of the recipient cell and the TCR.

### SUMMARY OF THE INVENTION

The present invention provides a novel functional TCR complex. This novel complex enables a reliable transfer of that TCR construct into cells (recipient cells). Due to the characteristics of the TCR construct according to the present invention it is possible to introduce it not only into T cells but also into other cell types that play a role in immune responses.

The proposed construct comprises a TCRα chain or a fragment thereof, a TCRβ chain or a fragment thereof and two CD3ζ chains or fragments thereof. Alternatively, the construct comprises a TCRγ chain or a fragment thereof, a TCRδ chain or a fragment thereof and two CD3ζ chains or fragments thereof. Preferably, the construct does not comprise other CD3 chains like CD3γ, CDδ or CD3ε chains or fragments thereof. Such a TCR construct according to the invention is smaller than a complete TCR construct with complete chains and all CD3 chains (γ, δ, and ε) and still ensures a reliable signalling mechanism.

Thus, the small TCR construct according to the present invention allows to be introduced into cells, not only T cells but also other cell types like, for example NK cells, B cells, macrophages, neutrophils etc.

Accordingly, in a first aspect the invention relates to a functional chimeric T cell receptor (TCR) construct comprising
a) a first fusion protein comprising a TCR α chain or fragment thereof and a first CD3ζ chain or fragment thereof, wherein the TCR α chain or fragment thereof comprises
   i. a first signal sequence,
   ii. a variable region (Vα), and
   iii. a constant region (Cα),
   and
b) a second fusion protein comprising a TCR β chain or fragment thereof and a second CD3ζ chain or fragment thereof, wherein the TCR β chain or fragment thereof comprises
   i. a second signal sequence,
   ii. a variable region (Vβ), and
   iii. a constant region (Cβ),
   or, alternatively, comprising
c) a first fusion protein comprising a TCR γ chain or fragment thereof and a first CD3ζ chain or fragment thereof, wherein the TCR γ chain or fragment thereof comprises
   i. a first signal sequence,
   ii. a variable region (Vγ), and
   iii. a constant region (Cγ),
   and
d) a second fusion protein comprising a TCR δ chain or fragment thereof and a second CD3ζ chain or fragment thereof, wherein the TCR δ chain or fragment thereof comprises
   i. a second signal sequence,
   ii. a variable region (Vδ), and
   iii. a constant region (Cδ).

Suitably, the TCR construct may not comprise a CD3γ, CD3δ, and/or CD3ε chain or fragment thereof.

Suitably, the first and/or the second signal sequence(s) may be (a) NK cell signal sequence(s), preferably from a protein selected from the group consisting of NKp46, NKp44, NKp30, and CD16A.

Suitably, the TCR construct may further comprise one or two DAP10 signalling domain(s).

According to another aspect, the invention refers to a method of producing a TCR construct comprising the step of culturing a cell comprising a nucleic acid encoding the TCR construct according to the invention.

According to another aspect, the invention refers to a nucleic acid encoding a TCR construct according to the invention.

According to another aspect, the invention refers to a vector comprising the nucleic acid of the invention.

According to another aspect, the invention refers to a cell expressing the TCR construct according to the invention.

Suitably, the cell may comprise
a) an expression vector which comprises at least one nucleic acid according to the invention and/or
b) a first expression vector which comprises a nucleic acid encoding the TCR α chain and the first CD3ζ chain according to the invention, and a second expression vector which comprises a nucleic acid encoding the TCR β chain and the second CD3ζ according to the invention.

Suitably, the cell may be a NK cell.

Suitably, the first and/or second signal sequence may be (a) NK cell signal sequence(s), preferably from a protein selected from the group consisting of NKp46, NKp44, NKp30, and CD16A.

According to a further aspect, the invention refers to a pharmaceutical composition comprising the TCR construct according to the invention, the nucleic acid according to the invention, the vector according to the invention, or the cell according to the invention.

According to a further aspect, the invention refers to the use of the TCR construct according to the invention, the nucleic acid according to the invention, the vector according to the invention, the cell according to the invention, or the pharmaceutical composition according to the invention as a medicament.

According to a further aspect, the invention refers to the use of the TCR construct according to the invention, the nucleic acid according to the invention, the vector according to the invention, the cell according to the invention, or the pharmaceutical composition according to the invention in the treatment of cancer.

According to a further aspect, the invention refers to adoptive immunotherapy using the TCR construct according to the invention, the nucleic acid according to the invention, the vector according to the invention, the cell according to the invention, or the pharmaceutical composition according to the invention.

Various aspects of the invention are described in further detail below.

### BRIEF DESCRIPTION OF THE FIGURES

Embodiments of the invention are further described hereinafter with reference to the accompanying figures:

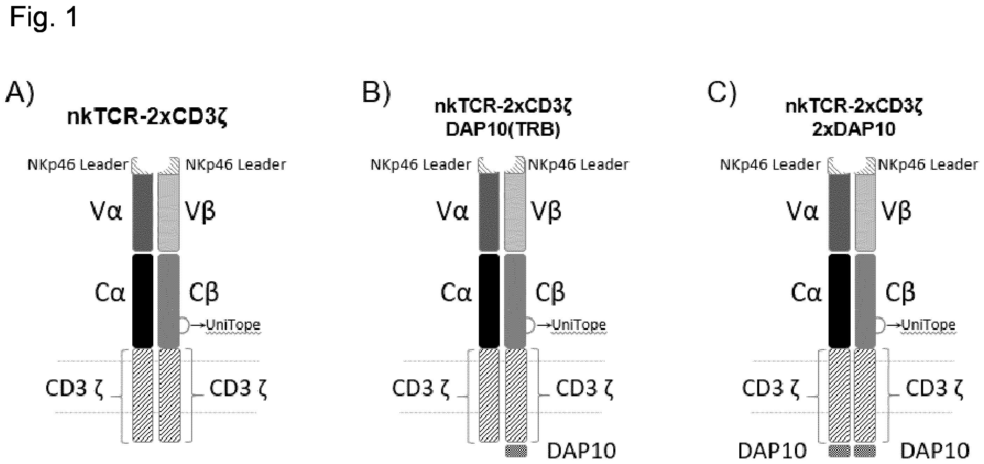

**Figure 1** shows a schematic representation of engineered TCR Constructs. **(A)** nkTCR-2xCD3ζ: This construct features a heterodimeric T cell receptor (TCR) composed of a TCRα and a TCRβchain, each followed by the transmembrane and cytoplasmic domains of CD3ζ. Both chains include a NKp46 signal peptide sequence for targeting the construct to the NK cell membrane, **(B)** nkTCR-2xCD3ζ DAP10(TRB): Incorporates a DAP10 activation domain linked to the TCRβ - CD3ζ part of the nkTCR-2xCD3ζ configuration, **(C)** nkTCR-2xCD3ζ 2xDAP10: This variant includes DAP10 activation domains fused to each CD3ζ of the nkTCR-2xCD3ζ structure. Each construct includes UniTope tag sequence with GSG linker incorporated into the Cβ regions. The Vα and Vβ domains used in all three chimeric NK-TCR constructs are specific for the SLL peptide of the NY-ESO-1 cancer-testis antigen presented by HLA-A*02:01 molecules.

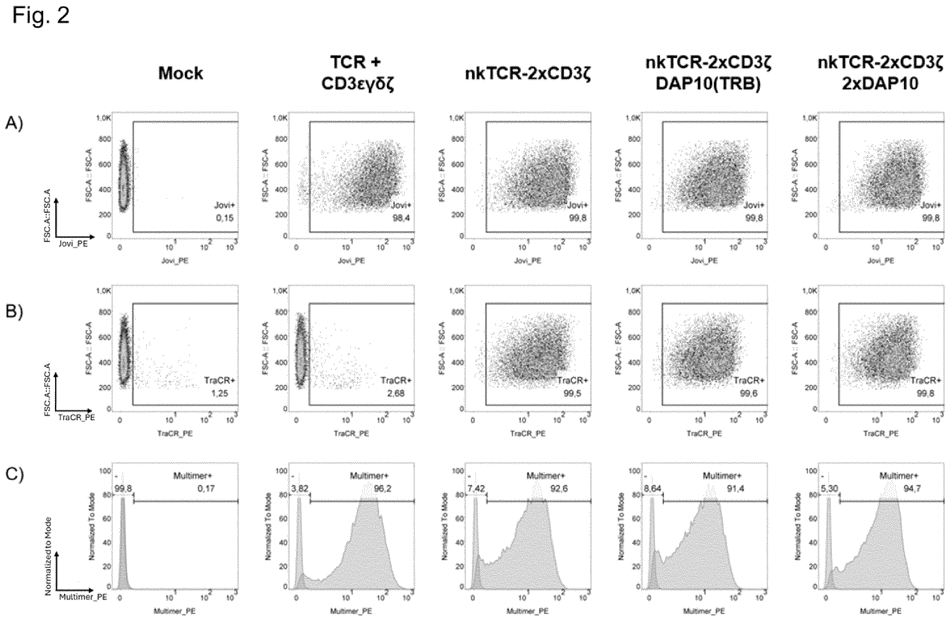

**Figure 2** shows the expression of chimeric NK-TCR in NK92 cells. **(A), (B)** and **(C)** Flow cytometric evaluation of NK92 cells transduced with TCR+CD3εγδζ (positive control), nkTCR-2xCD3ζ, nkTCR-2xCD3ζ DAP10(TRB) or nkTCR-2xCD3ζ 2xDAP10 constructs stained with PE-coupled anti Cb1 JOVI.1 antibody (Ab) or PE-coupled anti UniTope TraCR Ab or PE-coupled HLA-A*02:01-SLL multimer. **(A)** NK92 cells expressing the three chimeric constructs, as well as the positive control (TCR+CD3εγδζ), were successfully stained by PE-coupled anti-Cβ1 JOVI.1 Ab; **(B)** Cells expressing all three chimeric constructs containing the UniTope sequence were successfully stained by PE-coupled anti-UniTope TraCR Ab, whereas, as expected, the TCR+CD3εγδζ construct did not show any staining; **(C)** Proper TCR folding was assessed by staining NK92 cells with each chimeric NK-TCR construct as well as the positive control with PE-coupled HLA-A*02:01-SLL multimer, which successfully stained all NK-TCR transduced NK92 cells studied as well as the positive control. The TCR corresponds to NY-ESO1-specific TCR in all chimeric NK-TCR and control TCR (+CD3εγδζ) expressing NK92 cell populations.

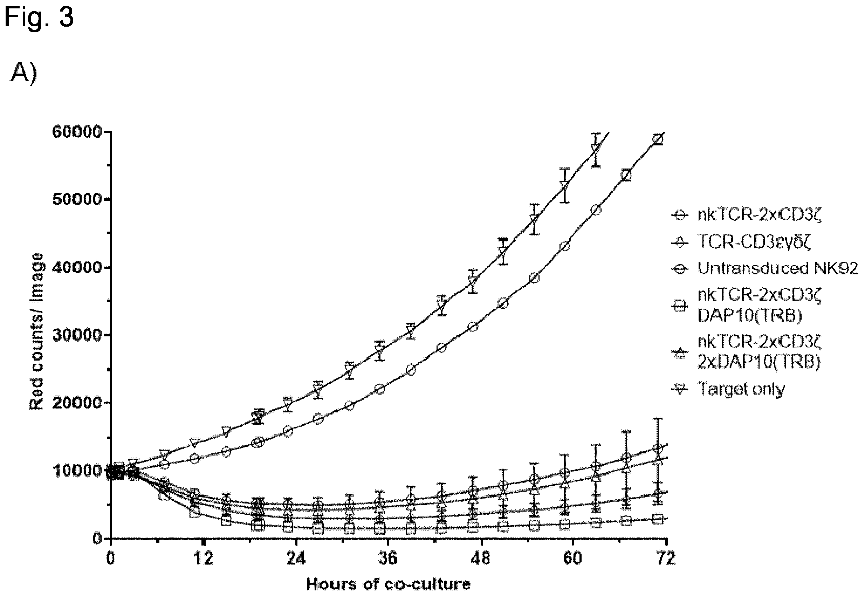

**Figure 3** shows the cytotoxic activity of chimeric NK-TCR transduced NK92 cells against Mel624.38 cells. **(A)** Live-cell imaging of a 2D killing assay with NK92 cells transduced with TCR+CD3εγδζ (positive control), nkTCR-2xCD3ζ, nkTCR-2xCD3ζ DAP10(TRB) or nkTCR-2xCD3ζ 2xDAP10 constructs or untransduced NK92 cells. Transduced or untranstuced NK92 effector cells were co-cultured with NuclightRed (NLR) labelled Mel624.38 target cells at an effector-to-target (E:T) ratio of 2:1 and red fluorescence intensity (red counts/image) was monitored and quantified using the Incucycte life-cell imaging system. The reduction in red fluorescence (red counts/Image) indicates target cell killing. NK92 cells expressing all three chimeric NK-TCR constructs as well as the positive control demonstrated specific cytotoxicity against Mel624.38_NLR target cells, whereas untransduced NK92 cells did not exhibit any killing activity. The Vα and Vβ domains used in all three chimeric NK-TCR constructs and positive control are specific for NY-ESO-1.

**Figure 1** shows the expression of chimeric NK-TCR in primary NK cells. **(A)** Flow cytometric evaluation of primary NK cells transduced with TCR+CD3εγδζ (positive control), nkTCR-2xCD3ζ, nkTCR-2xCD3ζ DAP10(TRB) or nkTCR-2xCD3ζ 2xDAP10 constructs stained with PE-coupled anti-TRBV12-3/4. Cells transduced with all three chimeric constructs, as well as the positive control (TCR+CD3εγδζ), were successfully stained by PE-coupled anti-TRBV12-3/4 antibody. The TCR corresponds to the NY-ESO-1-specific TCR in the three chimeric NK-TCRs and the positive control (TCR+CD3eydl). The Vα and Vβ domains used in the three chimeric NK-TCR constructs, and the positive control are specific for NY-ESO-1.

**Figure 2** shows a cytotoxic assay (2D killing assay) of Mel624.38 Cells by chimeric NK-TCR transduced blood derived NK cells. Primary NK cells transduced with the following constructs: TCR+CD3εγδζ (positive control), nkTCR-2xCD3ζ **(A),** nkTCR-2xCD3ζ-DAP10(TRB) **(B),** or nkTCR-2xCD3ζ-2xDAP10 (C), were used as effector cells against Mel624.38 target cells labeled with NuclightRed (NLR). Effector and target cells were co-cultured at an effector-to-target (E:T) ratio of 2:1 or 0.5:1 (labelled in figure legend). The reduction in red fluorescence (red counts/Image), indicating target cell killing, was monitored and quantified via the Incucyte device. Primary NK cells expressing all three chimeric NK-TCR constructs, as well as the positive control, demonstrated specific cytotoxicity against Mel624.38_NLR target cells, whereas untransduced blood derived NK cells did not exhibit any killing activity. The Vα and Vβ domains used in all three chimeric NK-TCR constructs, and positive control are specific for NY-ESO-1.

**Figure 3** shows the expression of chimeric NK-TCR in blood derived NK cells and cytotoxic assay (2D killing assay) of Mel624.38 cells by NK92 cells transduced with chimeric NK-TCRs (nkTCR-2xCD3ζ) equipped with different signal peptides (SP) (NKp44, NKp30, and TCRαβ). (A) Flow cytometric evaluation of NK92 cells transduced with NKp46SP_nkTCR-2xCD3ζ, NKp30SP_nkTCR-2xCD3ζ, NKp44SP_nkTCR-2xCD3ζ, and TCRαβSP_nkTCR-2xCD3ζ constructs stained with PE-coupled anti Cb1 JOVI.1 antibody, (B) Mean fluorescence intensity of positively stained NK92 cells with PE-coupled anti Cb1 JOVI.1 antibody, (C) Transduced NK92 cells with constructs NKp46SP_nkTCR-2xCD3ζ, NKp30SP_nkTCR-2xCD3ζ, NKp44SP_nkTCR-2xCD3ζ or TCRαβSP_nkTCR-2xCD3ζ or untransduced NK92 cells were used as effector cells against Mel624.38 target cells labeled with NuclightRed (NLR). Effector and target cells were co-cultured at an effector-to-target (E:T) ratio of 2:1. The reduction in red fluorescence (red counts/image), indicating target cell killing, was monitored and quantified via the Incucyte device. All three NK92 cell populations expressing chimeric NK-TCR constructs as well as the positive control cells demonstrated specific cytotoxicity against Mel624.38_NLR target cells, whereas untransduced NK92 cells did not exhibit any killing activity. The Vα and Vβ domains used in all three chimeric NK-TCR constructs as well as the positive control are specific for the SLL peptide of the NY-ESO-1 cancer-testis antigen presented by HLA-A*02:01 encoded molecules. SP indicated signal peptide in the figure legend.

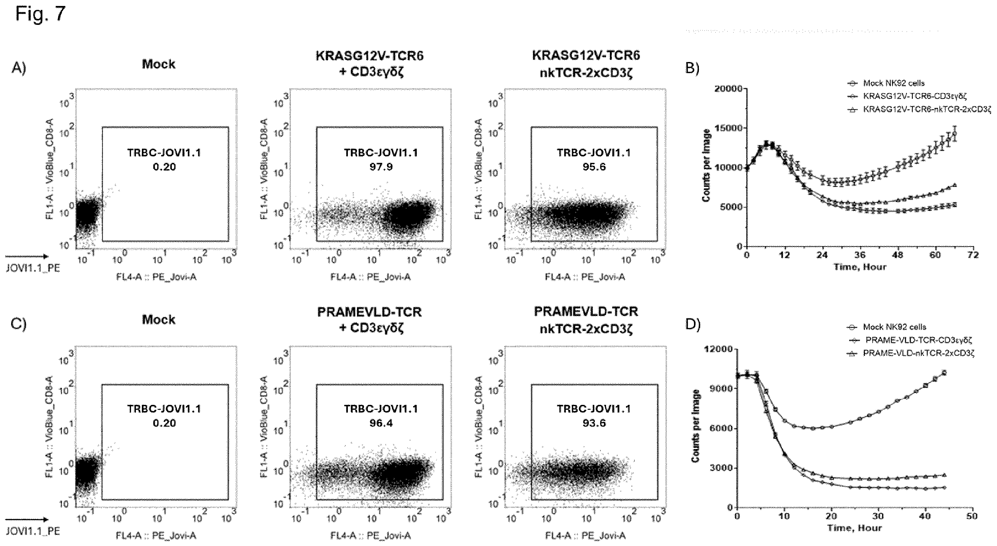

**Figure 7** shows the expression of KRASG12V & PRAME-VLD specific chimeric NK-TCR in NK92 cells and cytotoxic assay (2D killing assay) of DAN-G & Mel624.38 cells by NK92 cells transduced with chimeric KRASG12V-TCR6-nkTCR-2xCD3ζ & PRAME-VLD-nkTCR-2xCD3ζ respectively. **(A)** Flow cytometric evaluation of NK92 cells transduced with KRASG12V-TCR6-nkTCR-2xCD3ζ construct stained with PE-coupled anti Cb1 JOVI.1 antibody along with negative control (Mock) & positive control (KRASG12V-TCR6 + CD3εγδζ) , **(B)** Flow cytometric evaluation of NK92 cells transduced with PRAME-VLD-nkTCR-2xCD3ζ construct stained with PE-coupled anti Cb1 JOVI.1 antibody along with negative control (Mock) & positive control (PRAME-VLD-TCR + CD3εγδζ), **(C)** Transduced NK92 cells with construct KRASG12V-TCR6-nkTCR-2xCD3ζ or KRASG12V-TCR6 + CD3εγδζ or Mock transduced NK92 cells were used as effector cells against DAN-G target cells labeled with NuclightRed (NLR). Effector and target cells were co-cultured at an effector-to-target (E:T) ratio of 4:1, **(D)** Transduced NK92 cells with construct PRAME-VLD-nkTCR-2xCD3ζ or PRAME-VLD-TCR + CD3εγδζ or Mock transduced NK92 cells were used as effector cells against Mel624.38 target cells labeled with NuclightRed (NLR). Effector and target cells were co-cultured at an effector-to-target (E:T) ratio of 4:1. The reduction in red fluorescence (red counts/image), indicating target cell killing, was monitored and quantified via the Incucyte device. Both KRASG12V-TCR6-nkTCR-2xCD3ζ & PRAME-VLD-nkTCR-2xCD3ζ transduced NK92 cell populations as well as the positive control cells demonstrated specific cytotoxicity against DAN-G & Mel624.38_NLR target cells respectively, whereas Mock transduced NK92 cells did not exhibit any killing activity.

### DETAILED DESCRIPTION

The present invention provides a novel functional TCR complex which is independent of CD3 complex (γ, δ, ε, and ζ chains) expression. Further, a method for producing the same and their uses are provided.

The novel TCR complex according to the invention enables a reliable transfer of that TCR construct into cells (recipient cells). Due to the characteristics of the TCR construct according to the present invention it is possible to introduce it not only into T cells but also into other cell types.

The inventors surprisingly found that in order to be functional the TCR complex does not have to be made up of all CD3 chains. Rather the inventors found that even without the CD3 γ, δ, and ε chains the TCR complex can be functional, i.e. provides a reliable antigen binding and signaling mechanism. In addition, the CD3ζ chains do not have to be complete either. Just by using certain parts of the CD3ζ chains together with certain parts of the alpha and beta chains a functional TCR complex construct can be obtained.

The surprising finding that just certain chains and that just certain parts of those chains of a TCR complex are sufficient to be functional allow to engineer a functional TCR construct that is small in size and thus can much easier be introduced into cells in one step.

So far, all approaches to introduce TCR complexes, i.e. TCR and all or most of the CD3 chains, into cells (recipient cells) were of limited success. The main issues being the complexity of the process, the payload burden, and the efficiency.

To address these limitations, the inventors developed a novel chimeric fusion protein/fusion molecule that combines TCR antigen recognition capabilities with cell activation domains. The terms "chimeric fusion molecule", "chimeric fusion protein", "chimeric TCR construct", and "chimeric TCR molecule" are used interchangeably herein.

This innovative approach offers several advantages over existing methods:
1. Simplified design: the chimeric fusion molecule consists of two chains comprising either the TCR α or TCR β chains or fragments thereof. Both chains incorporate a signal peptide sequence replacing the TCR signal peptide sequences in cases where the recipient cell is not a T cell, followed by the TCR variable and constant regions (for antigen recognition) of either α or β chains, followed by activation chains CD3ζ or fragments thereof.
2. CD3γ-, CD3δ-, and CD3ε-independent expression: by integrating the necessary signaling components into a single molecule, CD3γ-, CD3δ-, and CD3ε -independent expression and functionality is ensured.
3. Antigen-specific recognition: the chimeric fusion molecule maintains specificity in recognizing TCR-specific antigens, enhancing the targeting capabilities of recipient cells.
4. Universal applicability: the chimeric fusion molecule provides a versatile construct that can accommodate any specific TCR variable chains. This universal design allows for the incorporation of diverse TCR specificities, enabling the targeting of a wide range of antigens.
5. Improved efficiency: the streamlined design potentially increases the efficiency of generating functional antigen-specific effector cells.

The chimeric T cell construct according to the invention may differ from natural TCRs in at least one of the following features:
1. Cell Type Adaptation: Designed for expression in the recipient cells using a cell type specific signal peptide for proper targeting.
2. Structural Modifications: Omission of TCR transmembrane and cytoplasmic domains, replacing their structures with at least partly engineered elements.
3. Signalling Mechanism: Direct incorporation of signalling domains (CD3ζ and optionally DAP10) into the construct, contrasting with the natural TCR's reliance on separate CD3 chains.
4. (Optional): Dimerization Approach: Use of a Boulter cysteine bridge for chain dimerization, compensating for the absence of the CD3 complex in the recipient cell environment.
5. (Optional): Enhanced Signaling Capacity: The addition of DAP10 domains provides signaling capabilities beyond those of natural TCRs. These designs strategically combine and modify TCR components to optimize expression and functionality in recipient cells, representing a significant advancement in synthetic immunoreceptor engineering.

With the TCR construct as described herein it is possible to use the properties of a TCR together with the properties of the recipient cell.

For B cells, the introduction of B cell-specific TCR constructs according to the inention, could be used to deliver therapeutic antibodies, such as anti-PD1 antibody and increase target presentation (in this way increasing potential immune answer to the tumor). TCR-macrophages might be able to directly kill tumour cells and facilitate antigen presentation. TCR-macrophages can also remodel the extracellular matrix through the production of MMP.

### Functional chimeric TCR construct

According to a first aspect of the invention a functional chimeric T cell receptor (TCR) construct is provided comprising
a) a first fusion protein comprising a TCR α chain or fragment thereof and a first CD3ζ chain or fragment thereof, wherein the TCR α chain or fragment thereof comprises
   i. a first signal sequence,
   ii. a variable region (Vα), and
   iii. a constant region (Cα),
   and
b) a second fusion protein comprising a TCR β chain or fragment thereof and a second CD3ζ chain or fragment thereof, wherein the TCR β chain or fragment thereof comprises
   i. a second signal sequence,
   ii. a variable region (Vβ), and
   iii. a constant region (Cβ),
   or, alternatively, comprising
c) a first fusion protein comprising a TCR γ chain or fragment thereof and a first CD3ζ chain or fragment thereof, wherein the TCR γ chain or fragment thereof comprises
   i. a first signal sequence,
   ii. a variable region (Vγ), and
   iii. a constant region (Cγ),
   and
d) a second fusion protein comprising a TCR δ chain or fragment thereof and a second CD3ζ chain or fragment thereof, wherein the TCR δ chain or fragment thereof comprises
   i. a second signal sequence,
   ii. a variable region (Vδ), and
   iii. a constant region (Cδ).

The term signal peptide (in literature also known as leader sequence peptide) refers to a sequence of amino acids at the N-terminus the TCR-construct described herein that determines their ultimate destination (i.e. cell surface). The signal peptide segment is cleaved off and not part of the mature protein.

"Recipient cell" refers to a cell which has typically been engineered to express a certain construct such as a receptor to recognize and bind specific antigens on target cells. Recipient cells enable targeted immune responses against malignant target cells. The terms "receptor cell" and "recipient cell" and "cell" as used herein refer to the cell into which the TCR construct is introduced. Recipient cells can be any immune cells, preferably, T cells, B cells, natural killer (NK) cells, macrophages, innate lymphoid cells (ILCs) or neutrophils.

The inventive designs strategically combine and modify TCR components to optimize expression and functionality in recipient cells, representing a significant advancement in synthetic immunoreceptor engineering. The constructs aim to harness the specificity of TCRs within the potent framework of recipient cells, potentially opening new avenues for targeted immunotherapies.

The variable regions of the TCR construct, including the highly diverse CDR3 region, can be selected from any naturally occurring or non-naturally occurring variable TCR regions. This flexibility allows for the generation of TCR constructs capable of recognizing all possible antigens as targets. The inventive design of the TCR construct can be applied across different types of TCRs, independent of target specificity. Thus, in other words, the present invention is not particularly limited in terms of TCR sequence. The present invention can be used as basic underlying concept for any desired TCR specificity. However, non-limiting examples of targets, which TCR constructs in accordance with the present invention may be specific for, include NYESO1, such as NYESO1-SSL; WT1, such as WT1-RMF; and PRAME, a preferentially expressed antigen in melanoma (PRAME) peptide.

The term "specific for" in the context of the invention means that the TCR construct is specifically binding to the target peptide.

TCR constructs in accordance with the invention may be isolated or purified. "Isolated" in the context of the invention means that the TCR is not present in the context in which it originally occurred in nature.

"Purified" in the context of the invention means, e.g., that the TCR is free or substantially free of other proteins and non-protein parts of the cell it originally stems from.

In one embodiment, the TCR construct does not comprise a CD3γ, CD3δ, and/or CD3ε chain or fragment thereof.

Hence, the only CD3 chains comprised by a TCR construct according to the invention are CD3 ζ chains or fragments thereof.

The designed chimeric TCR construct offers several advantages over expressing TCR α and β or TCR γ and δ chains with other CD3 chains in recipient cells:
A. Reduced Construct Size and Complexity
   1. Simplified design: This preferred construct incorporates only the essential
      components - TCR α and β chains or TCR γ and δ chains with dual CD3ζ signalling domains - eliminating the need for additional CD3 chains (γ, δ, ε).
   2. Smaller Genetic Payload: With the present design it can be avoided to also express CD3 zeta (492bp), CD3 epsilon (621bp), CD3 delta (513bp), and CD3 gamma (546 bp), which sums up to in total 2172 bp. The compact design results in a smaller overall construct size, which improves transduction efficiency and expression in recipient cells.
B. Optimized Signalling
   Direct signalling incorporation: By including dual CD3ζ domains directly in the construct, it bypasses the need for separate CD3 complex assembly, potentially leading to more efficient signal transduction.
C. Enhanced Functionality
   1. Recipient cell-specific optimization: The construct is tailored for recipient cell biology, potentially leading to better functional outcomes compared to a more T cell-centric approach (in cases when recipient cell is not a T cell).
   2. Potential for better activation: The dual CD3ζ domains directly attached to the TCR alpha and beta chain may provide signals comparable strong as a TCR+CD3 complex (positive control), compensating for the lack of other CD3 chains in non-T cells. The TCR zeta chain is an essential component of the T cell receptor (TCR) complex, which plays a critical role in initiating T cell activation. Upon antigen recognition, the TCR associates with co-receptors and signaling molecules, and the zeta chain becomes phosphorylated on tyrosine residues by Src family kinases like Lck. This phosphorylation event creates docking sites for various adaptor proteins, such as ZAP-70, which further propagates downstream signaling pathways. These signaling cascades lead to the activation of key molecules, including PLC-γ, IP3, and DAG, which mediate calcium flux and activation of protein kinase C, crucial for T cell activation and function. Ultimately, the TCR zeta chain signaling results in the activation of transcription factors like NF-κB and NFAT, driving T cell proliferation and cytokine production. Attaching the CD3ζ domains directly on the TCRα and β chains allows this signal transduction without the presence of CD3 chains γ, δ, ε, which stabilize the CD3 complex and have no direct influence on the signal transduction.

In one embodiment, the first and the second signal sequences are signal sequences from immune cells.

In one embodiment the first and the second signal sequences are signal sequences form B cells, natural killer (NK) cells, macrophages, innate lymphoid cells (ILCs), or neutrophils.

Since the signal sequence is responsible for the surface expression and targeting within the recipient cell the signal sequence(s) should be chosen to be of the same cell type as the recipient cell. For example, if the recipient cell is an NK cell the signal sequence(s) should originate from NK cells as well.

The first and the second signal sequences may be different or may be identical. The first and the second signal sequences may originate from a protein specifically expressed in the same cell type or from a protein specifically expressed in different cell types. For example, one signal sequence may originate from a T cell specific protein whereas the other signal sequence derives from an NK cell specific protein.

In one embodiment the first signal sequence is an NK cell signal sequence.

In one embodiment the second signal sequence is an NK cell signal sequence.

In one embodiment the first and the second signal sequences are NK cell signal sequences.

In one embodiment the first and the second signal sequences are identical.

In one embodiment, the first and/or the second signal sequence(s) is/are (a) NK cell signal sequence(s), preferably from a protein selected from the group consisting of NKp46, NKp44, NKp30, and CD16A.

NKp46 (also known as NKP46, NK-p46, or LY94) or also known as natural cytotoxicity triggering receptor 1 (NCR1) is a protein that in humans is encoded by the *NCR1* gene. NCR1 has also been designated as CD335.

NKp44 (also known as NKP44; NK-p44, LY95, and dJ149M18.1) or also known as natural cytotoxicity triggering receptor 2 (NCR2) is a protein that in humans is encoded by the *NCR2* gene. NCR2 has also been designated as CD336.

NKp30 also known as natural cytotoxicity triggering receptor 3 (NCR3) is a protein that in humans is encoded by the NCR3 gene. NCR3 has also been designated as CD337. NCR3 belongs to the family of NCR membrane receptors together with NCR1 (NKp46) and NCR2 (NKp44).

CD16A, also known as FcγRIIIa, is a low-affinity Fc receptor for IgG antibodies. CD16A is expressed on natural killer (NK) cells, monocytes, macrophages, and certain T cells. In humans, CD16A exists as a transmembrane receptor, while a closely related variant, CD16B (FcγRIIIb), is expressed exclusively on neutrophils as a GPl-anchored protein. CD16A binds to the Fc portion of IgG antibodies, particularly IgG1 and IgG3 isotypes, and plays a crucial role in antibody-dependent cellular cytotoxicity (ADCC)2. Upon engagement, CD16A triggers potent activation signals in NK cells, leading to target cell lysis, cytokine production, and enhanced NK cell proliferation

In one embodiment, the first and/or the second signal sequence(s) is/are (a) NK cell signal sequence(s) from the NKp46 protein.

In one embodiment, the first and the second signal sequences are NK cell signal sequences from the NKp46 protein.

In one embodiment, the first and/or the second signal sequence(s) is/are (a) NK cell signal sequence(s) from the NKp44 protein.

In one embodiment, the first and the second signal sequences are NK cell signal sequences from the NKp44 protein.

In one embodiment, the first and/or the second signal sequence(s) is/are (a) NK cell signal sequence(s) from the NKp30 protein.

In one embodiment, the first and the second signal sequences are NK cell signal sequences from the NKp30 protein.

In one embodiment, the first and/or the second signal sequence(s) is/are (a) NK cell signal sequence(s) from the CD16A protein.

In one embodiment, the first and the second signal sequences are NK cell signal sequences from the CD16A protein.

In one embodiment the NKp46 signal sequence comprises or consists of SEQ ID NO: 1 (MSSTLPALLCVGLCLSQRISA). In another embodiment the NKp46 signal peptide is at least 70%, at least 75%, at least 80%, at least 85%, at least 90%, at least 95%, at least 96%, at least 97%, at least 98%, or at least 99% identical to SEQ ID NO: 1.

In one embodiment the NKp30 signal sequence comprises or consists of SEQ ID NO: 2 (MAWMLLLILIMVHPGSCA). In another embodiment the NKp30 signal peptide is at least 70%, at least 75%, at least 80%, at least 85%, at least 90%, at least 95%, at least 96%, at least 97%, at least 98%, or at least 99% identical to SEQ ID NO: 2.

In one embodiment the NKp44 signal sequence comprises or consists of SEQ ID NO: 3 (MAWRALHPLLLLLLLFPGSQA). In another embodiment the NKp44 signal peptide is at least 70%, at least 75%, at least 80%, at least 85%, at least 90%, at least 95%, at least 96%, at least 97%, at least 98%, or at least 99% identical to SEQ ID NO: 3.

In one embodiment the CD16A signal sequence comprises or consists of SEQ ID NO: 4 (MWQLLLPTALLLLVSA). In another embodiment the CD16A signal peptide is at least 70%, at least 75%, at least 80%, at least 85%, at least 90%, at least 95%, at least 96%, at least 97%, at least 98%, or at least 99% identical to SEQ ID NO: 4.

In case where the recipient cell is an NK cell this novel chimeric NK-TCR construct according to the invention has the potential to create a new class of cellular therapeutics with significant advantages over current T cell-based approaches:
1. Enhanced Cytotoxicity: By combining the natural cytotoxic capabilities of NK cells with the antigen-specific targeting of TCRs an improved tumor cell elimination can be achieved.
2. Reduced Off-Target Effects: The antigen-specific targeting could potentially reduce off-target effects compared to conventional NK cell therapies.
3. Broader Applicability: This approach expands the range of targetable antigens for NK cell-based immunotherapies.
4. Simplified Manufacturing: The single-molecule design streamlines the manufacturing process, reducing costs and improving scalability.

The constructs aim to harness the specificity of TCRs within the potent cytotoxic framework of NK cells, potentially opening new avenues for targeted immunotherapies.

These advantages make chimeric NK-TCR a more streamlined and potentially more effective approach for expressing functional TCRs in NK cells compared to expressing full TCR α and β or γ and δ chains with all CD3 components. The significantly smaller construct size is particularly beneficial for improving transduction efficiency and expression in NK cells.

Furthermore, this approach substantially reduces the complexity of generating NK cells with TCR functionality. By utilizing a single, compact construct instead of multiple components, it simplifies the genetic modification process, potentially leading to more consistent and efficient production of TCR-expressing NK cells. This reduction in complexity not only streamlines the manufacturing process but may also contribute to improved quality control and scalability in clinical applications.

In one embodiment the TCRα signal sequence comprises or consists of SEQ ID NO: 5 (MKSLRVLLVILWLQLSWVWSQ). In another embodiment the TCRα signal sequence is at least 70%, at least 75%, at least 80%, at least 85%, at least 90%, at least 95%, at least 96%, at least 97%, at least 98%, or at least 99% identical to SEQ ID NO: 5.

In one embodiment the TCRβ signal sequence comprises or consists of SEQ ID NO: 6 (MGSWTLCCVSLCILVAKHT). In another embodiment the TCRβ signal sequence is at least 70%, at least 75%, at least 80%, at least 85%, at least 90%, at least 95%, at least 96%, at least 97%, at least 98%, or at least 99% identical to SEQ ID NO: 6.

In one embodiment the B-cell signal sequence (CD79A) comprises or consists of SEQ ID NO: 50 (MPGGPGVLQALPATIFLLFLLSAVYLGPGCQA). In another embodiment the TCRα signal sequence is at least 70%, at least 75%, at least 80%, at least 85%, at least 90%, at least 95%, at least 96%, at least 97%, at least 98%, or at least 99% identical to SEQ ID NO: 50.

In one embodiment the B-cell signal sequence (CD79B) comprises or consists of SEQ ID NO: 51 (MARLALSPVPSHWMVALLLLLSAEPVPA). In another embodiment the TCRα signal sequence is at least 70%, at least 75%, at least 80%, at least 85%, at least 90%, at least 95%, at least 96%, at least 97%, at least 98%, or at least 99% identical to SEQ ID NO: 51.

In one embodiment the macrophage signal sequence (CD14) comprises or consists of SEQ ID NO: 52 (MERASCLLLLLLPLVHVSA). In another embodiment the TCRα signal sequence is at least 70%, at least 75%, at least 80%, at least 85%, at least 90%, at least 95%, at least 96%, at least 97%, at least 98%, or at least 99% identical to SEQ ID NO: 52.

In one embodiment the macrophage signal sequence (CD40) comprises or consists of SEQ ID NO: 53 (MVRLPLQCVLWGCLLTAVHP). In another embodiment the TCRα signal sequence is at least 70%, at least 75%, at least 80%, at least 85%, at least 90%, at least 95%, at least 96%, at least 97%, at least 98%, or at least 99% identical to SEQ ID NO: 53.

In one embodiment the macrophage signal sequence (CD11b) comprises or consists of SEQ ID NO: 54 (MALRVLLLTALTLCHG). In another embodiment the TCRα signal sequence is at least 70%, at least 75%, at least 80%, at least 85%, at least 90%, at least 95%, at least 96%, at least 97%, at least 98%, or at least 99% identical to SEQ ID NO: 54.

In one embodiment the macrophage signal pe sequence ptide (CD64) comprises or consists of SEQ ID NO: 55 (MWFLTTLLLWVPVDG). In another embodiment the TCRα signal sequence is at least 70%, at least 75%, at least 80%, at least 85%, at least 90%, at least 95%, at least 96%, at least 97%, at least 98%, or at least 99% identical to SEQ ID NO: 55.

Although indicated herein as being a cell type specific signal sequence (e.g. a "macrophage signal peptide") these signal peptides may be found in other cell types as well. For example, CD14 is also found in neutrophils.

The TCR construct comprises a first and a second CD3ζ chain as part of the two fusion proteins. The CD3ζ chains are important for a functional signalling of the TCR construct. Within each fusion protein the CD3ζ chains are linked to the constant region of the TCR α, β, γ or δ chains, respectively (see Fig. 1 for α and β chains). The linking can either be via a linker or without any linker. The first and second CD3ζ chains may be different or may be identical. They may originate from the same cell type as the recipient cell or they originate from other cell types.

In one embodiment the first and the second CD3ζ chain or fragments thereof are identical.

The CD3ζ chains (UniProt ID P20963, the transmembrane domain ranging from amino acid positions 31 to 51 & the cytoplasmic domain ranging from amino acids positions 52 to 164) replace (at least partially) the transmembrane and/or the cytoplasmic parts of the constant regions of the α and/or β chains or the γ and/or δ chains of a natural TCR. Specifically, for the α chain (TRAC, UniProt ID-P01848), the transmembrane region spans amino acids 116-138, and the cytoplasmic part includes amino acids 139-140. For the β chain (TRBC1, UniProt ID-P01850), the transmembrane region spans amino acids 150-170, and the cytoplasmic domain includes amino acids 171-176. The CD3ζ chains themselves, as used for the TCR constructs according to the invention, may not be complete CD3ζ chains as in natural TCR complexes. Rather, the TCR constructs according to the invention may comprise as first and/or second CD3ζ chain the transmembrane and the cytoplasmic domain of the CD3ζ chain, i.e. CD3 ζ chains wherein the extracellular domain is omitted.

In one embodiment the first and/or second CD3ζ chain(s) comprises or consists of SEQ ID NO: 7. In another embodiment the first and/or second CD3ζ chain(s) is/are at least 70%, at least 75%, at least 80%, at least 85%, at least 90%, at least 95%, at least 96%, at least 97%, at least 98%, or at least 99% identical to SEQ ID NO: 7.

In one embodiment the first and second CD3ζ chains comprise or consist of SEQ ID NO: 7. In another embodiment the first and second CD3ζ chains are at least 70%, at least 75%, at least 80%, at least 85%, at least 90%, at least 95%, at least 96%, at least 97%, at least 98%, or at least 99% identical to SEQ ID NO: 7.

Due to the replacement of certain parts of the TCR α and/or β beta chain or the TCR γ and/or δ chains, respectively, as well as by using only the transmembrane and the cytoplasmic part of the CD3ζ chain the size of the TCR construct can be reduced leading to the advantages of a smaller overall construct size as set forth herein.

In one embodiment, the complete membrane and cytoplasmic domain of TCR α and TCRβ chain are omitted and replaced by the CD3ζ membrane and cytoplasmic domain. In one embodiment the CD3 ζ chain does not comprise any extracellular domain.

The direct link of the CD3 zeta part to the TCR alpha/beta chain or fragment thereof in the fusion protein it is possible to actually obtain the TCR construct expression and also to obtain an expressed TCR construct with a functional signalling capacity. Due to the direct link in the fusion protein construct the CD3 zeta chains are able to assist in expression (pairing of the two chains alpha and beta or gamma and delta) and in signal transduction.

In one embodiment the TCR construct further comprises one or two DAP10 signaling domain(s).

The DAP10 signaling domain(s) can enhance the activation potential of the TCR construct.

In a preferred embodiment the TCR construct comprises two DAP10 signaling domains.

In one embodiment the first and/or second DAP10 signaling domain comprises the cytoplasmic part of the DAP10 protein.

In one embodiment the first and/or second DAP10 signaling domain(s) comprise(s) or consist(s) of SEQ ID NO: 8 (LCARPRRSPAQEDGKVYINMPGRG). In another embodiment the first and/or second DAP10 signaling domain(s) is/are at least 70%, at least 75%, at least 80%, at least 85%, at least 90%, at least 95%, at least 96%, at least 97%, at least 98%, or at least 99% identical to SEQ ID NO: 8.

In one embodiment the first and the second DAP10 signaling domains comprise or consist of SEQ ID NO: 8.

The DAP10 signaling domain is linked to the CD3ζ chain. The linking can either be via a linker or without any linker.

Within the first and the second fusion proteins the comprised domains/chains/regions/sequences (like signal sequences, variable TCR regions, constant TCR regions, CD3ζ, and DAP10) can be linked with each other either via a linker or by direct linkage to each other. It is also possible that some domains/chains/regions/sequences have no linker between each other whereas others do. On each fusion protein in can be a mixture of direct fusions and linker usage between the different parts. Also, between the first and the second fusion protein it can differ. For example, if on the first fusion protein the CD3ζ chain has a linker it does not have to be the case on the second fusion protein as well.

Appropriate linkers for linking the chains/domains/regions/sequences within the first and/or second fusion protein are well known in the art. Suitable linker sequences could. For example, be identified by literature search. Generally, however, the linker sequence may be any sequence which does not impair the function of the TCR construct.

In one embodiment, the linker sequence has a length of at least 2 amino acids, preferably at least 3 amino acids.

In one embodiment, the linker sequence is composed of amino acids selected from the group consisting of Glycine, Serine, Alanine, Threonine, Glutamate and Proline.

In one embodiment the linker comprises or consists of a sequence selected from the group consisting of: GGS, GSG, AP, APAP (SEQ ID NO: 9), ESGS (SEQ ID NO: 10), GGGS (SEQ ID NO: 11), GGGGS (SEQ ID NO: 12), and GSGGSG (SEQ ID NO: 13). As elsewhere, standard single letter amino acid codes are used.

In a preferred embodiment the linker used is GGS

In a preferred embodiment only the CD3ζ chain and the DAP10 signalling domain are linked via a linker on one or both fusion proteins.

In a preferred embodiment the CD3ζ chain and the DAP10 signalling domain are linked via a GGS linker.

In one embodiment the Vα comprises a CDR 1 comprising or consisting of the amino acid sequence of SEQ ID NO: 14, a CDR 2 having the amino acid sequence of SEQ ID NO: 15, and a CDR 3 having the amino acid sequence of SEQ ID NO: 16. In another embodiment the Vα is at least 70%, at least 75%, at least 80%, at least 85%, at least 90%, at least 95%, at least 96%, at least 97%, at least 98%, or at least 99% identical to SEQ ID NOs: 14, 15 and/or 16.

In one embodiment the Vα comprises a CDR 1 comprising or consisting of the amino acid sequence of SEQ ID NO: 29, a CDR 2 having the amino acid sequence of SEQ ID NO: 30, and a CDR 3 having the amino acid sequence of SEQ ID NO: 31. In another embodiment the Vα is at least 70%, at least 75%, at least 80%, at least 85%, at least 90%, at least 95%, at least 96%, at least 97%, at least 98%, or at least 99% identical to SEQ ID NOs: 29, 30 and/or 31.

In one embodiment the Vα comprises a CDR 1 comprising or consisting of the amino acid sequence of SEQ ID NO: 40, a CDR 2 having the amino acid sequence of SEQ ID NO: 41, and a CDR 3 having the amino acid sequence of SEQ ID NO: 42. In another embodiment the Vα is at least 70%, at least 75%, at least 80%, at least 85%, at least 90%, at least 95%, at least 96%, at least 97%, at least 98%, or at least 99% identical to SEQ ID NOs: 40, 41 and/or 42.

In one embodiment the Vβ comprises a CDR 1 comprising or consisting of the amino acid sequence of SEQ ID NO: 17, a CDR 2 having the amino acid sequence of SEQ ID NO: 18, and a CDR 3 having the amino acid sequence of SEQ ID NO: 19. In another embodiment the Vβ is at least 70%, at least 75%, at least 80%, at least 85%, at least 90%, at least 95%, at least 96%, at least 97%, at least 98%, or at least 99% identical to SEQ ID NOs: 17, 18 and/or 19.

In one embodiment the Vβ comprises a CDR 1 comprising or consisting of the amino acid sequence of SEQ ID NO: 32, a CDR 2 having the amino acid sequence of SEQ ID NO: 33, and a CDR 3 having the amino acid sequence of SEQ ID NO: 34. In another embodiment the Vβ is at least 70%, at least 75%, at least 80%, at least 85%, at least 90%, at least 95%, at least 96%, at least 97%, at least 98%, or at least 99% identical to SEQ ID NOs: 32, 33 and/or 34.

In one embodiment the Vβ comprises a CDR 1 comprising or consisting of the amino acid sequence of SEQ ID NO: 43, a CDR 2 having the amino acid sequence of SEQ ID NO: 44, and a CDR 3 having the amino acid sequence of SEQ ID NO: 45. In another embodiment the Vβ is at least 70%, at least 75%, at least 80%, at least 85%, at least 90%, at least 95%, at least 96%, at least 97%, at least 98%, or at least 99% identical to SEQ ID NOs: 43, 44 and/or 45.

In one embodiment the Vα comprises or consists of the amino acid sequence of SEQ ID NO: 20. In another embodiment the Vα is at least 70%, at least 75%, at least 80%, at least 85%, at least 90%, at least 95%, at least 96%, at least 97%, at least 98%, or at least 99% identical to SEQ ID NO:20.

In one embodiment the Vα comprises or consists of the amino acid sequence of SEQ ID NO: 35. In another embodiment the Vα is at least 70%, at least 75%, at least 80%, at least 85%, at least 90%, at least 95%, at least 96%, at least 97%, at least 98%, or at least 99% identical to SEQ ID NO:35.

In one embodiment the Vα comprises or consists of the amino acid sequence of SEQ ID NO: 46. In another embodiment the Vα is at least 70%, at least 75%, at least 80%, at least 85%, at least 90%, at least 95%, at least 96%, at least 97%, at least 98%, or at least 99% identical to SEQ ID NO:46.

In one embodiment the Vβ comprises or consists of the amino acid sequence of SEQ ID NO: 21. In another embodiment the Vβ is at least 70%, at least 75%, at least 80%, at least 85%, at least 90%, at least 95%, at least 96%, at least 97%, at least 98%, or at least 99% identical to SEQ ID NO:21.

In one embodiment the Vβ comprises or consists of the amino acid sequence of SEQ ID NO: 36. In another embodiment the Vβ is at least 70%, at least 75%, at least 80%, at least 85%, at least 90%, at least 95%, at least 96%, at least 97%, at least 98%, or at least 99% identical to SEQ ID NO:36.

In one embodiment the Vβ comprises or consists of the amino acid sequence of SEQ ID NO: 47. In another embodiment the Vβ is at least 70%, at least 75%, at least 80%, at least 85%, at least 90%, at least 95%, at least 96%, at least 97%, at least 98%, or at least 99% identical to SEQ ID NO:47.

In one embodiment the Cα comprises or consists of the amino acid sequence of SEQ ID NO: 22. In another embodiment the Cα is at least 70%, at least 75%, at least 80%, at least 85%, at least 90%, at least 95%, at least 96%, at least 97%, at least 98%, or at least 99% identical to SEQ ID NO:22.

In one embodiment the Cβ comprises or consists of the amino acid sequence of SEQ ID NO: 23. In another embodiment the Cβ is at least 70%, at least 75%, at least 80%, at least 85%, at least 90%, at least 95%, at least 96%, at least 97%, at least 98%, or at least 99% identical to SEQ ID NO:23.

In one embodiment the Cβ comprises or consists of the amino acid sequence of SEQ ID NO: 24. In another embodiment the Cβ is at least 70%, at least 75%, at least 80%, at least 85%, at least 90%, at least 95%, at least 96%, at least 97%, at least 98%, or at least 99% identical to SEQ ID NO:24.

In one embodiment the TCRα chain or fragment thereof and the first CD3ζ chain or fragment thereof comprise or consist of the amino acid sequence of SEQ ID NO: 25. In another embodiment the TCRα chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:25.

In one embodiment the TCRα chain or fragment thereof and the first CD3ζ chain or fragment thereof comprise or consist of the amino acid sequence of SEQ ID NO: 37. In another embodiment the TCRα chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:37.

In one embodiment the TCRα chain or fragment thereof and the first CD3ζ chain or fragment thereof comprise or consist of the amino acid sequence of SEQ ID NO: 48. In another embodiment the TCRα chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:48.

In one embodiment the TCRβ or fragment thereof and the first CD3ζ chain or fragment thereof comprise or consist of the amino acid sequence of SEQ ID NO: 26. In another embodiment the TCRβ chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:26.

In one embodiment the TCRβ or fragment thereof and the first CD3ζ chain or fragment thereof comprise or consist of the amino acid sequence of SEQ ID NO: 27. In another embodiment the TCRβ chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:27.

In one embodiment the TCRβ or fragment thereof and the first CD3ζ chain or fragment thereof comprise or consist of the amino acid sequence of SEQ ID NO: 38. In another embodiment the TCRβ chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:38.

In one embodiment the TCRβ or fragment thereof and the first CD3ζ chain or fragment thereof comprise or consist of the amino acid sequence of SEQ ID NO: 39. In another embodiment the TCRβ chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:39.

In one embodiment the TCRβ or fragment thereof and the first CD3ζ chain or fragment thereof comprise or consist of the amino acid sequence of SEQ ID NO: 49. In another embodiment the TCRβ chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:49.

In one embodiment the TCRβ or fragment thereof and the first CD3ζ chain or fragment thereof comprise or consist of the amino acid sequence of SEQ ID NO: 50. In another embodiment the TCRβ chain or fragment thereof and the first CD3ζ chain or fragment thereof are at least 70%, at least 75%, at least 80%, at least 85%, at least 90%, at least 95%, at least 96%, at least 97%, at least 98%, or at least 99% identical to SEQ ID NO:50.

In one embodiment the TCRα chain or fragment thereof and the first CD3ζ chain or fragment thereof with a signal sequence comprise or consist of one of the amino acid sequences of SEQ ID NO: 58, 60, 62, 64, or 66. In another embodiment the TCRα chain or fragment thereof and the first CD3ζ chain or fragment thereof with a signal sequence are at least 70%, at least 75%, at least 80%, at least 85%, at least 90%, at least 95%, at least 96%, at least 97%, at least 98%, or at least 99% identical to one of the amino acid sequences SEQ ID NO: 58, 60, 62, 64, or 66.

In one embodiment the TCRβ chain or fragment thereof and the first CD3ζ chain or fragment thereof with a signal sequence comprise or consist of one of the amino acid sequences of SEQ ID NO: 57, 59, 61, 63, or 65. In another embodiment the TCRβ chain or fragment thereof and the first CD3ζ chain or fragment thereof with a signal sequence are at least 70%, at least 75%, at least 80%, at least 85%, at least 90%, at least 95%, at least 96%, at least 97%, at least 98%, or at least 99% identical to one of the amino acid sequences SEQ ID NO: 57, 59, 61, 63, or 65.

In a natural TCR complex certain CD3 chains are responsible for the dimerization of the TCR alpha and the TCR beta chains. Since the TCR construct according to the invention does not comprise all CD3 chains also the ones responsible for the dimerization might be lacking. In order to compensate for the absence of the responsible CD3 chains the TCR construct according to the invention can comprise a dimerization bridge (disulfide bond by replacing Thr163 of Cα and Ser167 of Cβ with cysteine residues) in one embodiment. The ,Boulter cysteine bridge" as disclosed herein is a structural modification in T-cell receptors (TCRs) that enhances their stability and expression. This modification involves introducing an additional disulfide bond between the constant domains of the TCR α and β chains. Specifically, Boulter et al. 2003 engineered an interchain disulfide bond by replacing Thr163 of Cα and Ser167 of Cβ with cysteine residues. This additional bond promotes preferential pairing of the introduced TCR chains, reducing mispairing with endogenous TCR subunits.

Preferably the TCR construct has an introduced disulfide bond between residues of the constant regions, as described, for example, in WO 03/020763. TCRs may contain a disulfide bond corresponding to that found in nature between the respective α and β constant regions (Cα and Cβ) or γ and δ constant regions (Cγ and Cδ), additionally or alternatively a non-native disulfide bond may be present.

In order to be able to track the TCR construct it may comprise a certain tag. A person skilled in the art would be well aware of including and using appropriate tags to track the TCR construct.

In one embodiment the TCR construct comprises at least one epitope tag. In one embodiment the epitope tag is located within one of the constant regions C alpha, C beta, C gamma or C delta. In one embodiment the epitope tag is a 9-mer epitope. In one embodiment the 9-mer epitope tag comprises or consists of the amino acid sequence GEVPKDRFS (SEQ ID NO: 28). The epitope tag may be flanked by one or two linkers. Appropriate linkers are described elsewhere herein and would be well known to a person skilled in the art. In one example the epitope is flanked by a GSG linker on both the N- and C-terminal end. In another embodiment the epitope tag is the epitope tag as described in EP24171426.0 and EP24199974.7, which are incorporated by reference herein.

In one embodiment the functional TCR construct comprises
a) a first fusion protein comprising a fragment of a TCR α chain, a fragment of a first CD3ζ chain, wherein the TCR α chain comprises
   i. a first signal sequence, wherein the first signal sequence is a NK cell signal sequence from the NKp46 protein,
   ii. a variable region (Vα), and
   iii. a constant region (Cα),
   and
b) a second fusion protein comprising a fragment of a TCR β chain, a fragment of a second CD3ζ chain, wherein the fragment of a TCR β chain comprises
   i. a second signal sequence, wherein the second signal sequence is a NK cell signal sequence form the NKp46 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain and a DAP-10 signaling domain, wherein the DAP-10 signaling domain is connected to the fragment of a first CD3ζ chain, wherein the TCR α chain comprises
   i. a first signal sequence, wherein the first signal sequence is a NK cell signal sequence from the NKp46 protein,
   ii. a variable region (Vα), and
   iii. a constant region (Cα),
   and
b) a second fusion protein comprising a fragment of a TCR β chain, a fragment of a second CD3ζ chain, wherein the fragment of a TCR β chain comprises
   i. a second signal sequence, wherein the second signal sequence is a NK cell signal sequence form the NKp46 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain, wherein the TCR α chain comprises
   i. a first signal sequence, wherein the first signal sequence is a NK cell signal sequence from the NKp46 protein,
   ii. a variable region (Vα), and
   iii. a constant region (Cα),
   and
b) a second fusion protein comprising a fragment of a TCR β chain, a fragment of a second CD3ζ chain and a DAP-10 signaling domain, wherein the DAP-10 signaling domain is connected to the fragment of a second CD3ζ chain, wherein the fragment of a TCR β chain comprises
   i. a second signal sequence, wherein the second signal sequence is a NK cell signal sequence form the NKp46 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain and a DAP-10 signaling domain, wherein the DAP-10 signaling domain is connected to the fragment of a first CD3ζ chain, wherein the TCR α chain comprises
   i. a first signal sequence, wherein the first signal sequence is a NK cell signal sequence from the NKp46 protein,
   ii. a variable region (Vα), and
   iii. a constant region (Cα),
   and
b) a second fusion protein comprising a fragment of a TCR β chain, a fragment of a second CD3ζ chain and a DAP-10 signaling domain, wherein the DAP-10 signalling domain is connected to the fragment of a second CD3ζ chain, wherein the fragment of a TCR β chain comprises
   i. a second signal sequence, wherein the second signal sequence is a NK cell signal sequence form the NKp46 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain, wherein the TCR α chain comprises
   i. a first signal sequence, wherein the first signal sequence is a NK cell signal sequence from the NKp44 protein,
   ii. a variable region (Vα), and
   iii. a constant region (Cα),
   and
b) a second fusion protein comprising a fragment of a TCR β chain, a fragment of a second CD3ζ chain, wherein the fragment of a TCR β chain comprises
   i. a second signal sequence, wherein the second signal sequence is a NK cell signal sequence form the NKp44 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain and a DAP-10 signaling domain, wherein the DAP-10 signaling domain is connected to the fragment of a first CD3ζ chain, wherein the TCR α chain comprises
   i. a first signal sequence, wherein the first signal sequence is a NK cell signal sequence from the NKp44 protein,
   ii. a variable region (Vα), and
   iii. a constant region (Cα),
   and
b) a second fusion protein comprising a fragment of a TCR β chain, a fragment of a second CD3ζ chain, wherein the fragment of a TCR β chain comprises
   i. a second signal sequence, wherein the second signal sequence is a NK cell signal sequence form the NKp44 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain, wherein the TCR α chain comprises
   i. a first signal sequence, wherein the first signal sequence is a NK cell signal sequence from the NKp44 protein,
   ii. a variable region (Vα), and
   iii. a constant region (Cα),
   and
b) a second fusion protein comprising a fragment of a TCR β chain, a fragment of a second CD3ζ chain and a DAP-10 signaling domain, wherein the DAP-10 signaling domain is connected to the fragment of a second CD3ζ chain, wherein the fragment of a TCR β chain comprises
   i. a second signal sequence, wherein the second signal sequence is a NK cell signal sequence form the NKp44 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain and a DAP-10 signaling domain, wherein the DAP-10 signaling domain is connected to the fragment of a first CD3ζ chain, wherein the TCR α chain comprises
   i. a first signal sequence, wherein the first signal sequence is a NK cell signal sequence from the NKp44 protein,
   ii. a variable region (Vα), and
   iii. a constant region (Cα),
   and
b) a second fusion protein comprising a fragment of a TCR β chain, a fragment of a second CD3ζ chain and a DAP-10 signaling domain, wherein the DAP-10 signalling domain is connected to the fragment of a second CD3ζ chain, wherein the fragment of a TCR β chain comprises
   i. a second signal sequence, wherein the second signal sequence is a NK cell signal sequence form the NKp44 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain, wherein the TCR α chain comprises
   i. a first signal sequence, wherein the first signal sequence is a NK cell signal sequence from the NKp30 protein,
   ii. a variable region (Vα), and
   iii. a constant region (Cα),
   and
b) a second fusion protein comprising a fragment of a TCR β chain, a fragment of a second CD3ζ chain, wherein the fragment of a TCR β chain comprises
   i. a second signal sequence, wherein the second signal sequence is a NK cell signal sequence form the NKp30 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain and a DAP-10 signaling domain, wherein the DAP-10 signaling domain is connected to the fragment of a first CD3ζ chain, wherein the TCR α chain comprises
   i. a first signal sequence, wherein the first signal sequence is a NK cell signal sequence from the NKp30 protein,
   ii. a variable region (Vα), and
   iii. a constant region (Cα),
   and
b) a second fusion protein comprising a fragment of a TCR β chain, a fragment of a second CD3ζ chain, wherein the fragment of a TCR β chain comprises
   i. a second signal sequence, wherein the second signal sequence is a NK cell signal sequence form the NKp30 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain, wherein the TCR α chain comprises
   i. a first signal sequence, wherein the first signal sequence is a NK cell signal sequence from the NKp30 protein,
   ii. a variable region (Vα), and
   iii. a constant region (Cα),
   and
b) a second fusion protein comprising a fragment of a TCR β chain, a fragment of a second CD3ζ chain and a DAP-10 signaling domain, wherein the DAP-10 signaling domain is connected to the fragment of a second CD3ζ chain, wherein the fragment of a TCR β chain comprises
   i. a second signal sequence, wherein the second signal sequence is a NK cell signal sequence form the NKp30 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain and a DAP-10 signaling domain, wherein the DAP-10 signaling domain is connected to the fragment of a first CD3ζ chain, wherein the TCR α chain comprises
   i. a first signal sequence, wherein the first signal sequence is a NK cell signal sequence from the NKp30 protein,
   ii. a variable region (Vα), and
   iii. a constant region (Cα),
   and
b) a second fusion protein comprising a fragment of a TCR β chain, a fragment of a second CD3ζ chain and a DAP-10 signaling domain, wherein the DAP-10 signalling domain is connected to the fragment of a second CD3ζ chain, wherein the fragment of a TCR β chain comprises
   i. a second signal sequence, wherein the second signal sequence is a NK cell signal sequence form the NKp30 protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain, wherein the TCR α chain comprises
   i. a first signal sequence, wherein the first signal sequence is a NK cell signal sequence from the CD16A protein,
   ii. a variable region (Vα), and
   iii. a constant region (Cα),
   and
b) a second fusion protein comprising a fragment of a TCR β chain, a fragment of a second CD3ζ chain, wherein the fragment of a TCR β chain comprises
   i. a second signal sequence, wherein the second signal sequence is a NK cell signal sequence form the CD16A protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain and a DAP-10 signaling domain, wherein the DAP-10 signaling domain is connected to the fragment of a first CD3ζ chain, wherein the TCR α chain comprises
   i. a first signal sequence, wherein the first signal sequence is a NK cell signal sequence from the CD16A protein,
   ii. a variable region (Vα), and
   iii. a constant region (Cα),
   and
b) a second fusion protein comprising a fragment of a TCR β chain, a fragment of a second CD3ζ chain, wherein the fragment of a TCR β chain comprises
   i. a second signal sequence, wherein the second signal sequence is a NK cell signal sequence form the CD16A protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain, wherein the TCR α chain comprises
   i. a first signal sequence, wherein the first signal sequence is a NK cell signal sequence from the CD16A protein,
   ii. a variable region (Vα), and
   iii. a constant region (Cα),
   and
b) a second fusion protein comprising a fragment of a TCR β chain, a fragment of a second CD3ζ chain and a DAP-10 signaling domain, wherein the DAP-10 signaling domain is connected to the fragment of a second CD3ζ chain, wherein the fragment of a TCR β chain comprises
   i. a second signal sequence, wherein the second signal sequence is a NK cell signal sequence form the CD16A protein,
   ii. a variable region (Vβ),
   iii. a constant region (Cβ).

In one embodiment the functional TCR construct comprises
a) a first fusion protein comprising a fragment of a TCR α chain, a fragment of a first CD3ζ chain and a DAP-10 signaling domain, wherein the DAP-10 signaling domain is connected to the fragment of a first CD3ζ chain, wherein the TCR α chain comprises
   i. a first signal sequence, wherein the first signal sequence is a NK cell signal sequence from the CD16A protein,
   ii. a variable region (Vα), and
   iii. a constant region (Cα),
   and
b) a second fusion protein comprising a fragment of a TCR β chain, a fragment of a second CD3ζ chain and a DAP-10 signaling domain, wherein the DAP-10 signalling domain is connected to the fragment of a second CD3ζ chain, wherein the fragment of a TCR β chain comprises
   i. a second signal sequence, wherein the second signal sequence is a NK cell signal sequence form the CD16A protein,
   ii. a variable region (Vβ),
   iii. a constant region (Cβ).

In a preferred embodiment the TCR α and TCR β chain fragment do not comprise the transmembrane and cytoplasmic domain or fragments thereof.

In a preferred embodiment the TCR α and TCR β chain fragment consist of the extracellular domain or of fragments thereof.

In a preferred embodiment the CD3ζ chain does not comprise the extracellular domain or fragments thereof.

In a preferred embodiment the CD3ζ chain consists of the transmembrane and cytoplasmic domain or fragments thereof.

In a preferred embodiment the TCR α and TCR β chain fragment do not comprise the transmembrane and cytoplasmic domain or fragments thereof and the CD3ζ chain does not comprise the extracellular domain or fragments thereof.

In a preferred embodiment the TCR α and TCR β chain fragment consist of the extracellular domain or of fragments thereof and the CD3ζ chain consists of the transmembrane and cytoplasmic domain or fragments thereof.

Definitions for the domains/regions/chains/sequences, the TCR construct, the tag, the dimerization bridge, the fusion protein, the cells, etc. are provided elsewhere herein and equally apply here. Definitions, embodiments, examples etc. herein for one aspect of the invention equally apply for all the other aspects of the invention. Unless it is apparent from the context, each of the embodiments listed above can be applied for use in any of the aspects of the invention.

### Nucleic acids, vectors, polypeptides, and cells,

Another aspect of the invention refers to a nucleic acid encoding a TCR construct as described herein or encoding the polynucleotide encoding a TCR construct as described herein.

**Table 1: signal sequences and exemplary TCR construct sequences**

| **TCR construct** | **Amino acid sequence** | **Nucleic acid sequence** |
|---|---|---|
| NKp46 signal sequence | SEQ ID NO: 1 | SEQ ID NO: 76 and 77 |
| NKp30 signal sequence | SEQ ID NO: 2 | SEQ ID NO: 78 and 79 |
| NKp44 signal sequence | SEQ ID NO: 3 | SEQ ID NO: 80 and 81 |
| CD16A signal sequence | SEQ ID NO: 4 | SEQ ID NO: 94 |
| Fusion protein NYESO1 TCRβ chain and CD3ζ chain sequence (N Kp46 signal sequence, with 9-mer epitope tag) | SEQ ID NO: 57 | SEQ ID NO: 71 |
| Fusion protein NYESO1 TCRα chain and CD3ζ chain sequence (NKp46 signal sequence) | SEQ ID NO: 58 | SEQ ID NO: 72 |
| Fusion protein NYESO1 TCRβ chain, CD3ζ chain and DAP10 sequence (NKp46 signal sequence, with 9-mer epitope tag) | SEQ ID NO: 59 | SEQ ID NO: 73 |
| Fusion protein NYESO1 TCRα chain, CD3ζ chain and DAP10 sequence (NKp46 signal sequence) | SEQ ID NO: 60 | SEQ ID NO: 75 |
| Fusion protein NYESO1 TCRβ chain and CD3ζ chain | SEQ ID NO: 61 | SEQ ID NO: 88 |
| sequence (NKp30 signal sequence, with 9-mer epitope tag) | | |
| Fusion protein NYESO1 TCRα chain and CD3ζ chain sequence (NKp30 signal sequence) | SEQ ID NO: 62 | SEQ ID NO: 89 |
| Fusion protein NYESO1 TCRβ chain and CD3ζ chain sequence (NKp44 signal sequence, with 9-mer epitope tag) | SEQ ID NO: 63 | SEQ ID NO: 90 |
| Fusion protein NYESO1 TCRα chain and CD3ζ chain sequence (NKp44 signal sequence) | SEQ ID NO: 64 | SEQ ID NO: 91 |
| Fusion protein NYESO1 TCRβ chain and CD3ζ chain sequence (TCRβ signal sequence, with 9-mer epitope tag) | SEQ ID NO: 65 | SEQ ID NO: 92 |
| Fusion protein NYESO1 TCRα chain and CD3ζ chain sequence (TCRα signal sequence) | SEQ ID NO: 66 | SEQ ID NO: 93 |

The disclosure also provides an isolated or purified nucleic acid comprising a nucleotide sequence which is complementary to the nucleotide sequence of any of the nucleic acids described herein or a nucleotide sequence which hybridizes under stringent conditions to the nucleotide sequence of any of the nucleic acids described herein.

The skilled person understands that also the nucleic acid encoding the TCR may be modified. Useful modifications in the overall nucleic acid sequence include codon optimization of the sequence. Alterations may be made which lead to conservative substitutions within the expressed amino acid sequence. These variations can be made in complementarity determining and non-complementarity determining regions of the amino acid sequence of the TCR chain that do not affect function. Usually, additions and deletions should not be performed in the CDR3 region.

Another embodiment refers to a vector comprising the nucleic acid encoding the TCR as described herein.

The vector is preferably a plasmid, shuttle vector, phagemide, cosmid, expression vector, retroviral vector, adenoviral vector or particle and/or vector to be used in gene therapy. Preferably, the vector is an expression vector. More preferably, the vector is a retroviral, more specifically a gamma-retroviral or lentiviral vector.

Another aspect of the invention refers to a (receptor) cell expressing the TCR construct as described herein.

In some embodiments, the cell is isolated or non-naturally occurring.

In specific embodiments, the cell may comprise the nucleic acid encoding the TCR construct as described herein or the vector comprising said nucleic acid.

Some embodiments refer to a cell comprising:
a) an expression vector which comprises at least one nucleic acid as described herein, or
b) a first expression vector which comprises a nucleic acid encoding the TCR α chain and the first CD3ζ chain as described herein, and a second expression vector which comprises a nucleic acid encoding the TCR β chain and the second CD3ζ as described herein.

In such embodiments b), the described at least one epitope tag may be present in said alpha chain and/or in said beta chain.

In another aspect, the present invention relates to an isolated polypeptide comprising a functional portion of the TCR construct of the present invention.

Some embodiments refer to a cell comprising a first expression vector which comprises a nucleic acid encoding the gamma chain of the TCR construct as described herein, and a second expression vector which comprises a nucleic acid encoding the delta chain of a TCR construct as described herein.

In a preferred embodiment the recipient cell is a NK cell, a B cell, a T cell, a macrophage, a neutrophil, or an innate lymphoid cell.

In a preferred embodiment the cell is an NK cell.

The advantages of using a TCR construct according to the invention in NK cells in particular has been described elsewhere herein.

In one embodiment, in order to make use of the cell specific environment and genomics and in order to obtain a reliable and efficient TCR construct expression, the first and/or second signal sequence(s), the first and/or second CD3ζ chain or fragment thereof, and/or any cell specific sequence enhancing the activation potential of the TCR construct originate from the (receptor) cell.

In one embodiment the (receptor) cell is an NK cell and the first and/or second signal sequence(s) is/are (a) NK cell signal sequence(s), preferably from a protein selected from the group consisting of NKp46, NKp44, NKp30, and CD16A.

In one embodiment the (receptor) cell is an NK cell and the first and/or second signal sequence(s) is/are from the NKp46 protein comprising or consisting of the amino acid sequence SEQ ID NO: 1. In another embodiment the NKp46 signal peptide is at least 70%, at least 75%, at least 80%, at least 85%, at least 90%, at least 95%, at least 96%, at least 97%, at least 98%, or at least 99% identical to SEQ ID NO: 1. Same applies *mutatis mutandis* for the signal sequences of proteins NKp44, NKp30, and CD16A.

In one embodiment the (receptor) cell is an NK cell and the first and second signal sequences are from the NKp46 protein comprising or consisting of the amino acid sequence SEQ ID NO: 1. In another embodiment the NKp46 signal peptide is at least 70%, at least 75%, at least 80%, at least 85%, at least 90%, at least 95%, at least 96%, at least 97%, at least 98%, or at least 99% identical to SEQ ID NO: 1. Same applies *mutatis mutandis* for the signal sequences of proteins NKp44, NKp30, and CD16A.

Definitions for the domains/regions/chains/sequences, the TCR construct, the tag, the dimerization bridge, the fusion protein, the cells, etc. are provided elsewhere herein and equally apply here. Definitions, embodiments, examples etc. herein for one aspect of the invention equally apply for all the other aspects of the invention. Unless it is apparent from the context, each of the embodiments listed above can be applied for use in any of the aspects of the invention.

### Method of producing a TCR construct

According to another aspect of the invention a method of producing a TCR construct or fragment thereof according to the invention is provided, said method comprises the step of culturing a (recipient) cell comprising a nucleic acid encoding the TCR as herein described.

Methods and means for culturing the recipient cell under suitable conditions to express the encoded TCR construct are generally known and can be applied accordingly in the context of the present invention.

Definitions for the domains/regions/chains/sequences, the TCR construct, the tag, the dimerization bridge, the fusion protein, the cells, etc. are provided elsewhere herein and equally apply here. Definitions, embodiments, examples etc. herein for one aspect of the invention equally apply for all the other aspects of the invention. Unless it is apparent from the context, each of the embodiments listed above can be applied for use in any of the aspects of the invention.

### Pharmaceutical compositions, medical treatments and kits

Another aspect of the invention refers to a pharmaceutical composition comprising the TCR construct as described herein, the nucleic acid encoding the TCR construct, the vector comprising said nucleic acid, and/or the cell comprising said TCR construct as described herein.

Those active components of the present invention are preferably used in such a pharmaceutical composition, in doses mixed with an acceptable carrier or carrier material, that the disease can be treated or at least alleviated. Such a composition can (in addition to the active component and the carrier) include filling material, salts, buffer, stabilizers, solubilizers and other materials, which are known state of the art. The choice of the carrier is dependent on the application.

The term "pharmaceutically acceptable" defines a non-toxic material, which does not interfere with effectiveness of the biological activity of the active component. Further, the phrase "pharmaceutically acceptable" refers to those compounds, materials, compositions, and/or dosage forms which are, within the scope of sound medical judgment, suitable for use in contact with the tissues of human beings or animals without excessive toxicity, irritation, allergic response, or other problem or complication, commensurate with a reasonable benefit/risk ratio.

The pharmaceutical composition may contain additional components which enhance the activity of the active component or which supplement the treatment. Such additional components and/or factors can be part of the pharmaceutical composition to achieve synergistic effects or to minimize adverse or unwanted effects.

Techniques for the formulation or preparation and application/medication of active components of the present invention are published in "Remington's Pharmaceutical Sciences", Mack Publishing Co., Easton, PA, latest edition.

The pharmaceutical composition comprising the TCR construct or fragment thereof may be for administration to the subject by any suitable route. An appropriate application is a parenteral application, for example intramuscular, subcutaneous, intramedullary injections as well as intrathecal, direct intraventricular, intravenous, intranodal, intraperitoneal or intratumoral injections. The intravenous injection is the preferred treatment of a patient.

According to one embodiment mRNA or DNA encoding the TCR construct according to the invention is directly administered.

According to a preferred embodiment, the pharmaceutical composition is an infusion or an injection.

An injectable composition is a pharmaceutically acceptable fluid composition comprising at least one active ingredient, eg. an expanded recipient cell population (for example autologous or allogenic to the patient to be treated) expressing a TCR construct. The active ingredient is usually dissolved or suspended in a physiologically acceptable carrier, and the composition can additionally comprise minor amounts of one or more non-toxic auxiliary substances, such as emulsifying agents, preservatives, and pH buffering agents and the like. Such injectable compositions that are useful for use with the fusion proteins of this disclosure are conventional; appropriate formulations are well known to those of ordinary skill in the art.

Typically, the pharmaceutical composition comprises at least one pharmaceutically acceptable carrier, diluent or excipient. A particularly useful carrier is saline, phosphate buffer saline (PBS) and/or polysorbate.

Pharmaceutical compositions may further routinely contain pharmaceutically acceptable concentrations of salt, buffering agents, preservatives, compatible carriers, supplementary immune potentiating agents such as adjuvants and cytokines and optionally other therapeutic agents. Suitably, the pharmaceutical composition may comprise a salt selected from the group consisting of sodium phosphate and sodium chloride.

The composition may also include antioxidants and/or preservatives.

Accordingly, another aspect of the invention refers to the TCR construct as described herein, the cell as described herein, the nucleic acid as described herein, and/or the vector as described herein, and the pharmaceutical composition as described herein for use as a medicament, for use as a screening agent or for the use in the therapy of malignancies.

Hence, in some embodiments the invention refers to the TCR construct as described herein, the cell as described herein, the nucleic acid as described herein, and/or the vector as described herein, and the pharmaceutical composition as described herein for use as a medicament.

In some embodiments the invention refers to the TCR construct as described herein, the cell as described herein, the nucleic acid as described herein, and/or the vector as described herein, and the pharmaceutical composition as described herein for use in the treatment of cancer.

In one embodiment the cancer is a hematological cancer or a solid tumor.

In one embodiment the cancer is a solid tumor.

Hematological cancers, also called blood cancers, are cancers which do not form solid tumors and therefore are dispersed in the body. Examples of hematological cancers are leukemia, lymphoma or multiple myeloma. There are two major types of solid tumors, sarcomas and carcinomas. Sarcomas are for example tumors of the blood vessel, bone, fat tissue, ligament, lymph vessel, muscle or tendon. In a specific embodiment the cancer is a solid tumor.

In one embodiment, the cancer is selected from the group consisting of prostate cancer, uterine cancer, thyroid cancer, testicular cancer, renal cancer, pancreatic cancer, ovarian cancer, esophageal cancer, non-small-cell lung cancer, lung adenocarcinoma, squamous cell carcinoma, non-Hodgkin's lymphoma, multiple myeloma, melanoma, hepatocellular carcinoma, head and neck cancer, gastric cancer, endometrial cancer, cervical cancer, colorectal cancer, stomach adenocarcinoma, cholangiocarcinoma, breast cancer, bladder cancer, myeloid leukemia and acute lymphoblastic leukemia, carcinoma, sarcoma, glioblastoma, astrocytoma or osteosarcoma.

In various embodiments, the hematological cancer may be selected from the group consisting of, non-Hodgkin's lymphoma (NHL), Hodgkin's lymphoma (HL), multiple myeloma, acute myeloid leukemia (AML) and acute lymphoblastic leukemia (ALL), mixed phenotype acute leukemia (MPAL), chronic myeloid leukemia (CML), B cell polymorphic lymphoma, hairy cell leukemia, chronic lymphocytic leukemia (CLL), small lymphocytic lymphoma (SLL), central nervous system lymphoma, CD37+ dendritic cell lymphoma, lymphoplasmatic lymphoma, splenic marginal zone lymphoma, plasma cell myeloma, extraosseuos plasmacytoma, extra-nodal marginal zone B-cell lymphoma of mucosa-associated lymphoid tissue (MALT tissue), nodal marginal zone B-cell lymphoma, follicular lymphoma, mantel cell lymphoma, diffuse large B-cell lymphoma, mediastinal (thymic) large B-cell lymphoma, precursor B-lymphoblastic lymphoma, immunoblastic large cell lymphoma, intravascular large B-cell lymphoma, primary effusion lymphoma, Burkitt's lymphoma/leukemia, B-cell proliferations of uncertain malignant potential, lymphomatoid granulomatosis, post-transplant lymphoproliferative disorder. Other hematological cancers or disorders include myelodysplastic disorder.

The TCR construct as described herein and compositions comprising the TCR construct as described herein can be utilized in methods and compositions for adoptive immunotherapy in accordance with known techniques, or variations thereof that will be apparent to those skilled in the art based on the instant disclosure.

In some embodiments, the (receptor) cells are formulated by first harvesting them from their culture medium, and then washing and concentrating the cells in a medium and container system suitable for administration (a "pharmaceutically acceptable" carrier) in a treatment-effective amount. Suitable infusion medium can be any isotonic medium formulation, typically normal saline, Normosol R (Abbott) or Plasma-Lyte A (Baxter), but also 5% dextrose in water or Ringer's lactate can be utilized. The infusion medium can be supplemented with human serum albumin.

The number of cells for an effective treatment in the composition is typically greater than 10 cells, and up to 106, up to and including 108 or 109 cells and can be more than 1010 cells. The number of cells will depend upon the ultimate use for which the composition is intended as will the type of cells included therein. For uses provided herein, the cells are generally in a volume of a litre or less, can be 500 ml or less, even 250 ml or 100 ml or less. Hence the density of the desired cells is typically greater than 106 cells/ml and generally is greater than 107 cells/ml, generally 108 cells/ml or greater. The clinically relevant number of immune cells can be apportioned into multiple infusions that cumulatively equal or exceed 109, 1010 or 1011 cells. Pharmaceutical compositions provided herein can be in various forms, e.g., in solid, liquid, powder, aqueous, or lyophilized form. Examples of suitable pharmaceutical carriers are known in the art. Such carriers and/or additives can be formulated by conventional methods and can be administered to the subject at a suitable dose. Stabilizing agents such as lipids, nuclease inhibitors, polymers, and chelating agents can preserve the compositions from degradation within the body. In a composition intended to be administered by injection, one or more of a surfactant, preservative, wetting agent, dispersing agent, suspending agent, buffer, stabilizer and isotonic agent may be included.

The TCR constructs as described herein, or the vectors comprising a nucleotide sequence encoding a TCR constructs provided herein, can be packaged as kits. Kits can optionally include one or more components such as instructions for use, devices, and additional reagents, and components, such as tubes, containers and syringes for practice of the methods. Exemplary kits can include the nucleic acids encoding the recombinant TCR constructs, and can optionally include instructions for use, a device for administering the compositions to a subject, and a device for administering the compositions to a subject.

In further embodiments, the kits herein may comprise reagents for detecting the TCR constructs disclosed herein, such as antibodies or other binding molecules.

A kit may also contain instructions. Instructions typically include a tangible expression describing the components included in the kit, and methods for administration, including methods for determining the proper state of the subject, the proper dosage amount, and the proper administration method. Instructions can also include guidance for monitoring the subject over the duration of the treatment time.

Kits provided herein also can include a device for administering a composition described herein to a subject. Any of a variety of devices known in the art for administering medications or vaccines can be included in the kits provided herein. Exemplary devices include, but are not limited to, a hypodermic needle, an intravenous needle, a catheter, a needle-less injection device, an inhaler, and a liquid dispenser, such as an eyedropper.

Any of a variety of devices known in the art for administering medications to a subject can be included in the kits provided herein.

Definitions for the domains/regions/chains/sequences, the TCR construct, the tag, the dimerization bridge, the fusion protein, the cells, etc. are provided elsewhere herein and equally apply here. Definitions, embodiments, examples etc. herein for one aspect of the invention equally apply for all the other aspects of the invention. Unless it is apparent from the context, each of the embodiments listed above can be applied for use in any of the aspects of the invention.

### Uses

The TCR constructs described herein can be used as a medicament.

The TCR constructs described herein can be used for treating cancer, as screening agent, or for the therapy of malignancies.

The TCR constructs described herein can be used in the manufacture of a medicament for treatment.

The TCR constructs described herein can be used in adoptive immunotherapy.

Accordingly, in another aspect, the present invention provides a TCR construct for use as a medicament.

Accordingly, in another aspect, the present invention provides a TCR construct for use in treating cancer.

In one embodiment the TCR construct described herein, the nucleic acid described herein, the vector described herein, the cell described herein, or the pharmaceutical composition described herein are used in adoptive immunotherapy.

Details with regards to the TCR constructs, treatment, therapies, and the diseases or disorders (cancer types) are described elsewhere herein and equally apply here.

A TCR construct of the invention can be for use or administration alone or in combination with at least one or more other compounds. Administration "in combination with" at least one or more other compounds includes simultaneous (concurrent) and consecutive administration in any order. "Combined use" and "combination" in the context of the invention also includes a pharmaceutical product comprising both the TCR construct and at least one or more other compounds, as discrete separate dosage forms, in separate containers or e. g. in blisters containing both types of drugs in discrete solid dosage units, e.g. in a form in which the dosage units which have to be taken together or which have to be taken within one day are grouped together in a manner which is convenient for the patient. Said pharmaceutical product itself or as a part of a kit may contain instructions for the simultaneous, sequential or separate administration of the discrete separate dosage units, to a subject in need thereof.

Definitions for the domains/regions/chains/sequences, the TCR construct, the tag, the dimerization bridge, the fusion protein, the cells, etc. are provided elsewhere herein and equally apply here. Definitions, embodiments, examples etc. herein for one aspect of the invention equally apply for all the other aspects of the invention. Unless it is apparent from the context, each of the embodiments listed above can be applied for use in any of the aspects of the invention.

### General definitions

Throughout the description and claims of this specification, the words "comprise" and "contain" and variations of them mean "including but not limited to", and they are not intended to (and do not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Also, as used herein, the singular terms "a", "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

Features, integers, characteristics, compounds, chemical moieties or groups described in conjunction with a particular aspect, embodiment or example of the invention are to be understood to be applicable to any other aspect, embodiment or example described herein unless incompatible therewith.

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionary of many of the terms used in the invention. Although any methods and materials similar or equivalent to those described herein find use in the practice of the present invention, the preferred methods and materials are described herein. Accordingly, the terms defined immediately below are more fully described by reference to the specification as a whole. It is to be understood that this invention is not limited to the particular methodology, protocols, and reagents described, as these may vary, depending upon the context they are used by those of skill in the art.

The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

Herein single amino acid codes are used to recite peptide or polypeptide sequences based on naturally occurring amino acid residues (i.e. encoded by the genetic code) and selected from the group consisting of: alanine (Ala, A); arginine (Arg, R); asparagine (Asn, N); aspartic acid (Asp, D); cysteine (Cys, C); glutamine (Gln, Q); glutamic acid (Glu, E); glycine (Gly, G); histidine (His, H); isoleucine (Ile, I): leucine (Leu; L); lysine (Lys, K); methionine (Met, M); phenylalanine (Phe, F); proline (Pro, P); serine (Ser, S); threonine (Thr, T); tryptophan (Trp, W); tyrosine (Tyr, Y); and valine (Val, V).

The terms alpha, beta, gamma, delta, epsilon and zeta and the symbols α, β, γ, δ, ε, and ζ are used interchangeably herein.

The term "T-cell receptor" is used herein in the conventional sense to mean a molecule typically capable of recognising a peptide when presented by an MHC molecule. The molecule may be a heterodimer of two chains α and β (or optionally γ and δ) or it may be a single chain TCR construct. The TCR α chain comprises variable (V), joining (J) and constant (C) regions. The TCR b chain comprises variable (V), diversity (D), joining (J) and constant (C) regions. The rearranged V(D)J regions of both the TCR α and the TCR β chain contain hypervariable regions (CDR, complementarity determining regions), among which the CDR3 region determines the specific epitope recognition. At the C-terminal region both TCR α chain and TCR β chain contain a hydrophobic transmembrane domain and end in a short cytoplasmic tail. Typically, the TCR is a heterodimer of one α chain and one β chain or of one γ chain and one δ chain. This heterodimer can bind to MHC molecules presenting a peptide.

The TCR loci and genes are named using the International Immunogenetics (IMGT) TCR nomenclature (IMGT Database, www. IMGT.org; Giudicelli, V., et al., IMGT/LIGM-DB, the IMGT® comprehensive database of immunoglobulin and T cell receptor nucleotide sequences, Nucl. Acids Res., 34, D781-D784 (2006). PMID: 16381979; T cell Receptor Factsbook, LeFranc and LeFranc, Academic Press ISBN 0-12- 441352-8).

The term "variable TCR α region", "variable region alpha", "variable region (Vα)", "Vα" or "TCR α variable chain" in the context of the invention refers to the variable region of a TCR α chain or fragment thereof. The term does not imply that it has to be the full length variable region. Same applies to the β, γ, and δ chains correspondingly.

The term "constant TCR α region", "constant region alpha", "constant region (Vα)", "Cα" or "TCR α constant chain" in the context of the invention refers to the constant region of a TCR α chain or fragment thereof. The term does not imply that it has to be the full length constant region. Same applies to the β, γ, and δ chains correspondingly.

The term "CD3" as used herein refers to CD3 (cluster of differentiation 3), which is a protein complex and T cell co-receptor that is involved in activating both the cytotoxic T cell (CD8⁺ naive T cells) and T helper cells (CD4⁺ naive T cells). It is composed of four distinct chains. In mammals, the complex contains a CD3γ chain, a CD3δ chain, and two CD3ε chains. These chains associate with the T-cell receptor (TCR) and the CD3-zeta (ζ-chain, CD3ζ) to generate an activation signal in T lymphocytes.

The term "TCR complex" as used herein depending on the context may refer to the TCR construct according to the present invention, i.e. to a TCR construct not comprising all CD3 chains, differing from its common meaning of a TCR with all CD3 chains γ, δ, ε, and ζ.

The term "TCR construct" as used herein refers to a construct based on the TCR which additionally to the TCR α and/or TCR β chain or fragments thereof may comprise further chains, in particular CD3 chains or fragments thereof, in particular CD3 ζ chains or fragments thereof.

The term "chimeric" as used herein is used in the sense that some (parts of certain) genes are omitted and replaced by (parts of) other genes. For example, a T cell specific signal peptide may be omitted and replaced by a NK cell specific signal peptide. Or in another example, the TCR Cα and Cβ transmembrane and cytoplasmic domains are omitted and their function is replaced by corresponding engineered elements from the CD3ζ chains.

The terms "functional TCR", "functional TCR construct", "functional TCR complex" and "functional TCR types" refer to TCR constructs that are expressed on the recipient cell surface, recognize fragments of antigen as peptides bound to MHC molecules and that enable a TCR-dependent activation of the recipient cell after antigen binding. That means that this term does not include TCR construct versions that are not expressed, for example, due to pseudogenes, i.e., genes with frameshift mutations or defects in the recombination signal. The annotation whether a TCR variable chain is functional or rather a pseudogene can be found for example in Folch ("The Human T cell Receptor Beta Variable (TRBV) Genes", Folch Géraldibem Lefranc Maire-Paule, Exp Clin Immunogenet 2000;17:42-54) or Su et al. (Chen Su and Masatoshi Nei, Mol.- Biol. Evol. 2001;18(4):505-513). In addition to being expressed the functional TCR constructs according to the invention have the capability to promote a functioning TCR-specific activation cascade like a natural TCR after activation, i.e. antigen binding. The signalling cascades can determine the cell fate through various pathways like, for example, cytokine production, cell survival, proliferation, and differentiation. In order to be "functional" in the sense of the present invention the recipient cell needs to be able to be activated by target peptide-MHC binding of the TCR construct. Depending on the cell type of the recipient cell the TCR-specific activation then leads to cell-specific actions as encountered during an immune reaction. For example, when using a NK cell as recipient cell and activating the NK cell via antigen binding through the TCR construct the NK cell will show its cytotoxic activity against respective target cells. In case of a B cell after being activated it may participate in a two-step differentiation process that yields both short-lived plasmablasts for immediate protection and long-lived plasma cells and memory B cells for persistent protection. Alternatively, the B cell activation can lead to release of Granzyme B and/or tumour cell killing by direct Fas-FasL interaction and/or release of chemokines and catokines. In case of macrophages the activation via the functional TCR construct may result in the elaboration of proinflammatory cytokines or phagocytosis of the target cell. Overall, a person skilled in the art would be able to evaluate whether the recipient cell is activated via the TCR construct and thus would be able to consider the TCR construct functional or not.

The term "fusion protein" as used herein refers to a protein consisting of at least two chains/domains that are encoded by separate genes that have been joined so that they are transcribed and translated as a single unit, producing a single polypeptide. The at least two chains/domains can be fused end-to-end, can be linked by a linker, or set up in a way that amino acids from both chains/domains are interspersed in the fusion protein product. Hence, fusion proteins as used herein are proteins with two or more different protein domains/chains integrated into one molecule.

As used herein the term "fragments thereof" means that not the complete sequence of a chain or domain is comprised. For example, "a TCR α chain or fragment thereof" means that either the complete α chain, including the complete variable and constant region, is comprised or that only a fragment of either both the variable and constant region is comprised or that one region is complete whereas the other region is just partially comprised (for example, the fragment of the TCR α chain could comprise the complete variable region but just a fragment of the constant region, or vice versa. Another example would be that the constant region (for example of the alpha and/or the beta chain) does not comprise the transmembrane and cytoplasmic domains. The examples provided apply *mutatis mutandis* for the other domains, regions, chains, proteins described herein, like beta chain, delta chain, gamma chain, CD3 zeta, signal peptides, etc.

As used herein the terms "domain" and "chain" are associated with each other in that a protein "domain" is a region of a protein's polypeptide "chain" that is self-stabilizing and that folds independently from the rest. Accordingly, a protein "domain" can be defined as a distinct functional and/or structural unit in a protein with a unique and independent well-defined three-dimensional fold.

The term "CD3 zeta" (or "CD3ζ") chain refers to the T-cell surface glycoprotein CD3 zeta chain also known as T-cell receptor T3 zeta chain or CD247 (Cluster of Differentiation 247) which is a protein that in humans is encoded by the CD247 gene. The CD3 zeta chain comprises a cytoplasmic, a membrane, and an extracellular domain with, based on UniProt ID P20963, the cytoplasmic domain ranging from amino acids positions 52 to 164, the membrane domain ranging from amino acid positions 31 to 51, and the extracellular domain ranging from amino acid positions 22 to 30. A TCR construct comprising the cytoplasmic and the membrane domains comprises amino acids 31 to 164 of the CD3 zeta chain.

As used herein the term "DAP10" refers to the membrane adaptor protein, DAP10, which is encoded by a gene immediately adjacent to DAP12 in the genome, but in opposite transcriptional orientation. Although DAP10 has only 20% overall amino acid homology with DAP12, it is expressed as a disulfide-bonded homodimer on the surface of NK cells, myeloid cells, and a subset of T cells. Unlike DAP12, DAP10 does not have an ITAM in its cytoplasmic region. DAP10 is noncovalently associated with CD16A, a receptor on NK cells, TCR-γ/δ+ T cells, and CD8+ T cells that recognizes the nonclassical MHC class I molecules MICA and MICB 8 10. The term "DAP 10 signalling domain" as used herein refers to the part of the protein responsible for the signalling function of DAP10 and comprises the signalling motif YINM in the cytoplasmic domain of DAP10.

As used herein, the term "% sequence identity" in connection with amino acid sequences of polypeptides/peptides and/or nucleic acid sequences or nucleic acid molecules describes the number of matches of identical amino acid or nucleic acid residues of two or more aligned sequences as compared to the number of residues making up the overall length of the compared sequences (or the overall compared portions thereof). Using an alignment of two or more sequences or sub-sequences, the percentage of residues that are the same may be determined when the (sub)sequences are compared and aligned for maximum correspondence over a window of comparison, or over a designated region as measured using a sequence comparison algorithm as known in the art, or when manually aligned and visually inspected. Non-limiting examples of algorithms for use in determining sequence identity include, for example, those based on the NCBI BLAST algorithm (Altschul et al., Nucleic Acids Res 25(1997), 3389-3402), CLUSTALW computer program (Thompson, Nucl. Acids Res. 2(1994), 4673-4680) or FASTA (Pearson and Lipman, Proc. Natl. Acad. Sci., 85(1988), 2444). Although the FASTA algorithm typically does not consider internal non-matching deletions or additions in sequences, i.e. gaps, in its calculation, this can be corrected manually to avoid an overestimation of the % sequence identity. CLUSTALW, however, does take sequence gaps into account in its identity calculations. Also available are the BLAST and BLAST 2.0 algorithms (Altschul et al., Nucl Acids Res., 25(1977), 3389). The term "% sequence identity", in the context of this invention, refers to the percentage of sequence identity over the entire length of the amino acid or nucleotide sequence of the gene, protein or polypeptide. The terms "protein" and "polypeptide" are, unless expressly indicated otherwise in the context of this description, interchangeable herein.

TCRs and their fragments can be produced using techniques well known in the art. For example, the TCRs can be produced recombinantly in cells.

For recombinant production, a polynucleotide sequence encoding the TCR construct is inserted into an appropriate expression vehicle, such as a vector which contains the necessary elements for the transcription and translation of the inserted coding sequence. The expression vehicle is then transfected into a suitable target cell which will express the peptide. Transfection techniques known in the art include, but are not limited to, calcium phosphate precipitation and electroporation. A variety of host-expression vector systems may be utilized to express the TCR constructs described herein.

In some embodiments, the amino acid sequence of the TCR may comprise one or more phenotypically silent substitutions. "Phenotypically silent substitutions" are also named "conservative amino acid substitutions". The concept of "conservative amino acid substitutions" is understood by the skilled artisan, and preferably means that codons encoding positively-charged residues (H, K, and R) are substituted with codons encoding positively-charged residues, codons encoding negatively- charged residues (D and E) are substituted with codons encoding negatively-charged residues, codons encoding neutral polar residues (C, G, N, Q, S, T, and Y) are substituted with codons encoding neutral polar residues, and codons encoding neutral non-polar residues (A, F, I, L, M, P, V, and W) are substituted with codons encoding neutral non-polar residues. These variations can spontaneously occur, be introduced by random mutagenesis, or can be introduced by directed mutagenesis. Those changes can be made without destroying the essential characteristics of these polypeptides. The ordinarily skilled artisan can readily and routinely screen variant amino acids and/or the nucleic acids encoding them to determine if these variations substantially reduce or destroy the ligand binding capacity by methods known in the art.

The presently disclosed invention further provides isolated nucleic acids encoding the TCR constructs or fragments disclosed or otherwise enabled herein. The nucleic acids may comprise DNA or RNA and may be wholly or partially synthetic or recombinant. Reference to a nucleotide sequence as set out herein encompasses a DNA molecule with the specified sequence, and encompasses an RNA molecule with the specified sequence in which uracil (U) is substituted for thymine (T), unless context requires otherwise.

Standard methods can be used to manipulate DNA as described in Sambrook, J. et al., Molecular cloning: A laboratory manual; Cold Spring Harbor Laboratory Press, Cold Spring Harbor, New York, 1989. Methods to modify nucleic acids encoding the disclosed antibodies in order to include the desired cleavage site(s) are well known to a person skilled in the art.

In another embodiment, the nucleic acid molecules which encode the TCR constructs of the presently disclosed inventive concepts also comprise nucleotide sequences that are, for example, at least 70% identical to the sequences disclosed herein. Also contemplated are embodiments in which a sequence is at least 75% identical, at least 80% identical, at least 85% identical, at least 90% identical, at least 91% identical, at least 92% identical, at least 93% identical, at least 94% identical, at least 95% identical, at least 96% identical, at least 97% identical, at least 98% identical, at least 99% identical, or at least 99.5% identical, to a sequence disclosed herein and/or which hybridize to a sequence of the presently disclosed inventive concepts under conditions of high or moderate stringency. The percent identity may be determined by visual inspection of sequence reads and/or mathematical calculation optionally including computer alignment using an appropriate software.

Stringency, including "high stringency," as used herein, includes conditions readily determined by the skilled artisan based on, for example, the length of the DNA. Generally, such conditions are defined as hybridization conditions of 50% formamide, 6xSSC at 42°C (or other similar hybridization solution, such as, e.g., Stark's solution, in 50% formamide at 42°C), and with washing at approximately 68°C with 0.2xSSC, 0.1% SDS. The skilled artisan will recognize that the temperature and wash solution salt concentration can be adjusted as necessary according to factors such as the length of the probe.

"Moderate stringency," as used herein, includes conditions that can be readily determined by those having ordinary skill in the art based on, for example, the length of the DNA. The basic conditions are set forth by Sambrook et al. (79) and include use of a prewashing solution for the nitrocellulose filters 5xSSC, 0.5% SDS, 1.0 mM EDTA (pH 8.0), hybridization conditions of 50% formamide, 6xSSC at 42°C (or other similar hybridization solution, such as Stark's solution, in 50% formamide at 42°C), and washing conditions of 60°C, 0.5xSSC, 0.1% SDS.

The term "vector" is well known in the art, and as used herein refers to a nucleic acid molecule, e.g. double-stranded DNA. In one embodiment, the vector has an exogenous nucleic acid sequence inserted into it. A vector can suitably be used to transport an inserted nucleic acid molecule into a suitable host cell. A vector typically contains all of the necessary elements that permit transcribing the insert nucleic acid molecule, and, preferably, translating the transcript into a polypeptide. A vector typically contains all of the necessary elements such that, once the vector is in a suitable host cell, the vector can replicate independently of, or coincidental with, the host chromosomal DNA; several copies of the vector and its inserted nucleic acid molecule may be generated. Vectors of the present invention can be episomal vectors (i.e., that do not integrate into the genome of a host cell), or can be vectors that integrate into the host cell genome. This definition includes both non-viral and viral vectors. Non-viral vectors include but are not limited to plasmid vectors (e.g. pMA-RQ, pUC vectors, bluescript vectors (pBS) and pBR322 or derivatives thereof that are devoid of bacterial sequences (minicircles)) transposons-based vectors (e.g. PiggyBac (PB) vectors or Sleeping Beauty (SB) vectors), etc. Larger vectors such as artificial chromosomes (bacteria (BAC), yeast (YAC), or human (HAC)) may be used to accommodate larger inserts. In one particular example, a vector described herein may therefore be a plasmid vector. Such plasmid vectors may be present within a cell. In one embodiment, therefore a cell may be provided which comprises a vector (e.g. a plasmid as described herein) comprising a nucleic acid sequence described herein. A cell may therefore be provided comprising a nucleic acid sequence of the invention.

A vector as defined herein may also be a viral vector. A "viral vector" is defined as a recombinantly produced virus or viral particle that comprises a polynucleotide to be delivered into a host cell, either in vivo, ex vivo or in vitro. Examples of viral vectors include retroviral vectors, lentiviral vectors, adenovirus vectors, adeno-associated virus vectors (AAV), alphavirus vectors and the like. Typically, but not necessarily, viral vectors are replication-deficient as they have lost the ability to propagate in a given cell since viral genes essential for replication have been eliminated from the viral vector. However, some viral vectors can also be adapted to replicate specifically or preferentially in a given cell, such as e.g. a cancer cell, and are typically used to trigger the (cancer) cell-specific (onco)lysis. These viral vectors are referred to herein as "oncolytic viruses". Virosomes are a non-limiting example of a vector that comprises both viral and non-viral elements, in particular they combine liposomes with an inactivated HIV or influenza virus (Yamada et al., 2003). Another example encompasses viral vectors mixed with cationic lipids.

Another aspect of the invention refers to a (receptor) cell expressing the TCR construct as described herein.

In the cell the above-described vector comprising a nucleic acid sequence coding for the above described TCR construct may be introduced or ivtRNA coding for said TCR construct may be introduced. The cell may be a peripheral blood lymphocyte such as a T cell. The method of cloning and exogenous expression of a TCR is for example described in Engels et al. (Relapse or eradication of cancer is predicted by peptide-major histocompatibility complex affinity. Cancer Cell, 23(4), 516-26. 2013). The transduction of primary human T cells with a lentiviral vector is, for example, described in Cribbs "simplified production and concentration of lentiviral vectors to achieve high transduction in primary human T cells" BMC Biotechnol. 2013; 13: 98.

The term "transfection" and "transduction" are interchangeable and refer to the process by which an exogenous nucleic acid sequence is introduced in a host cell, e.g. in a eukaryotic host cell. It is noted that introduction or transfer of nucleic acid sequences is not limited to the mentioned methods but can be achieved by any number of means including electroporation, microinjection, gene gun delivery, lipofection, superfection and the mentioned infection by retroviruses or other suitable viruses for transduction or transfection.

In some embodiments, the cell is a peripheral blood lymphocyte (PBL) or a peripheral blood mononuclear cell (PBMC). The cell may be a natural killer cell or a T cell. Preferably, the cell is a NK cell. The T cell may be a CD4⁺ or a CD8⁺ T cell. In some embodiments, the cell is selected from NK cells, and NK-like T cells. In a preferred embodiment the recipient cell is a NK cell, a B cell, a T cell, a macrophage, a neutrophil, or an innate lymphoid cell.

The cell can be treated so as to cause or allow expression of the antibody or antigen-binding fragment thereof of the invention from the polynucleotide(s), e.g. by culturing cells under conditions for expression of the encoding nucleic acid. The purification of the expressed product may be achieved by methods known to one of skill in the art.

Thus, the polynucleotide(s) of the invention, including vector nucleic acids that comprise the polynucleotide sequences that encode the polypeptide(s) capable of forming the TCR construct or fragments thereof of the invention, may be present, either transiently or stably, in an isolated cell. The cell is typically part of a clonal population of cells. As used herein, reference to a cell also encompasses a clonal population of said cell. A clonal population is one that has been grown from a single parent cell. The cell can be from any suitable organism.

### Sequences

*SEQ ID NO: 1: NKp46 signal sequence*
   MSSTLPALLCVGLCLSQRISA
*SEQ ID NO: 2: NKp30 signal sequence*
   MAWMLLLILIMVHPGSCA
*SEQ ID NO: 3: NKp44 signal sequence*
   MAWRALHPLLLLLLLFPGSQA
*SEQ ID NO: 4: CD16A signal sequence*
   MWQLLLPTALLLLVSA
*SEQ ID NO: 5: TCRα signal sequence*
   MKSLRVLLVILWLQLSWVWSQ
*SEQ ID NO: 6: TCRβ signal sequence*
   MGSWTLCCVSLCILVAKHT
*SEQ ID NO: 7: CD3ζ sequence* 
LCYLLDGILFIYGVILTALFLRVKFSRSADAPAYQQGQNQLYNELNLGRREEYDVLDKRRGRDP
EMGGKPQRRKNPQEGLYNELQKDKMAEAYSEIGMKGERRRGKGHDGLYQGLSTATKDTYDA
LHMQALPPR
*SEQ ID NO: 8: DAP10 signaling domain sequence* 
LCYLLDGILFIYGVILTALFLRVKFSRSADAPAYQQGQNQLYNELNLGRREEYDVLDKRRGRDP
EMGGKPQRRKNPQEGLYNELQKDKMAEAYSEIGMKGERRRGKGHDGLYQGLSTATKDTYDA
LHMQALPPR
*SEQ ID NO: 9: linker sequence 1*
   APAP
*SEQ ID NO: 10: linker sequence 2*
   ESGS
*SEQ ID NO: 11: linker sequence 3*
   GGGS
*SEQ ID NO: 12: linker sequence 4*
   GGGGS
*SEQ ID NO: 13: linker sequence 5*
   GSGGSG
*SEQ ID NO: 14: NYESO1 TCRα CDR1 sequence*
   DRGSQS
*SEQ ID NO: 15: NYESO1 TCRα CDR2 sequence*
   IYSNGD
*SEQ ID NO: 16: NYESO1 TCRα CDR3 sequence*
   AADDKII
*SEQ ID NO: 17: NYESO1 TCRβ CDR1 sequence*
   SGHDY
*SEQ ID NO: 18: NYESO1 TCRβ CDR2 sequence*
   FNNNVP
*SEQ ID NO: 19: NYESO1 TCRβ CDR3 sequence*
   ASSRGGSGYT
*SEQ ID NO: 20: NYESO1* Vα *sequence (no signal sequence)* 
QKEVEQNSGPLSVPEGAIASLNCTYSDRGSQSFFWYRQYSGKSPELIMFIYSNGDKEDGRFTA
QLNKASQYVSLLIRDSQPSDSATYLCAADDKIIFGKGTRLHILPN
*SEQ ID NO: 21: NYESO1* Vβ *sequence* (n*o signal sequence)* 
DAGVIQSPRHEVTEMGQEVTLRCKPISGHDYLFWYRQTMMRGLELLIYFNNNVPIDDSGMPED
RFSAKMPNASFSTLKIQPSEPRDSAVYFCASSRGGSGYTFGSGTRLTVV
*SEQ ID NO: 22:* Cα *sequence* 
IQNPDPAVYQLRDSKSSDKSVCLFTDFDSQTNVSQSKDSDVYITDKCVLDMRSMDFKSNSAVA
WSNKSDFACANAFNNSIIPEDTFFPSSDVPCDVKLVEKSFETDTNLNFQNLS
*SEQ ID NO: 23:* Cβ *sequence* 
EDLNKVFPPEVAVFEPSKAEIAHTQKATLVCLATGFFPDHVELSWWVNGKEVHSGVCTDPQPL
KEQPALNDSRYCLSSRLRVSATFWQNPRNHFRCQVQFYGLSENDEWTQDRAKPVTQIVSAEA
WGRADCGITSASYHQGVLSA
*SEQ ID NO: 24:* Cβ *sequence with 9-mer epitope tag* 
EDLNKVFPPEVAVFEPSKAEIAHTQKATLVCLATGFFPDHVELSWWVNGKEVHSGVCTDPQPL
KEQPALNDGSGGEVPKDRFSGSGSRYCLSSRLRVSATFWQNPRNHFRCQVQFYGLSENDEW
TQDRAKPVTQIVSAEAWGRADCGITSASYHQGVLSA
*SEQ ID NO: 25: Fusion protein NYESO1 TCRα chain and CD3ζ chain sequence (no signal sequence)* 
QKEVEQNSGPLSVPEGAIASLNCTYSDRGSQSFFWYRQYSGKSPELIMFIYSNGDKEDGRFTA
QLNKASQYVSLLIRDSQPSDSATYLCAADDKIIFGKGTRLHILPNIQNPDPAVYQLRDSKSSDKS
VCLFTDFDSQTNVSQSKDSDVYITDKCVLDMRSMDFKSNSAVAWSNKSDFACANAFNNSIIPE
DTFFPSSDVPCDVKLVEKSFETDTNLNFQNLSLCYLLDGILFIYGVILTALFLRVKFSRSADAPAY
QQGQNQLYNELNLGRREEYDVLDKRRGRDPEMGGKPQRRKNPQEGLYNELQKDKMAEAYS
EIGMKGERRRGKGHDGLYQGLSTATKDTYDALHMQALPP
*SEQ ID NO: 26: Fusion protein NYESO1 TCRβ chain and CD3ζ chain sequence (no signal sequence)* 
DAGVIQSPRHEVTEMGQEVTLRCKPISGHDYLFWYRQTMMRGLELLIYFNNNVPIDDSGMPED
RFSAKMPNASFSTLKIQPSEPRDSAVYFCASSRGGSGYTFGSGTRLTVVEDLNKVFPPEVAVF
EPSKAEIAHTQKATLVCLATGFFPDHVELSWWVNGKEVHSGVCTDPQPLKEQPALNDSRYCLS
SRLRVSATFWQNPRNHFRCQVQFYGLSENDEWTQDRAKPVTQIVSAEAWGRADCGITSASY
 
HQGVLSALCYLLDGILFIYGVILTALFLRVKFSRSADAPAYQQGQNQLYNELNLGRREEYDVLDK
RRGRDPEMGGKPQRRKNPQEGLYNELQKDKMAEAYSEIGMKGERRRGKGHDGLYQGLSTA
TKDTYDALHMQALPPR
*SEQ ID NO: 27: Fusion protein NYESO1 TCRβ chain and CD3ζ chain sequence (no signal sequence; with 9-mer epitope tag)* 
DAGVIQSPRHEVTEMGQEVTLRCKPISGHDYLFWYRQTMMRGLELLIYFNNNVPIDDSGMPED
RFSAKMPNASFSTLKIQPSEPRDSAVYFCASSRGGSGYTFGSGTRLTVVEDLNKVFPPEVAVF
EPSKAEIAHTQKATLVCLATGFFPDHVELSWWVNGKEVHSGVCTDPQPLKEQPALNDGSGGE
VPKDRFSGSGSRYCLSSRLRVSATFWQNPRNHFRCQVQFYGLSENDEWTQDRAKPVTQIVS
AEAWGRADCGITSASYHQGVLSALCYLLDGILFIYGVILTALFLRVKFSRSADAPAYQQGQNQL
YNELNLGRREEYDVLDKRRGRDPEMGGKPQRRKNPQEGLYNELQKDKMAEAYSEIGMKGER
RRGKGHDGLYQGLSTATKDTYDALHMQALPPR
*SEQ IDO NO: 28: 9-mer epitope tag*
   GEVPKDRFS
*SEQ ID NO: 29: KRASG12V-TCR6-TCRa CDR1 sequence*
   NSAFQY
*SEQ ID NO: 30: KRASG12V-TCR6-TCRα CDR2 sequence*
   TYSSGN
*SEQ ID NO: 31: KRASG12V-TCR6-TCRα CDR3 sequence*
   AMSARNNDMR
*SEQ ID NO: 32: KRASG12V-TCR6-TCRβ CDR1 sequence*
   SNHLY
*SEQ ID NO: 33: KRASG12V-TCR6-TCRβ CDR2 sequence*
   FYNNEI
*SEQ ID NO: 34: KRASG12V-TCR6-TCRβ CDR3 sequence*
   ARSGTSTGELF
*SEQ ID NO:* 35: *KRASG12V*-*TCR6-Vα sequence (no signal sequence)* 
QKEVEQDPGPLSVPEGAIVSLNCTYSNSAFQYFMWYRQYSRKGPELLMYTYSSGNKEDGRFT
AQVDKSSKYISLFIRDSQPSDSATYLCAMSARNNDMRFGAGTRLTVKPN
*SEQ ID NO:* 36: KRASG 12 V- *TCR6-Vβ sequence (no signal sequence)* 
EPEVTQTPSHQVTQMGQEVILRCVPISNHLYFYWYRQILGQKVEFLVSFYNNEISEKSEIFDDQF
SVERPDGSNFTLKIRSTKLEDSAMYFCARSGTSTGELFFGEGSRLTVL
*SEQ ID NO: 37: Fusion protein KRASG12V-TCR6-TCRα chain and CD3ζ chain sequence (no signal sequence)* 
QKEVEQDPGPLSVPEGAIVSLNCTYSNSAFQYFMWYRQYSRKGPELLMYTYSSGNKEDGRFT
AQVDKSSKYISLFIRDSQPSDSATYLCAMSARNNDMRFGAGTRLTVKPNIQNPDPAVYQLRDS
KSSDKSVCLFTDFDSQTNVSQSKDSDVYITDKCVLDMRSMDFKSNSAVAWSNKSDFACANAF
NNSIIPEDTFFPSSDVPCDVKLVEKSFETDTNLNFQNLSLCYLLDGILFIYGVILTALFLRVKFSRS
ADAPAYQQGQNQLYNELNLGRREEYDVLDKRRGRDPEMGGKPQRRKNPQEGLYNELQKDK
MAEAYSEIGMKGERRRGKGHDGLYQGLSTATKDTYDALHMQALPPR
*SEQ ID NO:* 38: *Fusion protein KRASG12V-TCR6-TCRβ chain and CD3ζ chain sequence (no signal sequence)* 
EPEVTQTPSHQVTQMGQEVILRCVPISNHLYFYWYRQILGQKVEFLVSFYNNEISEKSEIFDDQF
SVERPDGSNFTLKIRSTKLEDSAMYFCARSGTSTGELFFGEGSRLTVLEDLNKVFPPEVAVFEP
SKAEIAHTQKATLVCLATGFFPDHVELSWWVNGKEVHSGVCTDPQPLKEQPALNDGSGGSGS
RYCLSSRLRVSATFWQNPRNHFRCQVQFYGLSENDEWTQDRAKPVTQIVSAEAWGRADCGIT
SASYHQGVLSALCYLLDGILFIYGVILTALFLRVKFSRSADAPAYQQGQNQLYNELNLGRREEYD
VLDKRRGRDPEMGGKPQRRKNPQEGLYNELQKDKMAEAYSEIGMKGERRRGKGHDGLYQG
LSTATKDTYDALHMQALPPR
*SEQ ID NO:* 39: *Fusion protein KRASG12V-TCR6-TCRβ chain and CD3ζ chain sequence (no signal sequence; with epitope tag)* 
EPEVTQTPSHQVTQMGQEVILRCVPISNHLYFYWYRQILGQKVEFLVSFYNNEISEKSEIFDDQF
SVERPDGSNFTLKIRSTKLEDSAMYFCARSGTSTGELFFGEGSRLTVLEDLNKVFPPEVAVFEP
SKAEIAHTQKATLVCLATGFFPDHVELSWWVNGKEVHSGVCTDPQPLKEQPALNDGSGGEVP
KDRFSGSGSRYCLSSRLRVSATFWQNPRNHFRCQVQFYGLSENDEWTQDRAKPVTQIVSAE
AWGRADCGITSASYHQGVLSALCYLLDGILFIYGVILTALFLRVKFSRSADAPAYQQGQNQLYN
ELNLGRREEYDVLDKRRGRDPEMGGKPQRRKNPQEGLYNELQKDKMAEAYSEIGMKGERRR
GKGHDGLYQGLSTATKDTYDALHMQALPPR
*SEQ ID NO: 40: PRAME-VLD-TCRα CDR1 sequence*
   VSGLRG
*SEQ ID NO: 41: PRAME-VLD-TCRα CDR2 sequence*
   LYSAGEEE
*SEQ ID NO: 42: PRAME-VLD-TCRα CDR3 sequence*
   AVHSTAQAGTALI
*SEQ ID NO: 43: PRAME-VLD-TCRβ CDR1 sequence*
   SGDLS
*SEQ ID NO: 44: PRAME-VLD-TCRβ CDR2 sequence*
   YYNGEE
*SEQ ID NO: 45: PRAME-VLD-TCRβ CDR3 sequence*
   ASSTHRGQTNYGYT
*SEQ ID NO: 46: PRAME-VLD-Vα sequence (no signal sequence)* 
EDQVTQSPEALRLQEGESSSLNCSYTVSGLRGLFWYRQDPGKGPEFLFTLYSAGEEKEKERL
KATLTKKESFLHITAPKPEDSATYLCAVHSTAQAGTALIFGKGTTLSVSSN
*SEQ ID NO: 47: PRAME-VLD-Vβ sequence (no signal sequence)* 
DSGVTQTPKHLITATGQRVTLRCSPRSGDLSVYWYQQSLDQGLQFLIQYYNGEERAKGNILER
FSAQQFPDLHSELNLSSLELGDSALYFCASSTHRGQTNYGYTFGSGTRLTVV
*SEQ ID NO: 48: Fusion protein PRAME-VLD-TCRα chain and CD3ζ chain sequence (no signal sequence)* 
EDQVTQSPEALRLQEGESSSLNCSYTVSGLRGLFWYRQDPGKGPEFLFTLYSAGEEKEKERL
KATLTKKESFLHITAPKPEDSATYLCAVHSTAQAGTALIFGKGTTLSVSSNIQNPDPAVYQLRDS
KSSDKSVCLFTDFDSQTNVSQSKDSDVYITDKCVLDMRSMDFKSNSAVAWSNKSDFACANAF
NNSIIPEDTFFPSSDVPCDVKLVEKSFETDTNLNFQNLSLCYLLDGILFIYGVILTALFLRVKFSRS
ADAPAYQQGQNQLYNELNLGRREEYDVLDKRRGRDPEMGGKPQRRKNPQEGLYNELQKDK
MAEAYSEIGMKGERRRGKGHDGLYQGLSTATKDTYDALHMQALPPR
*SEQ ID NO: 49: Fusion protein PRAME-VLD-TCRβ chain and CD3ζ chain sequence (no signal sequence)* 
DSGVTQTPKHLITATGQRVTLRCSPRSGDLSVYWYQQSLDQGLQFLIQYYNGEERAKGNILER
FSAQQFPDLHSELNLSSLELGDSALYFCASSTHRGQTNYGYTFGSGTRLTVVEDLNKVFPPEV
AVFEPSKAEIAHTQKATLVCLATGFFPDHVELSWWVNGKEVHSGVCTDPQPLKEQPALNDSRY
CLSSRLRVSATFWQNPRNHFRCQVQFYGLSENDEWTQDRAKPVTQIVSAEAWGRADCGITSA
SYHQGVLSALCYLLDGILFIYGVILTALFLRVKFSRSADAPAYQQGQNQLYNELNLGRREEYDVL
DKRRGRDPEMGGKPQRRKNPQEGLYNELQKDKMAEAYSEIGMKGERRRGKGHDGLYQGLS
TATKDTYDALHMQALPPR
*SEQ ID NO: 50: Fusion protein PRAME-VLD-TCRβ chain and CD3ζ chain sequence (no signal sequence; with 9-mer epitope tag)* 
DSGVTQTPKHLITATGQRVTLRCSPRSGDLSVYWYQQSLDQGLQFLIQYYNGEERAKGNILER
FSAQQFPDLHSELNLSSLELGDSALYFCASSTHRGQTNYGYTFGSGTRLTVVEDLNKVFPPEV
AVFEPSKAEIAHTQKATLVCLATGFFPDHVELSWWVNGKEVHSGVCTDPQPLKEQPALNDGS
GGEVPKDRFSGSGSRYCLSSRLRVSATFWQNPRNHFRCQVQFYGLSENDEWTQDRAKPVTQ
IVSAEAWGRADCGITSASYHQGVLSALCYLLDGILFIYGVILTALFLRVKFSRSADAPAYQQGQN
QLYNELNLGRREEYDVLDKRRGRDPEMGGKPQRRKNPQEGLYNELQKDKMAEAYSEIGMKG
ERRRGKGHDGLYQGLSTATKDTYDALHMQALPPR
*SEQ ID NO: 51: Signal sequence CD79A*
   M PGGPGVLQALPA TI FLLFLLSA VYLGPGCQA
*SEQ ID NO: 52: Signal sequence CD79B*
   MARLALSPVPSHWMVALLLLLSAEPVPA
*SEQ ID NO:* 53: *Signal sequence* CD14
   MERASCLLLLLLPLVHVSA
*SEQ ID NO: 54: Signal sequence* CD40
   MVRLPLQCVLWGCLLTAVHP
*SEQ ID NO:* 55: *Signal sequence* CD11b
   MALRVLLLTALTLCHG
*SEQ ID NO:* 56: *Signal sequence* CD64
   MWFLTTLLLWVPVDG
*SEQ ID NO: 57: Fusion protein NYESO1 TCRβ chain and CD3ζ chain sequence (NKp46 signal sequence, with 9-mer epitope tag)* 
MSSTLPALLCVGLCLSQRISADAGVIQSPRHEVTEMGQEVTLRCKPISGHDYLFWYRQTMMRG
LELLIYFNNNVPIDDSGMPEDRFSAKMPNASFSTLKIQPSEPRDSAVYFCASSRGGSGYTFGSG
TRLTVVEDLNKVFPPEVAVFEPSKAEIAHTQKATLVCLATGFFPDHVELSWWVNGKEVHSGVC
TDPQPLKEQPALNDGSGGEVPKDRFSGSGSRYCLSSRLRVSATFWQNPRNHFRCQVQFYGL
SENDEWTQDRAKPVTQIVSAEAWGRADCGITSASYHQGVLSALCYLLDGILFIYGVILTALFLRV
KFSRSADAPAYQQGQNQLYNELNLGRREEYDVLDKRRGRDPEMGGKPQRRKNPQEGLYNEL
QKDKMAEAYSEIGMKGERRRGKGHDGLYQGLSTATKDTYDALHMQALPPR
*SEQ ID NO:* 58: *Fusion protein NYESO1 TCRα chain and CD3ζ chain sequence (NKp46 signal sequence)* 
MSSTLPALLCVGLCLSQRISAQKEVEQNSGPLSVPEGAIASLNCTYSDRGSQSFFWYRQYSGK
SPELIMFIYSNGDKEDGRFTAQLNKASQYVSLLIRDSQPSDSATYLCAADDKIIFGKGTRLHILPN
IQNPDPAVYQLRDSKSSDKSVCLFTDFDSQTNVSQSKDSDVYITDKCVLDMRSMDFKSNSAVA
WSNKSDFACANAFNNSIIPEDTFFPSSDVPCDVKLVEKSFETDTNLNFQNLSLCYLLDGILFIYG
VILTALFLRVKFSRSADAPAYQQGQNQLYNELNLGRREEYDVLDKRRGRDPEMGGKPQRRKN
PQEGLYNELQKDKMAEAYSEIGMKGERRRGKGHDGLYQGLSTATKDTYDALHMQALPPR
*SEQ ID NO:* 59: *Fusion protein NYESO1 TCRβ chain, CD3ζ chain and DAP10 sequence (NKp46 signal sequence, with 9-mer epitope tag)* 
MSSTLPALLCVGLCLSQRISADAGVIQSPRHEVTEMGQEVTLRCKPISGHDYLFWYRQTMMRG
LELLIYFNNNVPIDDSGMPEDRFSAKMPNASFSTLKIQPSEPRDSAVYFCASSRGGSGYTFGSG
TRLTVVEDLNKVFPPEVAVFEPSKAEIAHTQKATLVCLATGFFPDHVELSWWVNGKEVHSGVC
TDPQPLKEQPALNDGSGGEVPKDRFSGSGSRYCLSSRLRVSATFWQNPRNHFRCQVQFYGL
SENDEWTQDRAKPVTQIVSAEAWGRADCGITSASYHQGVLSALCYLLDGILFIYGVILTALFLRV
KFSRSADAPAYQQGQNQLYNELNLGRREEYDVLDKRRGRDPEMGGKPQRRKNPQEGLYNEL
QKDKMAEAYSEIGMKGERRRGKGHDGLYQGLSTATKDTYDALHMQALPPRGGSLCARPRRS
PAQEDGKVYINMPGRG
*SEQ ID NO: 60: Fusion protein NYESO1 TCRα chain, CD3ζ chain and DAP10 sequence (NKp46 signal sequence)* 
MSSTLPALLCVGLCLSQRISAQKEVEQNSGPLSVPEGAIASLNCTYSDRGSQSFFWYRQYSGK
SPELIMFIYSNGDKEDGRFTAQLNKASQYVSLLIRDSQPSDSATYLCAADDKIIFGKGTRLHILPN
IQNPDPAVYQLRDSKSSDKSVCLFTDFDSQTNVSQSKDSDVYITDKCVLDMRSMDFKSNSAVA
WSNKSDFACANAFNNSIIPEDTFFPSSDVPCDVKLVEKSFETDTNLNFQNLSLCYLLDGILFIYG
VILTALFLRVKFSRSADAPAYQQGQNQLYNELNLGRREEYDVLDKRRGRDPEMGGKPQRRKN
PQEGLYNELQKDKMAEAYSEIGMKGERRRGKGHDGLYQGLSTATKDTYDALHMQALPPRGG
SLCARPRRSPAQEDGKVYINMPGRG
*SEQ ID NO: 61: Fusion protein NYESO1 TCRβ chain and CD3ζ chain sequence (NKp30 signal sequence, with 9-mer epitope tag)* 
MAWMLLLILIMVHPGSCADAGVIQSPRHEVTEMGQEVTLRCKPISGHDYLFWYRQTMMRGLEL
LIYFNNNVPIDDSGMPEDRFSAKMPNASFSTLKIQPSEPRDSAVYFCASSRGGSGYTFGSGTR
LTVVEDLNKVFPPEVAVFEPSKAEIAHTQKATLVCLATGFFPDHVELSWWVNGKEVHSGVCTD
PQPLKEQPALNDGSGGEVPKDRFSGSGSRYCLSSRLRVSATFWQNPRNHFRCQVQFYGLSE
NDEWTQDRAKPVTQIVSAEAWGRADCGITSASYHQGVLSALCYLLDGILFIYGVILTALFLRVKF
SRSADAPAYQQGQNQLYNELNLGRREEYDVLDKRRGRDPEMGGKPQRRKNPQEGLYNELQ
KDKMAEAYSEIGMKGERRRGKGHDGLYQGLSTATKDTYDALHMQALPPR
*SEQ ID NO: 62: Fusion protein NYESO1 TCRα chain and CD3ζ chain sequence (NKp30 signal sequence)* 
MAWMLLLILIMVHPGSCAQKEVEQNSGPLSVPEGAIASLNCTYSDRGSQSFFWYRQYSGKSPE
LIMFIYSNGDKEDGRFTAQLNKASQYVSLLIRDSQPSDSATYLCAADDKIIFGKGTRLHILPNIQN
PDPAVYQLRDSKSSDKSVCLFTDFDSQTNVSQSKDSDVYITDKCVLDMRSMDFKSNSAVAWS
NKSDFACANAFNNSIIPEDTFFPSSDVPCDVKLVEKSFETDTNLNFQNLSLCYLLDGILFIYGVILT
ALFLRVKFSRSADAPAYQQGQNQLYNELNLGRREEYDVLDKRRGRDPEMGGKPQRRKNPQE
GLYNELQKDKMAEAYSEIGMKGERRRGKGHDGLYQGLSTATKDTYDALHMQALPPR
*SEQ ID NO:* 63: *Fusion protein NYESO1 TCRβ chain and CD3ζ chain sequence (NKp44 signal sequence, with 9-mer epitope tag)* 
MAWRALHPLLLLLLLFPGSQADAGVIQSPRHEVTEMGQEVTLRCKPISGHDYLFWYRQTMMR
GLELLIYFNNNVPIDDSGMPEDRFSAKMPNASFSTLKIQPSEPRDSAVYFCASSRGGSGYTFGS
GTRLTVVEDLNKVFPPEVAVFEPSKAEIAHTQKATLVCLATGFFPDHVELSWWVNGKEVHSGV
CTDPQPLKEQPALNDGSGGEVPKDRFSGSGSRYCLSSRLRVSATFWQNPRNHFRCQVQFYG
LSENDEWTQDRAKPVTQIVSAEAWGRADCGITSASYHQGVLSALCYLLDGILFIYGVILTALFLR
VKFSRSADAPAYQQGQNQLYNELNLGRREEYDVLDKRRGRDPEMGGKPQRRKNPQEGLYNE
LQKDKMAEAYSEIGMKGERRRGKGHDGLYQGLSTATKDTYDALHMQALPPR
*SEQ ID NO: 64: Fusion protein NYESO1 TCRα chain and CD3ζ chain sequence (NKp44 signal sequence)* 
MAWRALHPLLLLLLLFPGSQAQKEVEQNSGPLSVPEGAIASLNCTYSDRGSQSFFWYRQYSG
KSPELIMFIYSNGDKEDGRFTAQLNKASQYVSLLIRDSQPSDSATYLCAADDKIIFGKGTRLHILP
NIQNPDPAVYQLRDSKSSDKSVCLFTDFDSQTNVSQSKDSDVYITDKCVLDMRSMDFKSNSAV
 
AWSNKSDFACANAFNNSIIPEDTFFPSSDVPCDVKLVEKSFETDTNLNFQNLSLCYLLDGILFIY
GVILTALFLRVKFSRSADAPAYQQGQNQLYNELNLGRREEYDVLDKRRGRDPEMGGKPQRRK
NPQEGLYNELQKDKMAEAYSEIGMKGERRRGKGHDGLYQGLSTATKDTYDALHMQALPPR
*SEQ ID NO:* 65: *Fusion protein NYESO1 TCRβ chain and CD3ζ chain sequence (TCRβ signal sequence, with 9-mer epitope tag)* 
MGSWTLCCVSLCILVAKHTDAGVIQSPRHEVTEMGQEVTLRCKPISGHDYLFWYRQTMMRGL
ELLIYFNNNVPIDDSGMPEDRFSAKMPNASFSTLKIQPSEPRDSAVYFCASSRGGSGYTFGSGT
RLTVVEDLNKVFPPEVAVFEPSKAEIAHTQKATLVCLATGFFPDHVELSWWVNGKEVHSGVCT
DPQPLKEQPALNDGSGGEVPKDRFSGSGSRYCLSSRLRVSATFWQNPRNHFRCQVQFYGLS
ENDEWTQDRAKPVTQIVSAEAWGRADCGITSASYHQGVLSALCYLLDGILFIYGVILTALFLRVK
FSRSADAPAYQQGQNQLYNELNLGRREEYDVLDKRRGRDPEMGGKPQRRKNPQEGLYNELQ
KDKMAEAYSEIGMKGERRRGKGHDGLYQGLSTATKDTYDALHMQALPPR
*SEQ ID NO:* 66: *Fusion protein NYESO1 TCRα chain and CD3ζ chain sequence (TCRα signal sequence)* 
MKSLRVLLVILWLQLSWVWSQQKEVEQNSGPLSVPEGAIASLNCTYSDRGSQSFFWYRQYSG
KSPELIMFIYSNGDKEDGRFTAQLNKASQYVSLLIRDSQPSDSATYLCAADDKIIFGKGTRLHILP
NIQNPDPAVYQLRDSKSSDKSVCLFTDFDSQTNVSQSKDSDVYITDKCVLDMRSMDFKSNSAV
AWSNKSDFACANAFNNSIIPEDTFFPSSDVPCDVKLVEKSFETDTNLNFQNLSLCYLLDGILFIY
GVILTALFLRVKFSRSADAPAYQQGQNQLYNELNLGRREEYDVLDKRRGRDPEMGGKPQRRK
NPQEGLYNELQKDKMAEAYSEIGMKGERRRGKGHDGLYQGLSTATKDTYDALHMQALPPR*
*SEQ ID NO: 67: NYESO1 Vα sequence* 
cagaaagaggtggaacagaacagcggccctctgtctgtgcctgaaggcgccattgctagcctgaattgcacctactccgaccgggg
cagccagagcttcttctggtataggcagtacagcggcaagagccccgagctgatcatgtttatctacagcaacggcgacaaagagg
acggccggtttacagcccagctgaacaaagccagccagtacgtgtcactgctgattcgggatagccagccaagcgacagcgccac
atatctgtgtgccgccgacgacaagatcatcttcggcaagggaaccagactgcacatcctgcctaac
*SEQ ID NO:* 68: *NYESO1 Vβ sequence* 
gatgccggcgtgatccagtctcctagacacgaagtgaccgagatgggccaagaagtgaccctgcgctgcaagcctatcagcggcc
acgattacctgttctggtacagacagaccatgatgagaggcctggaactgctgatctacttcaacaacaacgtgcccatcgacgaca
gcggcatgcccgaggatagattcagcgccaagatgcccaacgccagctttagcaccctgaagatccagcctagcgagcccagag
atagcgccgtgtacttttgcgcctcttccagaggcggcagcggctacacatttggctctggcacaagactgaccgtggtg
*SEQ ID NO:* 69: *NYESO1 Cα sequence* 
attcagaaccccgatcctgccgtgtaccagctgagagacagcaagagcagcgacaagagcgtgtgcctgttcaccgacttcgacag
ccagaccaacgtgtcccagagcaaggacagcgacgtgtacatcaccgataagtgcgtgctggatatgcgcagcatggacttcaag
agcaacagcgccgtggcctggtccaacaagagcgatttcgcctgcgccaacgccttcaacaatagcattatccccgaggacacatt
cttccccagctccgatgtgccctgcgacgtgaagctggtggaaaagagcttcgaaaccgacaccaacctgaacttccagaacctga
gc
*SEQ ID NO: 70: NYESO 1 Cβ sequence (with 9-mer epitope tag)* 
gaagatctgaacaaggtgttccctccagaggtggccgtgttcgagccttctaaggccgagatcgcccacactcagaaagccacactc
gtgtgtctggccaccggctttttccccgatcacgtggaactgtcttggtgggtcaacggcaaagaggtgcacagcggcgtctgtaccga
tcctcagcctctgaaagagcagcccgctctgaacgatggatctggcggagaggtgcccaaggacagattttctggcagcggcagca
gatactgcctgagcagcagactgagagtgtccgccaccttctggcagaaccccagaaaccacttcagatgccaggtgcagttctacg
gcctgagcgagaacgatgagtggacccaggatagagccaagcctgtgacacagatcgtgtctgccgaagcctggggcagagccg
attgtggaattaccagcgccagctaccatcagggcgtgctgtctgcc
*SEQ ID NO: 71: Fusion protein NYESO1 TCRβ chain and CD3ζ chain sequence (NKp46 signal sequence, with 9-mer epitope tag)* 
atgagcagcaccctgcctgctctgctgtgtgtgggactgtgtctgagccagagaatctctgccgatgccggcgtgatccagtctcctaga
cacgaagtgaccgagatgggccaagaagtgaccctgcgctgcaagcctatcagcggccacgattacctgttctggtacagacaga
ccatgatgagaggcctggaactgctgatctacttcaacaacaacgtgcccatcgacgacagcggcatgcccgaggatagattcagc
gccaagatgcccaacgccagctttagcaccctgaagatccagcctagcgagcccagagatagcgccgtgtacttttgcgcctcttcca
gaggcggcagcggctacacatttggctctggcacaagactgaccgtggtggaagatctgaacaaggtgttccctccagaggtggcc
gtgttcgagccttctaaggccgagatcgcccacactcagaaagccacactcgtgtgtctggccaccggctttttccccgatcacgtgga
actgtcttggtgggtcaacggcaaagaggtgcacagcggcgtctgtaccgatcctcagcctctgaaagagcagcccgctctgaacg
atggatctggcggagaggtgcccaaggacagattttctggcagcggcagcagatactgcctgagcagcagactgagagtgtccgcc
accttctggcagaaccccagaaaccacttcagatgccaggtgcagttctacggcctgagcgagaacgatgagtggacccaggata
gagccaagcctgtgacacagatcgtgtctgccgaagcctggggcagagccgattgtggaattaccagcgccagctaccatcaggg
cgtgctgtctgccctgtgctatctgctggatggcatcctgttcatctacggcgttatcctgacagccctgttcctgagagtgaagttcagcag
aagcgccgacgctcctgcttatcagcagggccagaaccagctgtacaacgagctgaacctggggagaagagaagagtacgacgt
gctggacaagcggagaggcagagatcctgagatgggcggaaagccccagcggagaaagaatcctcaagagggcctgtataatg
agctgcagaaagacaagatggccgaggcctacagcgagatcggaatgaagggcgagcgcagaagaggcaagggacacgatg
gactgtaccagggcctgagcaccgccaccaaggatacctatgatgccctgcacatgcaggccctgcctccaaga
*SEQ ID NO: 72: Fusion protein NYESO1 TCRα chain and CD3ζ chain sequence (NKp46 signal sequence)* 
atgtctagcacactgcccgcactgctctgcgttggcctgtgtctgtctcagaggatcagcgcccagaaagaggtggaacagaacagc
ggccctctgtctgtgcctgaaggcgccattgctagcctgaattgcacctactccgaccggggcagccagagcttcttctggtataggca
gtacagcggcaagagccccgagctgatcatgtttatctacagcaacggcgacaaagaggacggccggtttacagcccagctgaac
aaagccagccagtacgtgtcactgctgattcgggatagccagccaagcgacagcgccacatatctgtgtgccgccgacgacaagat
catcttcggcaagggaaccagactgcacatcctgcctaacattcagaaccccgatcctgccgtgtaccagctgagagacagcaaga
gcagcgacaagagcgtgtgcctgttcaccgacttcgacagccagaccaacgtgtcccagagcaaggacagcgacgtgtacatcac
cgataagtgcgtgctggatatgcgcagcatggacttcaagagcaacagcgccgtggcctggtccaacaagagcgatttcgcctgcg
ccaacgccttcaacaatagcattatccccgaggacacattcttccccagctccgatgtgccctgcgacgtgaagctggtggaaaaga
gcttcgaaaccgacaccaacctgaacttccagaacctgagcctgtgttacctgctcgacgggattctctttatctatggcgtgatactgac
cgctctgtttctgcgggtcaagttctccagatccgctgacgccccagcataccagcagggacaaaatcagctctataacgaactgaat
ctcggcaggcgcgaggaatatgacgtcctggataagaggcggggacgcgaccctgaaatgggaggaaaacctcagaggcgga
agaacccacaagaaggcctttacaacgaactgcaaaaggataagatggctgaagcttactccgagattggcatgaagggcgaac
gtcggagaggaaaaggccacgacggactctatcagggactgtctacagccacaaaggacacatacgacgcactccatatgcagg
ctctcccacctaga
*SEQ ID NO: 73: Fusion protein NYESO1 TCRβ chain, CD3ζ chain and DAP10 sequence (NKp46 signal sequence, with 9-mer epitope tag)* 
atgagcagcaccctgcctgctctgctgtgtgtgggactgtgtctgagccagagaatctctgccgatgccggcgtgatccagtctcctaga
cacgaagtgaccgagatgggccaagaagtgaccctgcgctgcaagcctatcagcggccacgattacctgttctggtacagacaga
ccatgatgagaggcctggaactgctgatctacttcaacaacaacgtgcccatcgacgacagcggcatgcccgaggatagattcagc
gccaagatgcccaacgccagctttagcaccctgaagatccagcctagcgagcccagagatagcgccgtgtacttttgcgcctcttcca
gaggcggcagcggctacacatttggctctggcacaagactgaccgtggtggaagatctgaacaaggtgttccctccagaggtggcc
gtgttcgagccttctaaggccgagatcgcccacactcagaaagccacactcgtgtgtctggccaccggctttttccccgatcacgtgga
actgtcttggtgggtcaacggcaaagaggtgcacagcggcgtctgtaccgatcctcagcctctgaaagagcagcccgctctgaacg
atggatctggcggagaggtgcccaaggacagattttctggcagcggcagcagatactgcctgagcagcagactgagagtgtccgcc
accttctggcagaaccccagaaaccacttcagatgccaggtgcagttctacggcctgagcgagaacgatgagtggacccaggata
gagccaagcctgtgacacagatcgtgtctgccgaagcctggggcagagccgattgtggaattaccagcgccagctaccatcaggg
cgtgctgtctgccctgtgctatctgctggatggcatcctgttcatctacggcgttatcctgacagccctgttcctgagagtgaagttcagcag
aagcgccgacgctcctgcttatcagcagggccagaaccagctgtacaacgagctgaacctggggagaagagaagagtacgacgt
gctggacaagcggagaggcagagatcctgagatgggcggaaagccccagcggagaaagaatcctcaagagggcctgtataatg
agctgcagaaagacaagatggccgaggcctacagcgagatcggaatgaagggcgagcgcagaagaggcaagggacacgatg
gactgtaccagggcctgagcaccgccaccaaggatacctatgatgccctgcacatgcaggccctgcctccaagaggcggatctctg
tgtgcccggcctagaagatctcccgctcaagaggatggcaaggtgtacatcaacatgcccggcagaggt
*SEQ ID NO: 74: Fusion protein NYESO1 TCRα chain, CD3ζ chain sequence (NKp46 signal sequence)* 
atgtctagcacactgcccgcactgctctgcgttggcctgtgtctgtctcagaggatcagcgcccagaaagaagtggaacagaacagc
ggccctctgagcgttccagaaggcgccattgctagcctgaattgcacctactccgaccggggcagccagagcttcttctggtataggc
agtacagcggcaagagccccgagctgatcatgtttatctacagcaacggcgacaaagaggacggccggtttacagcccagctgaa
caaagccagccagtacgtgtcactgctgattcgggatagccagccaagcgacagcgccacatatctgtgcgccgctgacgacaag
atcatcttcggcaagggcaccagactgcacatcctgcctaacattcagaaccccgatcctgccgtgtaccagctgagagacagcaa
gagcagcgacaagagcgtgtgcctgttcaccgacttcgacagccagaccaacgtgtcccagagcaaggacagcgacgtgtacatt
accgataagtgcgtgctggatatgcgcagcatggacttcaagagcaacagcgccgtggcctggtccaacaagagcgatttcgcctg
cgccaacgccttcaacaatagcattatccccgaggacacattcttccccagctccgatgtgccctgcgacgtgaagctggtggaaaa
gagcttcgaaaccgacaccaacctgaacttccagaacctgagcctgtgttacctgctcgacgggattctctttatctatggcgtgatactg
accgctctgtttctgcgggtcaagttctccagatccgctgacgccccagcataccagcagggacaaaatcagctctataacgaactga
atctcggcaggcgcgaggaatatgacgtcctggataagaggcggggacgcgaccctgaaatgggaggaaaacctcagaggcgg
aagaacccacaagaaggcctttacaacgaactgcaaaaggataagatggctgaagcttactccgagattggcatgaagggcgaa
cgtcggagaggaaaaggccacgacggactctatcagggactgtctacagccacaaaggacacatacgacgcactccatatgcag
gctctcccacctaga
*SEQ ID NO: 75: Fusion protein NYESO1 TCRα chain, CD3ζ chain and DAP10 sequence (NKp46 signal sequence)* 
atgtctagcacactgcccgcactgctctgcgttggcctgtgtctgtctcagaggatcagcgcccagaaagaagtggaacagaacagc
ggccctctgagcgttccagaaggcgccattgctagcctgaattgcacctactccgaccggggcagccagagcttcttctggtataggc
agtacagcggcaagagccccgagctgatcatgtttatctacagcaacggcgacaaagaggacggccggtttacagcccagctgaa
caaagccagccagtacgtgtcactgctgattcgggatagccagccaagcgacagcgccacatatctgtgcgccgctgacgacaag
atcatcttcggcaagggcaccagactgcacatcctgcctaacattcagaaccccgatcctgccgtgtaccagctgagagacagcaa
gagcagcgacaagagcgtgtgcctgttcaccgacttcgacagccagaccaacgtgtcccagagcaaggacagcgacgtgtacatt
accgataagtgcgtgctggatatgcgcagcatggacttcaagagcaacagcgccgtggcctggtccaacaagagcgatttcgcctg
cgccaacgccttcaacaatagcattatccccgaggacacattcttccccagctccgatgtgccctgcgacgtgaagctggtggaaaa
gagcttcgaaaccgacaccaacctgaacttccagaacctgagcctgtgttacctgctcgacgggattctctttatctatggcgtgatactg
accgctctgtttctgcgggtcaagttctccagatccgctgacgccccagcataccagcagggacaaaatcagctctataacgaactga
atctcggcaggcgcgaggaatatgacgtcctggataagaggcggggacgcgaccctgaaatgggaggaaaacctcagaggcgg
aagaacccacaagaaggcctttacaacgaactgcaaaaggataagatggctgaagcttactccgagattggcatgaagggcgaa
cgtcggagaggaaaaggccacgacggactctatcagggactgtctacagccacaaaggacacatacgacgcactccatatgcag
gctctcccacctcgcggtggaagcctttgtgcaagacccagaaggtcccctgcacaagaagatgggaaagtctatatcaatatgcct
ggcaggggc
*SEQ ID NO: 76: Nkp46 signal sequence 1*
   atgagcagcaccctgcctgctctgctgtgtgtgggactgtgtctgagccagagaatctctgcc
*SEQ ID NO: 77: Nkp46 signal sequence 2*
   atgtctagcacactgcccgcactgctctgcgttggcctgtgtctgtctcagaggatcagcgcc
*SEQ ID NO: 78: Nkp30 signal sequence 1*
   atggcctggatgctgctgctgatcctgatcatggtgcaccctggctcttgtgcc
*SEQ ID NO: 79: Nkp30 signal sequence 2*
   atggcctggatgctgctgctgatcctgatcatggtgcaccctggcagctgcgcc
*SEQ ID NO: 80: Nkp44 signal sequence 1*
   atggcctggcgggctctgcatcctttgttgctg ctgctcctgctgttccctggctctcaagct
*SEQ ID NO: 81: Nkp44 signal sequence 2*
   atggcctggcgggctctgcatcctttgttgctgctgctcctgctgttccctggctctcaggcc
*SEQ ID NO: 82: TCRβ signal sequence*
   atgggcagctggaccctgtgctgtgtgtccctgtgtatcctggtggccaagcacaca
*SEQ ID NO:* 83: *TCRα signal nsequence*
   atgaagtccctgcgggtgctgctggttatcctgtggctgcagctgagctgggtctggtcccag
*SEQ ID NO: 84: CD3ζ sequence 1* 
ctgtgctatctgctggatggcatcctgttcatctacggcgttatcctgacagccctgttcctgagagtgaagttcagcagaagcgccgac
gctcctgcttatcagcagggccagaaccagctgtacaacgagctgaacctggggagaagagaagagtacgacgtgctggacaag
cggagaggcagagatcctgagatgggcggaaagccccagcggagaaagaatcctcaagagggcctgtataatgagctgcagaa
agacaagatggccgaggcctacagcgagatcggaatgaagggcgagcgcagaagaggcaagggacacgatggactgtacca
gggcctgagcaccgccaccaaggatacctatgatgccctgcacatgcaggccctgcctccaaga
*SEQ ID NO:* 85: *CD3ζ sequence 2* 
ctgtgttacctgctcgacgggattctctttatctatggcgtgatactgaccgctctgtttctgcgggtcaagttctccagatccgctgacgccc
cagcataccagcagggacaaaatcagctctataacgaactgaatctcggcaggcgcgaggaatatgacgtcctggataagaggc
ggggacgcgaccctgaaatgggaggaaaacctcagaggcggaagaacccacaagaaggcctttacaacgaactgcaaaagg
ataagatggctgaagcttactccgagattggcatgaagggcgaacgtcggagaggaaaaggccacgacggactctatcagggact
gtctacagccacaaaggacacatacgacgcactccatatgcaggctctcccacctaga
*SEQ ID NO:* 86: *DAP10 sequence 1*
   ctgtgtgcccggcctagaagatctcccgctcaagaggatggcaaggtgtacatcaacatgcccggcagaggt
*SEQ ID NO: 87: DAP10 sequence 2*
   ctttgtgcaagacccagaaggtcccctgcacaagaagatgggaaagtctatatcaatatgcctggcaggggc
*SEQ ID NO:* 88: *Fusion protein NYESO1 TCRβ chain and CD3ζ chain sequence (NKp30 signal* sequence, with 9-mer epitope tag) 
atggcctggatgctgctgctgatcctgatcatggtgcaccctggctcttgtgccgatgccggcgttatccagtctcctagacacgaagtga
ccgagatgggccaagaagtgaccctgcgctgcaagcctatcagcggccacgattacctgttctggtacagacagaccatgatgaga
ggcctggaactgctgatctacttcaacaacaacgtgcccatcgacgacagcggcatgcccgaggatagattcagcgccaagatgcc
caacgccagcttcagcaccctgaagatccagcctagcgagcccagagatagcgccgtgtacttttgcgcctcttccagaggcggca
gcggctacacatttggctctggcacaagactgaccgtggtggaagatctgaacaaggtgttccctccagaggtggccgtgttcgagcc
ttctaaggccgagattgcccacacacagaaagccacactcgtgtgcctggccaccggctttttccccgatcacgtggaactgtcttggt
gggtcaacggcaaagaggtgcacagcggcgtctgtaccgatcctcagcctctgaaagagcagcccgctctgaacgatggatctgg
cggagaggtgcccaaggacagattttctggcagcggcagcagatactgcctgagcagcagactgagagtgtccgccaccttctggc
agaaccccagaaaccacttcagatgccaggtgcagttctacggcctgagcgagaacgatgagtggacccaggatagagccaagc
ctgtgacacagatcgtgtctgccgaagcctggggcagagccgattgtggaattaccagcgccagctaccatcagggcgtgctgtctg
ccctgtgctatctgctggatggcatcctgttcatctacggcgtgatcctgacagccctgttcctgagagtgaagttcagcagaagcgccg
acgctcctgcttatcagcagggccagaaccagctgtacaacgagctgaacctggggagaagagaagagtacgacgtgctggaca
agcggagaggcagagatcctgagatgggcggaaagccccagcggagaaagaatcctcaagagggcctgtataatgagctgcag
aaagacaagatggccgaggcctacagcgagatcggaatgaagggcgagcgcagaagaggcaagggacacgatggactgtac
cagggcctgagcaccgccaccaaggatacctatgatgccctgcacatgcaggccctgcctccaaga
*SEQ ID NO:* 89: *Fusion protein NYESO1 TCRα chain and CD3ζ chain sequence (NKp30 signal sequence)* 
atggcctggatgctgctgctgatcctgatcatggtgcaccctggcagctgcgcccagaaagaggtggaacagaatagcggccctctg
agcgttccagaaggcgctatcgccagcctgaactgcacctacagcgatagaggcagccagagcttcttctggtacagacagtacag
cggcaagagccccgagctgatcatgttcatctacagcaacggcgacaaagaggacggccggtttacagcccagctgaacaaggc
cagccagtacgtgtccctgctgatcagagatagccagcctagcgacagcgccacctatctgtgtgccgccgacgacaagatcatctt
cggcaagggcaccagactgcacatcctgcctaacattcagaaccccgatcctgccgtgtaccagctgagagacagcaagagcag
cgacaagagcgtgtgcctgttcaccgacttcgacagccagaccaacgtgtcccagagcaaggacagcgacgtgtacatcaccgat
aagtgcgtgctggacatgcggagcatggacttcaagagcaacagcgccgtggcctggtccaacaagagcgatttcgcctgcgcca
acgccttcaacaacagcattatccccgaggacacattcttccccagctccgatgtgccctgcgacgtgaagctggtggaaaagagctt
cgagacagacaccaacctgaacttccagaacctgagcctgtgctacctgctggacggcatcctgtttatctacggcgtgatcctgaca
gccctgttcctgagagtgaagttcagcagaagcgccgacgctcctgcttaccagcagggccagaaccagctgtacaacgagctgaa
cctggggagaagagaagagtacgacgtgctggataagcgcagaggcagagatcctgagatgggcggaaagccccagcggaga
 
aagaatcctcaagagggcctgtataatgagctgcagaaagacaagatggccgaggcctactccgagatcggaatgaagggcgag
cgcagaagaggcaagggacacgatggactgtaccagggcctgagcaccgccaccaaggatacctatgatgccctgcacatgca
ggccctgcctccaaga
*SEQ ID NO: 90: Fusion protein NYESO1 TCRβ chain and CD3ζ chain sequence (NKp44 signal sequence, with 9-mer epitope tag)* 
atggcctggcgggctctgcatcctttgttgctgctgctcctgctgttccctggctctcaagctgatgccggcgtgatccagtctcctagacac
gaagtgaccgagatgggccaagaagtgaccctgcgctgcaagcctatcagcggccacgattacctgttctggtacagacagaccat
gatgagaggcctggaactgctgatctacttcaacaacaacgtgcccatcgacgacagcggcatgcccgaggatagattcagcgcc
aagatgcccaacgccagcttcagcaccctgaagatccagcctagcgagcccagagatagcgccgtgtacttttgcgcctcttccaga
ggcggcagcggctacacatttggctctggcacaagactgaccgtggtggaagatctgaacaaggtgttccctccagaggtggccgtg
ttcgagccttctaaggccgagattgcccacacacagaaagccacactcgtgtgcctggccaccggctttttccccgatcacgtggaact
gtcttggtgggtcaacggcaaagaggtgcacagcggcgtctgtaccgatcctcagcctctgaaagagcagcccgctctgaacgatg
gatctggcggagaggtgcccaaggacagattttctggcagcggcagcagatactgcctgagcagcagactgagagtgtccgccac
cttctggcagaaccccagaaaccacttcagatgccaggtgcagttctacggcctgagcgagaacgatgagtggacccaggataga
gccaagcctgtgacacagatcgtgtctgccgaagcctggggcagagccgattgtggaattaccagcgccagctaccatcagggcgt
gctgtctgccctgtgctatctgctggatggcatcctgttcatctacggcgttatcctgacagccctgttcctgagagtgaagttcagcagaa
gcgccgacgctcctgcttatcagcagggccagaaccagctgtacaacgagctgaacctggggagaagagaagagtacgacgtgc
tggacaagcggagaggcagagatcctgagatgggcggaaagccccagcggagaaagaatcctcaagagggcctgtataatgag
ctgcagaaagacaagatggccgaggcctacagcgagatcggaatgaagggcgagcgcagaagaggcaagggacacgatgga
ctgtaccagggcctgagcaccgccaccaaggatacctatgatgccctgcacatgcaggccctgcctccaaga
*SEQ ID NO: 91: Fusion protein NYESO1 TCRα chain and CD3ζ chain sequence (NKp44 signal sequence)* 
atggcctggcgggctctgcatcctttgttgctgctgctcctgctgttccctggctctcaggcccagaaagaggtggaacagaatagcggc
cctctgagcgttccagaaggcgctatcgccagcctgaactgcacctacagcgatagaggcagccagagcttcttctggtacagacag
tacagcggcaagagccccgagctgatcatgttcatctacagcaacggcgacaaagaggacggccggtttacagcccagctgaac
aaggccagccagtacgtgtccctgctgatcagagatagccagcctagcgacagcgccacctatctgtgtgccgccgacgacaagat
catcttcggcaagggcaccagactgcacatcctgcctaacattcagaaccccgatcctgccgtgtaccagctgagagacagcaagt
ccagcgacaagagcgtgtgcctgttcaccgacttcgacagccagaccaacgtgtcccagagcaaggacagcgacgtgtacatcac
cgataagtgcgtgctggacatgcggagcatggacttcaagagcaacagcgccgtggcctggtccaacaagagcgatttcgcctgcg
ccaacgccttcaacaacagcattatccccgaggacacattcttccccagctccgatgtgccctgcgacgtgaagctggtggaaaaga
gcttcgagacagacaccaacctgaacttccagaacctgagcctgtgctacctgctggacggcatcctgtttatctacggcgtgatcctg
acagccctgttcctgagagtgaagttcagcagaagcgccgacgctcctgcttaccagcagggccagaaccagctgtacaacgagct
gaacctggggagaagagaagagtacgacgtgctggataagcgcagaggcagagatcctgagatgggcggaaagccccagcgg
agaaagaatcctcaagagggcctgtataatgagctgcagaaagacaagatggccgaggcctactccgagatcggaatgaagggc
gagcgcagaagaggcaagggacacgatggactgtaccagggcctgagcaccgccaccaaggatacctatgatgccctgcacat
gcaggccctgcctccaaga
*SEQ ID NO: 92: Fusion protein NYESO1 TCRβ chain and CD3ζ chain sequence (TCRβ signal sequence, with 9-mer epitope tag)* 
atgggcagctggaccctgtgctgtgtgtccctgtgtatcctggtggccaagcacacagatgccggcgtgatccagtctcctagacacga
agtgaccgagatgggccaagaagtgaccctgcgctgcaagcctatcagcggccacgattacctgttctggtacagacagaccatga
tgagaggcctggaactgctgatctacttcaacaacaacgtgcccatcgacgacagcggcatgcccgaggatagattcagcgccaa
gatgcccaacgccagcttcagcaccctgaagatccagcctagcgagcccagagatagcgccgtgtacttttgcgcctcttccagagg
cggcagcggctacacatttggctctggcacaagactgaccgtggtggaagatctgaacaaggtgttccctccagaggtggccgtgttc
gagccttctaaggccgagattgcccacacacagaaagccacactcgtgtgcctggctaccggcttctttcccgatcacgtggaactgt
cttggtgggtcaacggcaaagaggtgcacagcggcgtctgtaccgatcctcagcctctgaaagagcagcccgctctgaacgatgga
tctggcggagaggtgcccaaggacagattttctggcagcggcagcagatactgcctgagcagcagactgagagtgtccgccacctt
ctggcagaaccccagaaaccacttcagatgccaggtgcagttctacggcctgagcgagaacgatgagtggacccaggatagagc
caagcctgtgacacagatcgtgtctgccgaagcctggggcagagccgattgtggaattaccagcgccagctaccatcagggcgtgc
tgagcgctctgtgctatctgctggatggcatcctgttcatctacggcgttatcctgacagccctgttcctgagagtgaagttcagcagaag
cgccgacgctcctgcttatcagcagggccagaaccagctgtacaacgagctgaacctggggagaagagaagagtacgacgtgct
ggacaagcggagaggcagagatcctgagatgggcggaaagccccagcggagaaagaatcctcaagagggcctgtataatgag
 
ctgcagaaagacaagatggccgaggcctacagcgagatcggaatgaagggcgagcgcagaagaggcaagggacacgatgga
ctgtaccagggcctgagcaccgccaccaaggacacatatgatgccctgcacatgcaggccctgcctccaaga
*SEQ ID NO:* 93: *Fusion protein NYESO1 TCRα chain and CD3ζ chain sequence (TCRα signal sequence)* 
atgaagtccctgcgggtgctgctggttatcctgtggctgcagctgagctgggtctggtcccagcagaaagaggtggaacagaacagc
ggccctctgtctgttcctgaaggcgctatcgccagcctgaactgcacctacagcgatagaggcagccagagcttcttctggtacagac
agtacagcggcaagagccccgagctgatcatgttcatctacagcaacggcgacaaagaggacggccggtttacagcccagctga
acaaggccagccagtacgtgtccctgctgatcagagatagccagcctagcgacagcgccacctatctgtgtgccgccgacgacaa
gatcatcttcggcaagggcaccagactgcacatcctgcctaacattcagaaccccgatcctgccgtgtaccagctgagagacagca
agagcagcgacaagagcgtgtgcctgttcaccgacttcgacagccagaccaacgtgtcccagagcaaggacagcgacgtgtaca
tcaccgataagtgcgtgctggacatgcggagcatggacttcaagagcaacagcgccgtggcctggtccaacaagagcgatttcgcc
tgcgccaacgccttcaacaacagcattatccccgaggacacattcttccccagctccgatgtgccctgcgacgtgaagctggtggaa
aagagcttcgagacagacaccaacctgaacttccagaacctgagcctgtgctacctgctggacggcatcctgtttatctacggcgtgat
cctgacagccctgtttctgagagtgaagttcagcagaagcgccgacgctcctgcttaccagcagggccagaaccagctgtacaacg
agctgaacctggggagaagagaagagtacgacgtgctggataagcgcagaggcagagatcctgagatgggcggaaagcccca
gcggagaaagaatcctcaagagggcctgtataatgagctgcagaaagacaagatggccgaggcctactccgagatcggaatgaa
gggcgagcgcagaagaggcaagggacacgatggactgtaccagggcctgagcaccgccaccaaggatacctatgatgccctgc
acatgcaggccctgcctccaagataa
*SEQ ID NO: 94: CD61A signal sequence*
   atgtggcaactgctgctgcctacagctctgctgcttctggtgtctgcc

### Embodiments

In further embodiments, the present invention relates to the following aspects:
1. A functional chimeric T cell receptor (TCR) construct comprising
   e) a first fusion protein comprising a TCRα chain or fragment thereof and a first CD3ζ chain or fragment thereof, wherein the TCRα chain or fragment thereof comprises
      i. a first signal sequence,
      ii. a variable region (Vα), and
      iii. a constant region (Cα),
      and
   f) a second fusion protein comprising a TCRβ chain or fragment thereof and a second CD3ζ chain or fragment thereof, wherein the TCRβ chain or fragment thereof comprises
      iv. a second signal sequence,
      v. a variable region (Vβ), and
      vi. a constant region (Cβ),
      or, alternatively, comprising
   g) a first fusion protein comprising a TCRγ chain or fragment thereof and a first CD3ζ chain or fragment thereof, wherein the TCRγ chain or fragment thereof comprises
      iv. a first signal sequence,
      v. a variable region (Vγ), and
      vi. a constant region (Cγ),
      and
   h) a second fusion protein comprising a TCRδ chain or fragment thereof and a second CD3ζ or fragment thereof chain, wherein the TCRδ chain or fragment thereof comprises
      iv. a second signal sequence,
      v. a variable region (Vδ), and
      vi. a constant region (Cδ).
2. The TCR construct according to aspect 1, wherein the TCR construct does not comprise a CD3γ, CD3δ, and/or CD3ε chain or fragment thereof.
3. The TCR construct according to aspect 1 or 2, wherein the first and the second signal sequences are signal sequences from immune cells, preferably from B cells, natural killer (NK) cells, macrophages, innate lymphoid cells (ILCs) or neutrophils.
4. The TCR construct according to any one of the previous aspects, wherein the first and the second signal sequences are identical.
5. The TCR construct according to any one of the previous aspects, wherein the first and/or the second signal sequence(s) is/are (a) NK cell signal sequence(s), preferably from a protein selected from the group consisting of NKp46, NKp44, NKp30, and CD16A.
6. The TCR construct according to any one of the previous aspects, wherein the first and the second CD3ζ chain or fragments thereof are identical.
7. The TCR construct according to any one of the previous aspects, wherein the first and the second CD3ζ chain or fragments thereof have an amino acid sequence which is at least 80% identical to SEQ ID NO: 7.
8. The TCR construct according to any one of the previous aspects, further comprising one or two DAP10 signaling domain(s).
9. The TCR construct according to aspect 8, wherein the one or two DAP10 signaling domain(s) is/are linked to the first and/or second CD3ζ chain or fragment thereof via a linker.
10. The TCR construct according to aspect 9, wherein the linker comprises or consists of a sequence selected from the group consisting of: GSG, AP, APAP (SEQ ID NO: 9), ESGS (SEQ ID NO: 10), GGGS (SEQ ID NO: 11), GGGGS (SEQ ID NO: 12) and GSGGSG (SEQ ID NO: 13).
11. The TCR construct according to one of the aspects 8 to 10, wherein the DAP10 signaling domain(s) has/have an amino acid sequence which is at least 80% identical to SEQ ID NO: 8.
12. The TCR construct according to any one of the previous aspects, wherein the Vα comprises a CDR 1 having the amino acid sequence of SEQ ID NO: 14, a CDR 2 having the amino acid sequence of SEQ ID NO: 15, and a CDR 3 having the amino acid sequence of SEQ ID NO: 16, and/or wherein the Vβ comprises a CDR 1 having the amino acid sequence of SEQ ID NO: 17, a CDR 2 having the amino acid sequence of SEQ ID NO: 18, and a CDR 3 having the amino acid sequence of SEQ ID NO: 19.
13. The TCR construct according to any one of the previous aspects, wherein the Vα has an amino acid sequence which is at least 80% identical to SEQ ID NO: 20 and/or wherein the Vβ has an amino acid sequence which is at least 80% identical to SEQ ID NO: 21.
14. The TCR construct according to any one of the previous aspects, wherein the Cα has an amino acid sequence which is at least 80% identical to SEQ ID NO: 22 and/or wherein the Cβ has an amino acid sequence which is at least 80% identical to SEQ ID NO: 23 or 24.
15. The TCR construct according to any one of the previous aspects, wherein the TCRα chain or fragment thereof and the first CD3ζ chain or fragment thereof have an amino acid sequence which is at least 80% identical to SEQ ID NO: 25 and/or wherein the TCR β chain or fragment thereof and the second CD3ζ chain or fragment thereof have an amino acid sequence which is at least 80% identical to SEQ ID NO: 26 or 27.
16. Method of producing a TCR construct according to any one of aspects 1 to 15.
17. Nucleic acid encoding a TCR construct according to any one of aspects 1 to 15.
18. Vector comprising the nucleic acid of aspect 17.
19. Vector according to aspect 18, wherein the vector is an expression vector.
20. Vector according to aspects 18 or 19, wherein the vector is a retroviral vector or a lentiviral vector.
21. Cell expressing the TCR construct according to any one of the aspects 1 to 15.
22. Cell according to aspect 21, wherein the cell is isolated or non-naturally occurring.
23. Cell according to aspects 21 or 22, wherein the cell comprises the nucleic acid according to aspect 17 or the vector according to any one of aspects 18 to 20.
24. Cell according to aspects 21 to 23, wherein the cell comprises
   a) an expression vector which comprises at least one nucleic acid according to aspect 17 and/or
   b) a first expression vector which comprises a nucleic acid encoding the TCR α chain and the first CD3ζ chain as in any one of the aspects 1 to 15, and a second expression vector which comprises a nucleic acid encoding the TCR β chain and the second CD3ζ as in any one of the aspects 1 to 15.
25. Cell according to any one of aspects 21 to 24, wherein the cell is a NK cell, a B cell or a macrophage, innate lymphoid cells (ILCs), or neutrophils.
26. Cell according to aspect 25, wherein the cell is a NK cell.
27. Cell according to any one of aspects 21 to 26, wherein the first and/or second signal sequence originate from the same cell type as the cell.
28. Cell according to aspect 27, wherein the cell is a NK cell and wherein the first and/or second signal sequence is/are (a) NK cell signal sequence(s), preferably from a protein selected from the group consisting of NKp46, NKp44, NKp30, and CD16A
29. Pharmaceutical composition comprising the TCR construct according to aspects 1 to 15, the nucleic acid according to aspect 17, the vector according to any ones of aspects 18 to 20, or the cell according to any one of aspects 21 to 28.
30. Pharmaceutical composition according to aspect 29, wherein the pharmaceutical composition comprises at least one pharmaceutically acceptable carrier.
31. The TCR construct according to aspects 1 to 15, the nucleic acid according to aspect 17, the vector according to any ones of aspects 18 to 20, the cell according to any one of aspects 21 to 28, or the pharmaceutical composition according to aspect 29 or 30 for use as a medicament.
32. The TCR construct according to aspects to 15, the nucleic acid according to aspect 17, the vector according to any ones of aspects 18 to 20, the cell according to any one of aspects 21 to 28, or the pharmaceutical composition according to aspect 29 or 30 for use in the treatment of cancer.
33. The TCR construct, the nucleic acid, the cell, or the pharmaceutical composition according to aspect 32, wherein the cancer is hematologic cancer or a solid tumor.
34. The TCR construct, the nucleic acid, the cell, or the pharmaceutical composition according to aspect 32, wherein the cancer is selected from the group consisting of prostate cancer, uterine cancer, thyroid cancer, testicular cancer, renal cancer, pancreatic cancer, ovarian cancer, esophageal cancer, non-small-cell lung cancer, lung adenocarcinoma, squamous cell carcinoma, non-Hodgkin's lymphoma, multiple myeloma, melanoma, hepatocellular carcinoma, head and neck cancer, gastric cancer, endometrial cancer, cervical cancer, colorectal cancer, stomach adenocarcinoma, cholangiocarcinoma, breast cancer, bladder cancer, myeloid leukemia and acute lymphoblastic leukemia, carcinoma, sarcoma, glioblastoma, astrocytoma or osteosarcoma.
35. Adoptive immunotherapy using a TCR construct according to aspects 1 to 15, the nucleic acid according to aspect 17, the vector according to any ones of aspects 18 to 20, the cell according to any one of aspects 21 to 28, or the pharmaceutical composition according to aspect 29 or 30.

### EXAMPLES

### Example 1: Designing Chimeric TCR Fusion Molecules for Expression in NK Cells

Method: All constructs were synthesized as gene fragments (Geneart, Life Technologies GmbH, Germany) and cloned into the retro gamma viral plasmid pES12-6 using the Gibson Assembly Master Mix (NEB, E2611S).

Result: Three distinct constructs were developed to express TCR on NK cell surface and for TCR specific activation of NK cells. These constructs represent significant departures from naturally occurring T cell receptors, particularly in their structural design and signaling mechanisms. Each construct utilizes the NKp46 signal peptide sequence to ensure proper expression and targeting within NK cells. Construct 1, labeled nkTCR-2xCD3ζ (Figure 1A), integrates variable regions Vα and Vβ (NY-ESO1 TCR) with constant regions Cα and Cβ, and dual CD3ζ signalling domains. Construct 2, nkTCR-2xCD3ζ-DAP10(TRB) (Figure 1B), builds upon this by adding a DAP10 signalling domain linked to one of the CD3ζ domains to further potentiate signalling. Construct 3, nkTCR-2xCD3ζ-2xDAP10 (Figure 1C), includes two DAP10 domains, each associated with a CD3ζ domain, aiming to maximize activation potential. All three constructs also incorporate UniTope tag (9-mer epitope sequence with GSG linker (GSGGEVPKDRFSGSG)) These designs strategically combine signaling domains to optimize the chimeric NK-TCR expression and functionality in NK cells.

### Construct 1: nkTCR-2xCD3ζ

This construct, labelled nkTCR-2xCD3ζ, incorporates several features:
1. NKp46 Signal Peptide: Utilizes the NKp46 signal peptide sequence to ensure proper expression and targeting within NK cells, adapting the TCR for a cell type that naturally lacks TCRs.
2. TCR Domain Selection: Integrates variable regions Vα and Vβ (from NY-ESO1 TCR) with constant regions Cα and Cβ, but crucially omits the TCR Cα and Cβ transmembrane and cytoplasmic domains.
3. Signaling Domains: Incorporates dual CD3ζ signaling domains directly into the construct, replacing the natural TCR's reliance on separate CD3 complexes for signaling.
4. Dimerization Strategy: Employs a Boulter cysteine bridge to facilitate dimerization of the different chains, compensating for the absence of the CD3 complex that typically assists in TCR assembly in natural T cells.
5. UniTope Tag: Includes a UniTope tag (a 9-mer epitope (GEVPKDRFSA sequence with GSG linker) as a synthetic addition for potential tracking.

### Constructs 2 and 3: Enhanced Signaling Capabilities

Building upon the foundation of Construct 1, these designs further augment signaling potential:
- Construct 2 (nkTCR-2xCD3ζ-DAP10(TRB)): Adds a DAP10 signaling domain linked to one of the CD3ζ domains via 'GGS' linker, enhancing activation potential.
- Construct 3 (nkTCR-2xCD3ζ-2xDAP10): Incorporates two DAP10 domains, each associated with a CD3ζ domain via 'GGS' linker, maximizing activation capabilities.

### Example 2: Expression of Chimeric NK-TCR in NK92 cells

Method: Chimeric NK-TCR-expressing NK-92 cells were cultured in a medium comprising MEM alpha supplemented with 12.5% horse serum, 12.5% FBS, 1% L-Glutamine, 1% HEPES, 1% Penicillin/Streptomycin, and 50 µM β-mercaptoethanol. Cell density was maintained between 0.3 and 0.4 million/mL, not exceeding 0.8 million/mL, with subculturing performed every 2-3 days. IL-2 (100 U/mL) was added every other day, and the medium was replaced weekly. Before functional assays, IL-2 concentration was reduced to 50 or 20 U/mL to minimize non-specific activation. Optimal growth conditions were ensured through regular medium changes, minimal disturbance to cell clusters, and removal of debris.

For transduction, NK-92 cells were seeded in retronectin-coated plates with IL-2 supplementation, followed by expansion in T75 flasks or GREX devices. To generate viral particles for transduction, the gamma retroviral vector (pES12-6) containing the NY-ESO-1-specific construct (nkTCR-2xCD3ζ, nkTCR-2xCD3ζ-DAP10(TRB), or nkTCR-2xCD3ζ-2xDAP10) or the positive control (NYESO-1 TCR+CD3εγδζ) was transfected into HEK293FT cells using Mirus Transfection Reagent (Cat # MIR2305, Lot: 02083632). A mixture of Mirus reagent and DMEM III was prepared, and DNA for each construct was added. After a 48-hour incubation at 37°C and 5% CO2, the virus supernatant (VSN) was collected.

The VSN was centrifuged to remove residual HEK cells, and 1.5 mL/well (24-well plate) of VSN was added to retronectin-coated wells. Subsequently, 0.25 million NK-92 cells were seeded per well and incubated overnight at 37°C and 5% CO2. After 72 hours, the transduced cells were analyzed for expression (via antibody or multimer staining) and functionality (via cytotoxic assays).

For expression analysis, transduced NK-92 cells were stained with PE-conjugated antibodies: anti-UniTope TraCR, anti-Cβ1 JOVI.1, or an NYESO-1-specific HLA-A02:01-SLL multimer. Briefly, 100,000 cells were transferred to a 96-well U-bottom plate, centrifuged at 450×g for 5 minutes, and the supernatant discarded. Cell pellets were resuspended in 50 µL of the antibody solution (anti-UniTope TraCR, 1:100; or anti-Cβ1 JOVI.1, 1:20; or HLA-A02:01-SLL Tetramer, 1:10) and incubated for 30 minutes at room temperature in the dark. After incubation, 150 µL of FACS buffer was added, and cells were centrifuged at 450×g for 5 minutes. The supernatant was discarded, and cells were resuspended in FACS buffer containing 7AAD (1:100 dilution) for flow cytometric analysis.

Results: Flow cytometry confirmed successful expression and proper folding of chimeric NK-TCR constructs in NK-92 cells. Three chimeric constructs (nkTCR-2xCD3ζ, nkTCR-2xCD3ζ-DAP10(TRB), and nkTCR-2xCD3ζ-2xDAP10) were analyzed alongside the positive control (NYESO-1 TCR+CD3εγδζ) using anti-Cβ1 JOVI.1 or anti-UniTope TraCR antibodies or HLA-A*02:01-SLL multimer staining.

### TCR β-Chain Expression:

Flow cytometry with anti-Cβ1 JOVI.1 (Figure 2A) and anti-UniTope TraCR (Figure 2B) antibodies demonstrated successful expression of the TCR β-chain across all chimeric constructs and the positive control, confirming proper incorporation and surface expression of the TCR β-chain.

### TCR Folding:

Staining with HLA-A02:01-SLL multimer (Figure 2C) showed that cells expressing all chimeric constructs and the positive control were successfully stained. This indicates correct folding of the TCR and its ability to recognize the specific HLA-A02:01-SLL complex.

These results collectively demonstrate the successful expression of chimeric NK-TCR constructs in NK-92 cells. The chimeric receptors exhibited proper folding and structural integrity, as evidenced by TCR β-chain expression and recognition of the HLA-peptide complex.

### Example 3: Cytotoxic Activity of Chimeric NK-TCR Transduced NK92 Cells Against Mel624.38 Cells

Method: The cytotoxic activity of NK92 cells expressing chimeric NK-TCR constructs was evaluated using a 2D killing assay. Three chimeric constructs - nkTCR-2xCD3ζ, nkTCR-2xCD3ζ-DAP10(TRB), and nkTCR-2xCD3ζ-2xDAP10 - were tested alongside a positive control (TCR+CD3εγδζ) and untransduced NK92 cells. Mel624.38 target cells were labeled with NuclightRed (NLR) dye following the manufacturer's instructions and seeded into 96-well flat-bottom plates at a density optimized for achieving an effector-to-target (E:T) ratio of 2:1. Effector cells, including chimeric NK-TCR-expressing NK92 cells and untransduced controls, were prepared by resuspending them in the appropriate medium to match the density of the target cells. The effector cells were then added to the wells containing the labeled Mel624.38_NLR target cells. Plates were transferred to an Incucyte live-cell imaging system to monitor changes in red fluorescence intensity, which served as an indicator of target cell viability. Imaging was conducted under standard incubation conditions (37°C, 5% CO2), with fluorescence images captured at regular intervals.

The reduction in red fluorescence (red counts/Image) over time was quantified to measure target cell killing. Untransduced NK92 cells were included as a negative control to confirm that observed cytotoxicity was specifically mediated by the chimeric NK-TCR constructs. Fluorescence data were analyzed using Incucyte software to calculate the extent and rate of target cell death, enabling a comparative assessment of the antigen-specific killing capabilities of the chimeric constructs and the positive control.

Results: The 2D killing assay demonstrated the cytotoxic activity of NK92 cells expressing chimeric NK-TCR constructs against Mel624.38_NLR target cells. All three chimeric constructs - nkTCR-2xCD3ζ, nkTCR-2xCD3ζ-DAP10(TRB), and nkTCR-2xCD3ζ-2xDAP10 - exhibited significant cytotoxicity, similar to the positive control (TCR+CD3εγδζ). Target cell killing was evident as a reduction in red fluorescence (red counts/Image), monitored and quantified using the Incucyte system (Figure 3A).

Importantly, untransduced NK92 cells showed no measurable cytotoxic activity, confirming that the observed killing was mediated specifically by the chimeric NK-TCR constructs. These results indicate that the chimeric receptors effectively redirected the cytotoxic potential of NK92 cells towards the antigen-expressing Mel624.38 target cells, demonstrating their ability to confer antigen-specific killing capability.

### Example 4: Expression of Chimeric NK-TCR in Primary NK Cells

Method: Primary human NK cells were cultured in Complete NK MACS Medium, consisting of NK MACS Basal Medium supplemented with NK MACS Supplement (100X) and 5% AB human serum. Routine subculturing was carried out with the addition of IL-2 (500 IU/mL) and IL-15 (140 IU/mL), while activation prior to transduction included IL-18 (2,000 IU/mL). Cells were maintained at a density of 0.5-1 million cells/mL, with medium and cytokines replenished every 2-3 days. For cryopreservation, cells were pre-conditioned for 24 hours in medium containing IL-15 (50 ng/mL) and IL-18 (250 ng/mL), resuspended in freezing medium (80% NK Basal medium, 10% human AB serum, 10% DMSO), and stored at -80°C.

For transduction, primary NK cells were seeded into retronectin-coated plates supplemented with IL-2 and subsequently expanded in T75 flasks or GREX devices. Viral particles for transduction were produced by transfecting HEK293FT cells with a gamma retroviral vector (pES12-6) containing one of the NY-ESO-1-specific constructs (nkTCR-2xCD3ζ, nkTCR-2xCD3ζ-DAP10(TRB), or nkTCR-2xCD3ζ-2xDAP10) or a positive control (NYESO-1 TCR+CD3εγδζ). Transfections were performed using Mirus Transfection Reagent (Cat # MIR2305, Lot: 02083632) by preparing a mixture of Mirus reagent and DMEM III and adding the respective DNA constructs. After a 48-hour incubation at 37°C and 5% CO₂, viral supernatants (VSN) were collected and centrifuged to remove residual HEK cells.

For the transduction process, 1.5 mL of VSN was added to each retronectin-coated well of a 24-well plate, and 0.25 million primary NK cells were seeded per well. Plates were incubated overnight at 37°C and 5% CO₂. Following a 72-hour culture period, transduced cells were analyzed for expression via antibody or multimer staining and functionality through cytotoxic assays.

Expression analysis was performed by staining transduced primary NK cells with a PE-conjugated anti-TRBV12-3/4 antibody (Beckman Coulter; Cat PN IM1148). For staining, 100,000 cells were transferred to 96-well U-bottom plates, centrifuged at 450×g for 5 minutes, and the supernatant discarded. Cell pellets were resuspended in 50 µL of antibody solution (anti-UniTope TraCR, 1:100; anti-Cβ1 JOVI.1, 1:20; or HLA-A02:01-SLL Tetramer, 1:10) and incubated for 30 minutes at room temperature in the dark. After incubation, cells were washed with 150 µL of FACS buffer, centrifuged, and resuspended in FACS buffer containing 7AAD (1:100 dilution) for flow cytometric analysis.

Results: The surface expression of chimeric NK-TCR constructs in primary NK cells was confirmed through flow cytometry. Three chimeric constructs - nkTCR-2xCD3ζ, nkTCR-2xCD3ζ-DAP10(TRB), and nkTCR-2xCD3ζ-2xDAP10 - were tested alongside a positive control (TCR+CD3εyδζ). Expression analysis focused on detecting the TCR β-chain using anti-TRBV12-3/4 staining.

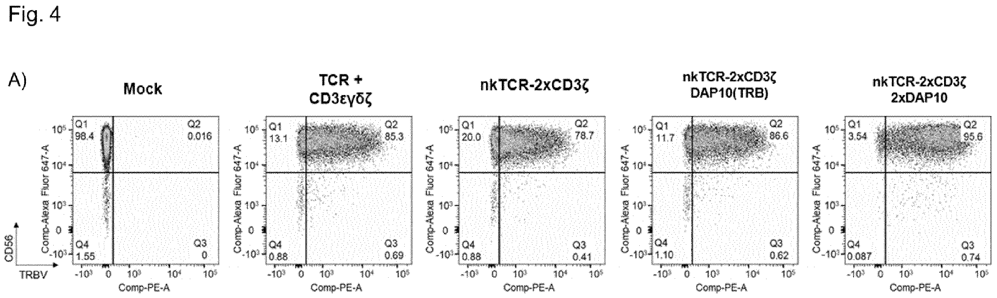

Flow cytometric analysis demonstrated successful expression of the NK-TCR β-chain for all three chimeric constructs and the positive control (Figure 4A). These results confirm the effective transduction and surface expression of chimeric NK-TCR constructs in primary NK cells.

### Example 5: Cytotoxic Activity of Chimeric NK-TCR Transduced primary NK Cells Against Mel624.38 Cells

Method: The cytotoxic activity of primary NK cells expressing chimeric NK-TCR constructs was evaluated using a 2D killing assay. Three chimeric constructs - nkTCR-2xCD3ζ, nkTCR-2xCD3ζ-DAP10(TRB), and nkTCR-2xCD3ζ-2xDAP10 - were tested alongside a positive control (TCR+CD3εγδζ) and untransduced NK92 cells. Mel624.38 target cells were labeled with NuclightRed (NLR) dye following the manufacturer's instructions and seeded into 96-well flat-bottom plates at a density optimized for achieving an effector-to-target (E:T) ratio of 2:1 or 0.5:1. Effector cells, including chimeric NK-TCR-expressing primary NK cells and untransduced controls, were prepared by resuspending them in the appropriate medium to match the density of the target cells. The effector cells were then added to the wells containing the labeled Mel624.38_NLR target cells. Plates were transferred to an Incucyte live-cell imaging system to monitor changes in intensity, which served as an indicator of target cell viability. Imaging was conducted under standard incubation conditions (37°C, 5% CO2), with fluorescence images captured at regular intervals.

The reduction in red fluorescence (red counts/Image) over time was quantified to measure target cell killing. Untransduced NK92 cells were included as a negative control to confirm that observed cytotoxicity was specifically mediated by the chimeric NK-TCR constructs. Fluorescence data were analyzed using Incucyte software to calculate the extent and rate of target cell death, enabling a comparative assessment of the antigen-specific killing capabilities of the chimeric constructs and the positive control.

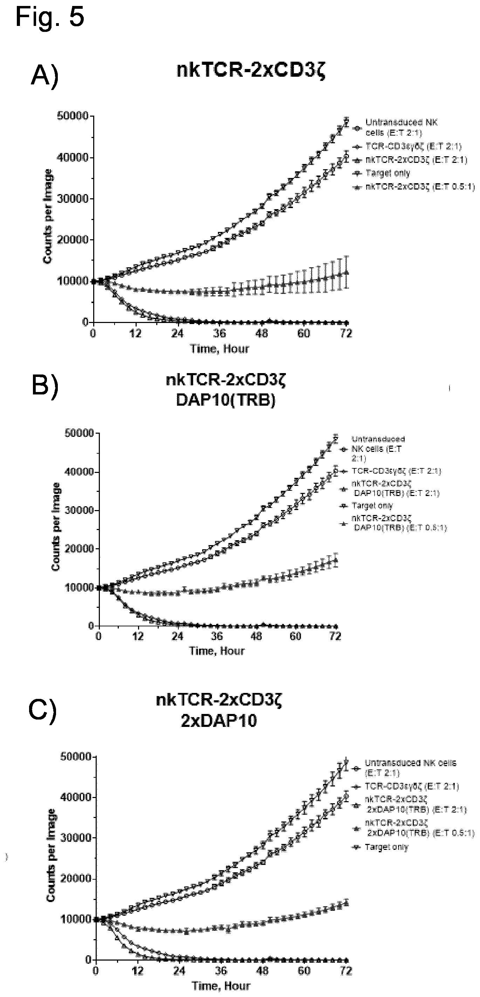

Result: The 2D killing assay demonstrated the cytotoxic activity of primary NK cells expressing chimeric NK-TCR constructs against Mel624.38_NLR target cells. All three chimeric constructs - nkTCR-2xCD3ζ (Figure 5A), nkTCR-2xCD3ζ-DAP10(TRB) (Figure 5B), and nkTCR-2xCD3ζ-2xDAP10(Figure 5C) - exhibited significant cytotoxicity, similar to the positive control (TCR+CD3εγδζ). Target cell killing was evident as a reduction in red fluorescence (red counts/Image), monitored and quantified using the Incucyte system (Figure 3A).

In contrast, untransduced NK92 cells showed no measurable cytotoxic activity toward Mel624.38_NLR target cells, confirming that the observed killing was mediated specifically by the chimeric NK-TCR constructs.

### Example 6: Expression & Cytotoxic Activity of Chimeric NK-TCR containing different signal peptide transduced NK92 Cells Against Mel624.38 Cells

Method: NK92 cells were cultured and transduced following the protocol described in 'Example 2'. Four constructs - NKp46SP_nkTCR-2xCD3ζ, NKp30SP_nkTCR-2xCD3ζ, NKp44SP_nkTCR-2xCD3ζ, and TCRαβSP_nkTCR-2xCD3ζ - were used, each containing the signal peptide of NKp46, NKp30, NKp44, and TCRαβ, respectively.

To analyze expression, transduced NK92 cells were stained with a PE-conjugated anti-Cβ1 (JOVI.1) antibody. Briefly, 100,000 cells were transferred to 96-well U-bottom plates, centrifuged at 450×g for 5 minutes, and the supernatant was removed. Cell pellets were resuspended in 50 µL of PE-conjugated anti-Cβ1 JOVI.1 (1:20 dilution) and incubated in the dark at room temperature for 30 minutes. Following incubation, cells were washed with 150 µL of FACS buffer, centrifuged, and resuspended in FACS buffer containing 7AAD (1:100 dilution) for flow cytometric analysis.

The cytotoxic activity of primary NK92 cells expressing chimeric NK-TCR constructs with different signal peptides was evaluated using a 2D killing assay. Four constructs - NKp46SP_nkTCR-2xCD3ζ, NKp30SP_nkTCR-2xCD3ζ, NKp44SP_nkTCR-2xCD3ζ, and TCRαβSP_nkTCR-2xCD3ζ - were tested alongside untransduced NK92 cells as a negative control.

Mel624.38 target cells were labeled with NuclightRed (NLR) dye per the manufacturer's protocol and seeded into 96-well flat-bottom plates at an optimized density to achieve an effector-to-target (E:T) ratio of 2:1. Effector cells were prepared and resuspended to match the target cell density, then added to the wells containing labeled Mel624.38_NLR cells. Plates were placed in an Incucyte live-cell imaging system to monitor changes in red fluorescence intensity over time, indicating target cell viability. Imaging was conducted under standard incubation conditions (37°C, 5% CO₂), with fluorescence images captured at regular intervals.

Reduction in red fluorescence intensity (red counts per image) over time was quantified to assess target cell killing. Data were analyzed using Incucyte software to determine the rate and extent of target cell death, facilitating a comparative evaluation of the antigen-specific killing capabilities of the chimeric NK-TCR constructs with different signal peptides and the negative control.

Results: Flow cytometry confirmed surface expression of all chimeric NK-TCR constructs in NK92 cells. Constructs NKp46SP_nkTCR-2xCD3ζ, NKp30SP_nkTCR-2xCD3ζ, NKp44SP_nkTCR-2xCD3ζ, and TCRαβSP_nkTCR-2xCD3ζ were compared to untransduced NK92 cells (negative control). Expression was assessed by detecting the TCR β-chain using PE-conjugated anti-Cβ1 staining.

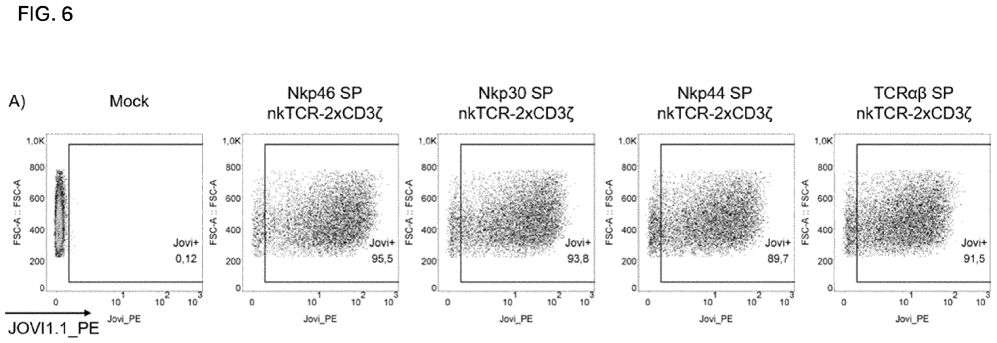

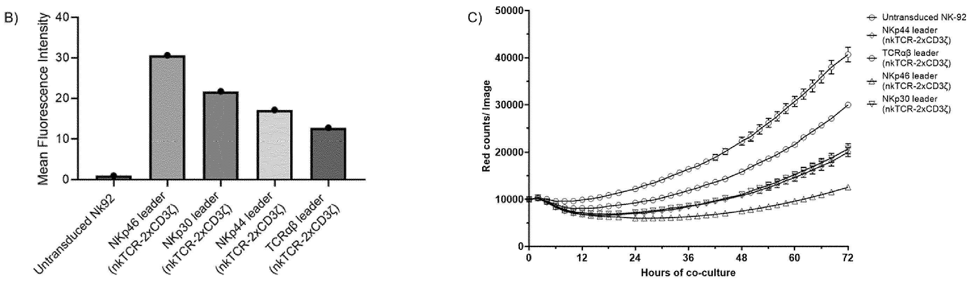

Flow cytometric analysis revealed successful expression of all constructs (Figure 6A), with varying efficiencies indicated by differences in the mean fluorescence intensity (MFI) of Cβ1-positive NK92 cells (Figure 6B). Constructs with the NKp46 signal peptide showed the highest expression efficiency (highest MFI), while those with the TCRαβ signal peptide exhibited the lowest expression efficiency (lowest MFI).

The 2D killing assay showed that NK92 cells expressing the chimeric NKp46SP_nkTCR-2xCD3ζ construct, containing the NKp46 signal peptide, had the highest cytotoxic activity. Conversely, cells expressing TCRαβSP_nkTCR-2xCD3ζ, with the TCRαβ signal peptide, exhibited the lowest cytotoxic activity (Figure 6C).

### Example 7: Expression & Cytotoxic Activity of KRASG12V & PRAME-VLD specific chimeric NK-TCR transduced NK92 cells against DAN-G & Mel624.38 target cells respectively.

Method: NK92 cells were cultured and transduced following the protocol described in 'Example 2' by constructs - KRASG12V-TCR6-nkTCR-2xCD3ζ, KRASG12V-TCR6 + CD3εγδζ (positive control), PRAME-VLD-nkTCR-2xCD3ζ, & PRAME-VLD-TCR + CD3εγδζ (positive control).

To analyze expression, transduced NK92 cells were stained with a PE-conjugated anti-Cβ1 (JOVI.1) antibody. Briefly, 100,000 cells were transferred to 96-well U-bottom plates, centrifuged at 450×g for 5 minutes, and the supernatant was removed. Cell pellets were resuspended in 50 µL of PE-conjugated anti-Cβ1 JOVI.1 (1:20 dilution) and incubated in the dark at room temperature for 30 minutes. Following incubation, cells were washed with 150 µL of FACS buffer, centrifuged, and resuspended in FACS buffer containing 7AAD (1:100 dilution) for flow cytometric analysis.

The cytotoxic activity of NK92 cells expressing KRASG12V-TCR6-nkTCR-2xCD3ζ, KRASG12V-TCR6 + CD3εγδζ (positive control), PRAME-VLD-nkTCR-2xCD3ζ, & PRAME-VLD-TCR + CD3εγδζ (positive control) alongside untransduced NK92 cells as a negative control in a 2D killing assay.

For NK92 cells expressing KRASG12V-TCR6-nkTCR-2xCD3ζ & KRASG12V-TCR6 + CD3εγδζ (positive control), NuclightRed (NLR) dye labelled DAN-G-NLR cells were used as target cells and seeded into 96-well flat-bottom plates at an optimized density to achieve an effector-to-target (E:T) ratio of 4:1. Effector cells were prepared and resuspended to match the target cell density, then added to the wells containing labeled DAN-G-NLR target cells.

Similarly, for NK92 cells expressing PRAME-VLD-nkTCR-2xCD3ζ, & PRAME-VLD-TCR + CD3εγδζ (positive control) NuclightRed (NLR) dye labelled Mel624.38-NLR cells were used as target cells and seeded into 96-well flat-bottom plates at an optimized density to achieve an effector-to-target (E:T) ratio of 4:1. Effector cells were prepared and resuspended to match the target cell density, then added to the wells containing labeled Mel624.38-NLR target cells. Plates were placed in an Incucyte live-cell imaging system to monitor changes in red fluorescence intensity over time, indicating target cell viability. Imaging was conducted under standard incubation conditions (37°C, 5% CO₂), with fluorescence images captured at regular intervals. Reduction in red fluorescence intensity (red counts per image) over time was quantified to assess target cell killing. Data were analyzed using Incucyte software to determine the rate and extent of target cell death, facilitating a comparative evaluation of the antigen-specific killing capabilities of the KRASG12V & PRAME-VLD specific chimeric NK-TCR alongside positive and the negative control.

Results: Flow cytometric analysis revealed successful expression of all KRASG12V-TCR6-nkTCR-2xCD3ζ, KRASG12V-TCR6 + CD3εγδζ (positive control), PRAME-VLD-nkTCR-2xCD3ζ, & PRAME-VLD-TCR + CD3εγδζ (positive control) constructs in NK92 cells (Figure 7A & 7C). Expression was assessed by detecting the TCR β-chain using PE-conjugated anti-Cβ1 staining. The 2D killing assay demonstrated the cytotoxic activity of NK92 cells expressing KRASG12V-TCR6-nkTCR-2xCD3ζ similar to positive control KRASG12V-TCR6 + CD3εγδζ against DAN-G-NLR target cells (Figure 7B). Similarly, PRAME-VLD-nkTCR-2xCD3ζ showed comparable cytotoxicity against MEL624.38-NLR target cells as PRAME-VLD-TCR + CD3εγδζ (positive control). Target cell killing was evident as a reduction in red fluorescence (red counts/Image), monitored and quantified using the Incucyte system (Figure 3A).

Importantly, untransduced NK92 cells showed no measurable cytotoxic activity, confirming that the observed killing was mediated specifically by the chimeric NK-TCR constructs. These results indicate that the universality of chimeric NK-TCR format for expressing the different antigen specific TCRs in NK cells.

## Claims

1. A functional chimeric T cell receptor (TCR) construct comprising
a) a first fusion protein comprising a TCR α chain or fragment thereof and a first CD3ζ chain or fragment thereof, wherein the TCR α chain or fragment thereof comprises
i. a first signal sequence,
ii. a variable region (Vα), and
iii. a constant region (Cα),
and
b) a second fusion protein comprising a TCR β chain or fragment thereof and a second CD3ζ chain or fragment thereof, wherein the TCR β chain or fragment thereof comprises
i. a second signal sequence,
ii. a variable region (Vβ), and
iii. a constant region (Cβ),
or, alternatively, comprising
c) a first fusion protein comprising a TCR γ chain or fragment thereof and a first CD3ζ chain or fragment thereof, wherein the TCR γ chain or fragment thereof comprises
i. a first signal sequence,
ii. a variable region (Vγ), and
iii. a constant region (Cγ),
and
d) a second fusion protein comprising a TCR δ chain or fragment thereof and a second CD3ζ chain or fragment thereof, wherein the TCR δ chain or fragment thereof comprises
i. a second signal sequence,
ii. a variable region (Vδ), and
iii. a constant region (Cδ).

2. The TCR construct according to claim 1, wherein the TCR construct does not comprise a CD3γ, CD3δ, and/or CD3ε chain or fragment thereof.

3. The TCR construct according to claim 1 or 2, wherein the first and/or the second signal sequence(s) is/are (a) NK cell signal sequence(s), preferably from a protein selected from the group consisting of NKp46, NKp44, NKp30, and CD16A.

4. The TCR construct according to any one of the previous claims, further comprising one or two DAP10 signaling domain(s).

5. Method of producing a TCR construct comprising the step of culturing a cell comprising a nucleic acid encoding the TCR construct according to any one of claims 1 to 4.

6. Nucleic acid encoding a TCR construct according to any one of claims 1 to 4.

7. Vector comprising the nucleic acid of claim 6.

8. Cell expressing the TCR construct according to any one of the claims 1 to 4.

9. Cell according to claim 8, wherein the cell comprises
c) an expression vector which comprises at least one nucleic acid according to claim 14 and/or
d) a first expression vector which comprises a nucleic acid encoding the TCR α chain and the first CD3ζ chain as claimed in any one of the claims 1 to 13, and a second expression vector which comprises a nucleic acid encoding the TCR β chain and the second CD3ζ as claimed in any one of the claims 1 to 13.

10. Cell according to claim 8 or 9, wherein the cell is a NK cell.

11. Cell according to claim 10, wherein the first and/or second signal sequence is/are (a) NK cell signal sequence(s), preferably from a protein selected from the group consisting of NKp46, NKp44, NKp30, and CD16A.

12. Pharmaceutical composition comprising the TCR construct according to claims 1 to 4, the nucleic acid according to claim 6, the vector according to claim 7, or the cell according to any one of claims 8 to 11.

13. The TCR construct according to claims 1 to 4, the nucleic acid according to claim 6, the vector according to claim 7, the cell according to any one of claims 8 to 11, or the pharmaceutical composition according to claim 12 for use as a medicament.

14. The TCR construct according to claims 1 to 4, the nucleic acid according to claim 6, the vector according to claim 7, the cell according to any one of claims 8 to 11, or the pharmaceutical composition according to claim 12 for use in the treatment of cancer.

15. The TCR construct according to claims 1 to 4, the nucleic acid according to claim 6, the vector according to claim 7, the cell according to any one of claims 8 to 11, or the pharmaceutical composition according to claim 12 for use in adoptive immunotherapy.
